# EUROPEAN PATENT APPLICATION

(11) **EP 2 995 690 A1**
(43) Date of publication of application: **16.03.2016**
(21) Application number: 15187435.1
(22) Date of filing: 06.10.2008
(51) Int. Cl.: C12Q 1/68

(54) **PROLIFERATION SIGNATURE AND PROGNOSIS FOR GASTROINTESTINAL CANCER**

(30) Priority: 05.10.2007 NZ 56223707
(62) Divisional of application: 08835078.0
(71) Applicant: Pacific Edge Limited, Dunedin, 9016 (NZ)
(72) Inventor: ANJOMSHOAA, Ahmad, Dunedin (NZ); REEVE, Anthony Edward, Dunedin (NZ); LIN, Yu-Hsin, Dunedin (NZ); BLACK, Michael A., Dunedin (NZ)
(74) Representative: Wright, Simon Mark

(57) **Abstract**

This invention relates to methods and compositions for determining the prognosis of cancer in a patient, particularly for gastrointestinal cancer, such as gastric or colorectal cancer. Specifically, this invention relates to the use of genetic markers for the prediction of the prognosis of cancer, such as gastric or colorectal cancer, based on cell proliferation signatures. In various aspects, the invention relates to a method of predicting the likelihood of long-term survival of a cancer patient, a method of determining a treatment regime for a cancer patient, a method of preparing a personalized genomics profile for a cancer patient, among other methods as well as kits and devices for carrying out these methods.

## Description

### FIELD OF THE INVENTION

This invention relates to methods and compositions for determining the prognosis of cancer, particularly gastrointestinal cancer, in a patient. Specifically, this invention relates to the use of genetic markers for determining the prognosis of cancer, such as gastrointestinal cancer, based on cell proliferation signatures.

### BACKGROUND OF THE INVENTION

Cellular proliferation is the most fundamental process in living organisms, and as such is precisely regulated by the expression level of proliferation-associated genes (1). Loss of proliferation control is a hallmark of cancer, and it is thus not surprising that growth-regulating genes are abnormally expressed in tumours relative to the neighbouring normal tissue (2). Proliferative changes may accompany other changes in cellular properties, such as invasion and ability to metastasize, and therefore could affect patient outcome. This association has attracted substantial interest and many studies have been devoted to the exploration of tumour cell proliferation as a potential indicator of outcome.

Cell proliferation is usually assessed by flow cytometry or, more commonly, in tissues, by immunohistochemical evaluation of proliferation markers (3). The most widely used proliferation marker is Ki-67, a protein expressed in all cell cycle phases except for the resting phase G₀ (4). Using Ki-67, a clear association between the proportion of cycling cells and clinical outcome has been established in malignancies such as breast cancer, lung cancer, soft tissue tumours, and astrocytoma (5). In breast cancer, this association has also been confirmed by microarray analysis, leading to a proliferative gene expression profile that has been employed for identifying patients at increased risk of recurrence (6).

However, in colorectal cancer (CRC), the proliferation index (PI) has produced conflicting results as a prognostic factor and therefore cannot be applied in a clinical context (see below). Studies vary with respect to patient selection, sampling methods, cut-off point levels, antibody choices, staining techniques and the way data have been collected and interpreted. The methodological differences and heterogeneity of these studies may partly explain the contradictory results (7),(8). The use of Ki-67 as a proliferation marker also has limitations. The Ki-67 PI estimates the fraction of actively cycling cells, but gives no indication of cell cycle length (3),(9). Thus, tumours with a similar PI may grow at dissimilar rates due to different cycling speeds. In addition, while Ki-67 mRNA is not produced in resting cells, protein may still be detectable in a proportion of colorectal tumours leading to an overestimated proliferation rate (10).

Since the assessment of a prognosis using a single proliferation marker does not appear to be reliable in CRC (see below), there is a need for further tools to predict the prognosis of gastrointestinal cancer. This invention provides further methods and compositions based on prognostic cancer markers, specifically gastrointestinal cancer prognostic markers, to aid in the prognosis and treatment of cancer.

### SUMMARY OF THE INVENTION

In certain aspects of the invention, microarray analysis is used to identify genes that provide a proliferation signature for cancer cells. These genes, and the proteins encoded by those genes, are herein termed gastrointestinal cancer proliferation markers (GCPMs). In one aspect of the invention, the cancer for prognosis is gastrointestinal cancer, particularly gastric or colorectal cancer.

In particular aspects, the invention includes a method for determining the prognosis of a cancer by identifying the expression levels of at least one GCPM in a sample. Selected GCPMs encode proteins that associated with cell proliferation, e.g., cell cycle components. These GCPMs have the added utility in methods for determining the best treatment regime for a particular cancer based on the prognosis. In particular aspects, GCPM levels are higher in non-recurring tumour tissue as compared to recurring tumour tissue. These markers can be used either alone or in combination with each other, or other known cancer markers.

In an additional aspect, this invention includes a method for determining the prognosis of a cancer, comprising: (a) providing a sample of the cancer; (b) detecting the expression level of at least one GCPM family member in the sample; and (c) determining the prognosis of the cancer.

In another aspect, the invention includes a step of detecting the expression level of at least one GCPM RNA, for example, at least one mRNA. In a further aspect, the invention includes a step of detecting the expression level of at least one GCPM protein. In yet a further aspect, the invention includes a step of detecting the level of at least one GCPM peptide. In yet another aspect, the invention includes detecting the expression level of at least one GCPM family member in the sample. In an additional aspect, the GCPM is a gene associated with cell proliferation, such as a cell cycle component. In other aspects, the at least one GCPM is selected from Table A, Table B, Table C or Table D, herein.

In a still further aspect, the invention includes a method for detecting the expression level of at least one GCPM set forth in Table A, Table B, Table C or Table D, herein. In an even further aspect, the invention includes a method for detecting the expression level of at least one of CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37. In yet a further aspect, the invention comprises detecting the expression level of at least one of CDC2, RFC4, PCNA, CCNE1, CCND1, CDK7, MCM genes, FEN1, MAD2L1, MYBL2, RRM2, and BUB3.

In additional aspects, the expression levels of at least two, or at least 5, or at least 10, at least 15, at least 20, at least 25, at least 30, at least 35, at least 40, at least 45, at least 50, or at least 75 of the proliferation markers or their expression products are determined, for example, as selected from Table A, Table, B, Table C or Table D; as selected from CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as selected from CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In other aspects, the expression levels of all proliferation markers or their expression products are determined, for example, as listed in Table A, Table, B, Table C or Table D; as listed for the group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as listed for the group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In yet a further aspect, the invention includes a method of determining a treatment regime for a cancer comprising: (a) providing a sample of the cancer; (b) detecting the expression level of at least one GCPM family member in the sample; (c) determining the prognosis of the cancer based on the expression level of at least one GCPM family member; and (d) determining the treatment regime according to the prognosis.

In yet another aspect, the invention includes a device for detecting at least one GCPM, comprising: (a) a substrate having at least one GCPM capture reagent thereon; and (b) a detector capable of detecting the at least one captured GCPM, the capture reagent, or a complex thereof.

An additional aspect of the invention includes a kit for detecting cancer, comprising: (a) a GCPM capture reagent; (b) a detector capable of detecting the captured GCPM, the capture reagent, or a complex thereof; and, optionally, (c) instructions for use. In certain aspects, the kit also includes a substrate for the GCPM as captured.

Yet a further aspect of the invention includes a method for detecting at least one GCPM using quantitative PCR, comprising: (a) a forward primer specific for the at least one GCPM; (b) a reverse primer specific for the at least one GCPM; (c) PCR reagents; and, optionally, at least one of: (d) a reaction vial; and (e) instructions for use.

Additional aspects of this invention include a kit for detecting the presence of at least one GCPM protein or peptide, comprising: (a) an antibody or antibody fragment specific for the at least one GCPM protein or peptide; and, optionally, at least one of: (b) a label for the antibody or antibody fragment; and (c) instructions for use. In certain aspects, the kit also includes a substrate having a capture agent for the at least one GCPM protein or peptide.

In specific aspects, this invention includes a method for determining the prognosis of gastrointestinal cancer, especially colorectal or gastric cancer, comprising the steps of: (a) providing a sample, e.g., tumour sample, from a patient suspected of having gastrointestinal cancer; (b) measuring the presence of a GCPM protein using an ELISA method.

In additional aspects of this invention, one or more GCPMs of the invention are selected from the group outlined in Table A, Table B, Table C or Table D, herein. Other aspects and embodiments of the invention are described herein below.

### BRIEF DESCRIPTION OF THE DRAWINGS

This invention is described with reference to specific embodiments thereof and with reference to the figures.

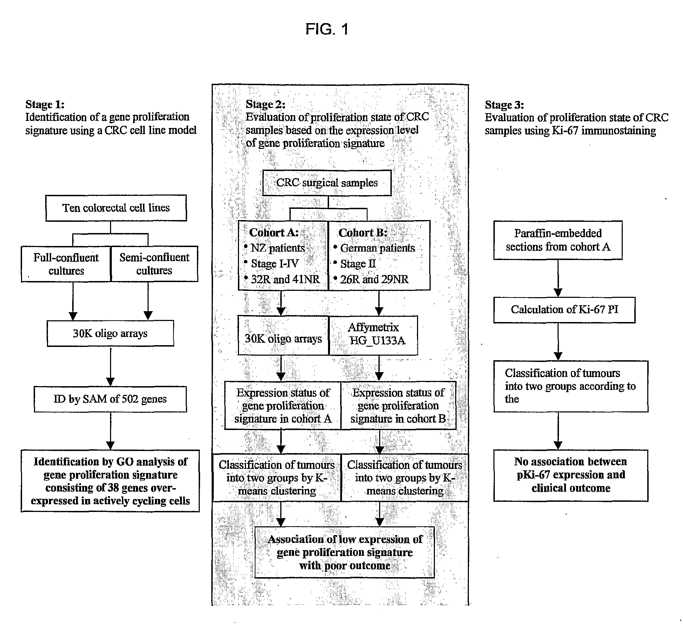

FIG. 1: An overview of the approach used to derive and apply the gene proliferation signature (GPS) disclosed herein.

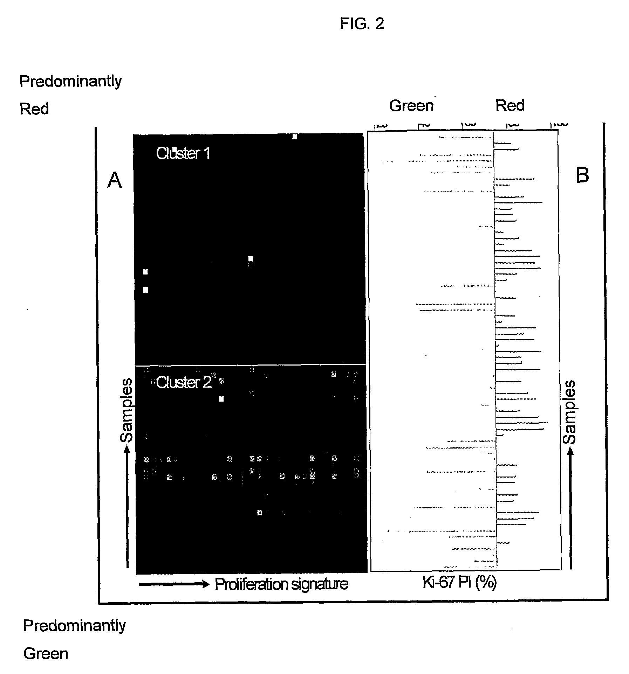

FIG. 2A: K-means clustering of 73 Cohort A tumours into two groups according to the expression level of the gene proliferation signature. FIG. 2B: Bar graph of Ki-67 PI (%); vertical line represents the mean Ki-67 PI across all samples. Tumours with a proliferation index about and below the mean are shown in red and green, respectively. The results show that over-expression of the proliferation signature is not always associated with a higher Ki-67 PI.

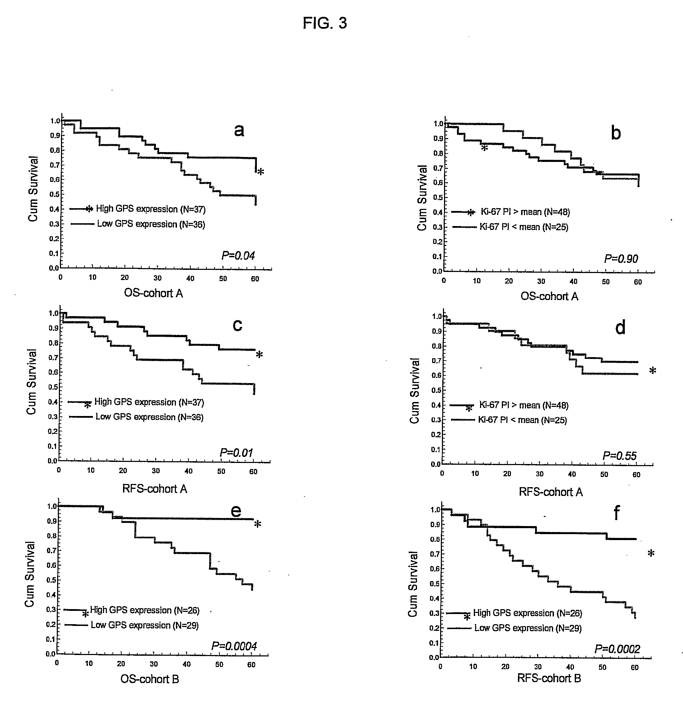

FIG. 3: Kaplan-Meier survival curves according to the expression level of GPS (gene proliferation signal) and Ki-67 PI. Both overall (OS) and recurrence-free survival (RFS) are significantly shorter in patients with low GPS expression in colorectal cancer Cohort A (a, b) and colorectal cancer Cohort B (c, d). No difference was observed in the survival rates of Cohort A patients according to Ki-67 PI (e, f). *P* values from Log rank test are indicated.

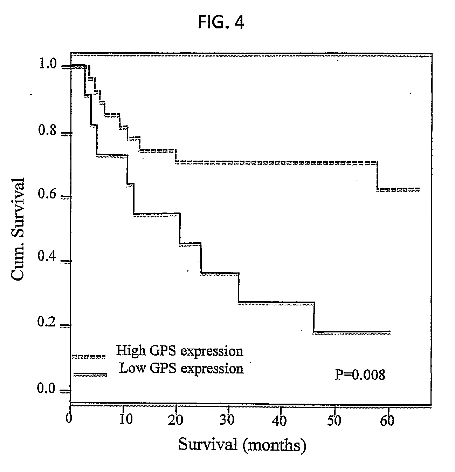

FIG. 4: Kaplan-Meier survival curves according to the expression level of GPS (gene proliferation signal) in gastric cancer patients. Overall survival is significantly shorter in patients with low GPS expression in this cohort of 38 gastric cancer patients of mixed stage. *P* values from Log rank test are indicated.

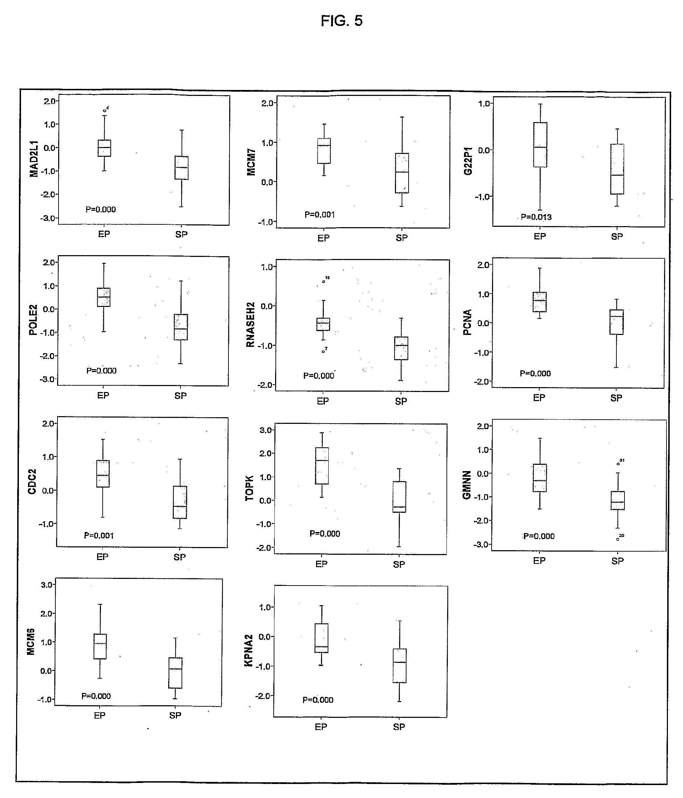

FIG. 5: A box-and-whisker plot showing differential expression between cycling cells in the exponential phase (EP) and growth-inhibited cells in the stationary phase (SP) of 11 QRT-PCR-validated genes. The box range includes the 25 to the 75 percentiles of the data. The horizontal line in the box represents the median value. The "whiskers" are the largest and smallest values (excluding outliers). Any points more than 3/2 times of the interquartile range from the end of a box will be outliers and presented as a dot. The Y axis represents the log 2 fold change of the ratio between cell line RNA and reference RNA. Analysis was performed using SPSS software.

### DETAILED DESCRIPTION OF THE INVENTION

Because a single proliferation marker is insufficient for obtaining reliable CRC prognosis, the simultaneous analysis of several growth-related genes by microarray was employed to provide a more quantitative and objective method to determine the proliferation state of a gastrointestinal tumour. Table 1 (below) illustrates the previously published and conflicting results shown for use of the proliferation index (PI) as a prognostic factor for colorectal cancer.

**Table 1: Summary of studies on the association of proliferation indices with the CRC patients' survival**

| Study | Number of patients | Dukes stage | Marker | Association with survival |
|---|---|---|---|---|
| Evans et al, 2006¹¹ | 40 | A-C | Ki-67 | |
| Rosati et al, 2004¹² | 103 | B-C | Ki-67 | |
| Ishida et al, 2004¹³ | 51 | C | Ki-67 | |
| Buglioni et al, 1999¹⁴ | 171 | A-D | Ki-67 | |
| Guerra et al, 1998¹⁵ | 108 | A-C | PCNA | No association was found between proliferation index and survival |
| Kyzer and Gordon, 1997¹⁶ | 30 | B-D | Ki-67 | |
| Jansson and Sun, 1997¹⁷ | 255 | A-D | Ki-67 | |
| Baretton et al, 1996¹⁸ | 95 | A-B | Ki-67 | |
| Sun et al, 1996¹⁹ | 293 | A-C | PCNA | |
| Kubota et al, 1992²⁰ | 100 | A-D | Ki-67 | |
| Valera et al, 2005²¹ | 106 | A-D | Ki-67 | |
| Dziegiel et al, 2003²² | 81 | NI | Ki-67 | |
| Scopa et al, 2003²³ | 117 | A-D | Ki-67 | **High** proliferation index was associated with **shorter** survival |
| Bhatavdekar et al, 2001²⁴ | 98 | B-C | Ki-67 | |
| Chen et al, 1997²⁵ | 70 | B-C | Ki-67 | |
| Choi et al, 1997²⁶ | 86 | B-D | PCNA | |
| Hilska et al, 2005²⁷ | 363 | A-D | Ki-67 | |
| Salminen et al, 2005²⁸ | 146 | A-D | Ki-67 | |
| Garrity et al, 2004²⁹ | 366 | B-C | Ki-67 | **Low** proliferation index was associated with **shorter** survival |
| Allegra et al, 2003³⁰ | 706 | B-C | Ki-67 | |
| Palmqvist et al, 1999³¹ | 56 | B | Ki-67 | |
| Pazadiso et al, 1996³² | 71 | NI | PCNA | |
| Neoptolemos et al, 1995³³ | 79 | A-C | PCNA | |

| | | | | |
|---|---|---|---|---|
| NI: No Information available | | | | |

In contrast, the present disclosure has succeeded in (i) defining a CRC-specific gene proliferation signature (GPS) using a cell line model; and (ii) determining the prognostic significance of the GPS in the prediction of patient outcome and its association with clinico-pathologic variables in two independent cohorts of CRC patients.

### Definitions

Before describing embodiments of the invention in detail, it will be useful to provide some definitions of terms used herein.

As used herein "antibodies" and like terms refer to immunoglobulin molecules and immunologically active portions of immunoglobulin (Ig) molecules, i.e., molecules that contain an antigen binding site that specifically binds (immunoreacts with) an antigen. These include, but are not limited to, polyclonal, monoclonal, chimeric, single chain, Fc, Fab, Fab', and Fab₂ fragments, and a Fab expression library. Antibody molecules relate to any of the classes IgG, IgM, IgA, IgE, and IgD, which differ from one another by the nature of heavy chain present in the molecule. These include subclasses as well, such as IgG1, IgG2, and others. The light chain may be a kappa chain or a lambda chain. Reference herein to antibodies includes a reference to all classes, subclasses, and types. Also included are chimeric antibodies, for example, monoclonal antibodies or fragments thereof that are specific to more than one source, e.g., a mouse or human sequence. Further included are camelid antibodies, shark antibodies or nanobodies.

The term "marker" refers to a molecule that is associated quantitatively or qualitatively with the presence of a biological phenomenon. Examples of "markers" include a polynucleotide, such as a gene or gene fragment, RNA or RNA fragment; or a polypeptide such as a peptide, oligopeptide, protein, or protein fragment; or any related metabolites, by products, or any other identifying molecules, such as antibodies or antibody fragments, whether related directly or indirectly to a mechanism underlying the phenomenon. The markers of the invention include the nucleotide sequences (e.g., GenBank sequences) as disclosed herein, in particular, the full-length sequences, any coding sequences, any fragments, or any complements thereof.

The terms "GCPM" or "gastrointestinal cancer proliferation marker" or "GCPM family member" refer to a marker with increased expression that is associated with a positive prognosis, e.g., a lower likelihood of recurrence cancer, as described herein, but can exclude molecules that are known in the prior art to be associated with prognosis of gastrointestinal cancer. It is to be understood that the term GCPM does not require that the marker be specific only for gastrointestinal tumours. Rather, expression of GCPM can be altered in other types of tumours, including malignant tumours.

Non-limiting examples of GCPMs are included in Table A, Table B, Table C or Table D, herein below, and include, but are not limited to, the specific group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; and the specific group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

The terms "cancer" and "cancerous" refer to or describe the physiological condition in mammals that is typically characterized by abnormal or unregulated cell growth. Cancer and cancer pathology can be associated, for example, with metastasis, interference with the normal functioning of neighbouring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc. Specifically included are gastrointestinal cancers, such as esophageal, stomach, small bowel, large bowel, anal, and rectal cancers, particularly included are gastric and colorectal cancers.

The term "colorectal cancer" includes cancer of the colon, rectum, and/or anus, and especially, adenocarcinomas, and may also include carcinomas (e.g., squamous cloacogenic carcinomas), melanomas, lymphomas, and sarcomas. Epidermoid (nonkeratinizing squamous cell or basaloid) carcinomas are also included. The cancer may be associated with particular types of polyps or other lesions, for example, tubular adenomas, tubulovillous adenomas (e.g., villoglandular polyps), villous (e.g., papillary) adenomas (with or without adenocarcinoma), hyperplastic polyps, hamartomas, juvenile polyps, polypoid carcinomas, pseudopolyps, lipomas, or leiomyomas. The cancer may be associated with familial polyposis and related conditions such as Gardner's syndrome or Peutz-Jeghers syndrome. The cancer may be associated, for example, with chronic fistulas, irradiated anal skin, leukoplakia, lymphogranuloma venereum, Bowen's disease (intraepithelial carcinoma), condyloma acuminatum, or human papillomavirus. In other aspects, the cancer may be associated with basal cell carcinoma, extramammary Paget's disease, cloacogenic carcinoma, or malignant melanoma.

The terms "differentially expressed gene," "differential gene expression," and like phrases, refer to a gene whose expression is activated to a higher or lower level in a subject (e.g., test sample), specifically cancer, such as gastrointestinal cancer, relative to its expression in a control subject (e.g., control sample). The terms also include genes whose expression is activated to a higher or lower level at different stages of the same disease; in recurrent or non-recurrent disease; or in cells with higher or lower levels of proliferation. A differentially expressed gene may be either activated or inhibited at the polynucleotide level or polypeptide level, or may be subject to alternative splicing to result in a different polypeptide product. Such differences may be evidenced by a change in mRNA levels, surface expression, secretion or other partitioning of a polypeptide, for example.

Differential gene expression may include a comparison of expression between two or more genes or their gene products; or a comparison of the ratios of the expression between two or more genes or their gene products; or a comparison of two differently processed products of the same gene, which differ between normal subjects and diseased subjects; or between various stages of the same disease; or between recurring and non-recurring disease; or between cells with higher and lower levels of proliferation; or between normal tissue and diseased tissue, specifically cancer, or gastrointestinal cancer. Differential expression includes both quantitative, as well as qualitative, differences in the temporal or cellular expression pattern in a gene or its expression products among, for example, normal and diseased cells, or among cells which have undergone different disease events or disease stages, or cells with different levels of proliferation.

The term "expression" includes production of polynucleotides and polypeptides, in particular, the production of RNA (e.g., mRNA) from a gene or portion of a gene, and includes the production of a protein encoded by an RNA or gene or portion of a gene, and the appearance of a detectable material associated with expression. For example, the formation of a complex, for example, from a protein-protein interaction, protein-nucleotide interaction, or the like, is included within the scope of the term "expression". Another example is the binding of a binding ligand, such as a hybridization probe or antibody, to a gene or other oligonucleotide, a protein or a protein fragment and the visualization of the binding ligand. Thus, increased intensity of a spot on a microarray, on a hybridization blot such as a Northern blot, or on an immunoblot such as a Western blot, or on a bead array, or by PCR analysis, is included within the term "expression" of the underlying biological molecule.

The term "gastric cancer" includes cancer of the stomach and surrounding tissue, especially adenocarcinomas, and may also include lymphomas and leiomyosarcomas. The cancer may be associated with gastric ulcers or gastric polyps, and may be classified as protruding, penetrating, spreading, or any combination of these categories, or, alternatively, classified as superficial (elevated, flat, or depressed) or excavated.

The term "long-term survival" is used herein to refer to survival for at least 5 years, more preferably for at least 8 years, most preferably for at least 10 years following surgery or other treatment

The term "microarray" refers to an ordered arrangement of capture agents, preferably polynucleotides (e.g., probes) or polypeptides on a substrate. See, e.g., Microarray Analysis, M. Schena, John Wiley & Sons, 2002; Microarray Biochip Technology, M. Schena, ed., Eaton Publishing, 2000; Guide to Analysis of DNA Microarray Data, S. Knudsen, John Wiley & Sons, 2004; and Protein Microarray Technology, D. Kambhampati, ed., John Wiley & Sons, 2004.

The term "oligonucleotide" refers to a polynucleotide, typically a probe or primer, including, without limitation, single-stranded deoxyribonucleotides, single- or double-stranded ribonucleotides, RNA:DNA hybrids, and double-stranded DNAs. Oligonucleotides, such as single-stranded DNA probe oligonucleotides, are often synthesized by chemical methods, for example using automated oligonucleotide synthesizers that are commercially available, or by a variety of other methods, including *in vitro* expression systems, recombinant techniques, and expression in cells and organisms.

The term "polynucleotide," when used in the singular or plural, generally refers to any polyribonucleotide or polydeoxribonucleotide, which may be unmodified RNA or DNA or modified RNA or DNA. This includes, without limitation, single- and double-stranded DNA, DNA including single- and double- stranded regions, single- and double-stranded RNA, and RNA including single- and double-stranded regions, hybrid molecules comprising DNA and RNA that may be single-stranded or, more typically, double-stranded or include single- and double-stranded regions. Also included are triple-stranded regions comprising RNA or DNA or both RNA and DNA. Specifically included are mRNAs, cDNAs, and genomic DNAs. The term includes DNAs and RNAs that contain one or more modified bases, such as tritiated bases, or unusual bases, such as inosine. The polynucleotides of the invention can encompass coding or non-coding sequences, or sense or antisense sequences.

"Polypeptide," as used herein, refers to an oligopeptide, peptide, or protein sequence, or fragment thereof, and to naturally occurring, recombinant, synthetic, or semi-synthetic molecules. Where "polypeptide" is recited herein to refer to an amino acid sequence of a naturally occurring protein molecule, "polypeptide" and like terms, are not meant to limit the amino acid sequence to the complete, native amino acid sequence for the full-length molecule. It will be understood that each reference to a "polypeptide" or like term, herein, will include the full-length sequence, as well as any fragments, derivatives, or variants thereof.

The term "prognosis" refers to a prediction of medical outcome (e.g., likelihood of long-term survival); a negative prognosis, or bad outcome, includes a prediction of relapse, disease progression (e.g., tumour growth or metastasis, or drug resistance), or mortality; a positive prognosis, or good outcome, includes a prediction of disease remission, (e.g., disease-free status), amelioration (e.g., tumour regression), or stabilization.

The terms "prognostic signature," "signature," and the like refer to a set of two or more markers, for example GCPMs, that when analysed together as a set allow for the determination of or prediction of an event, for example the prognostic outcome of colorectal cancer. The use of a signature comprising two or more markers reduces the effect of individual variation and allows for a more robust prediction. Non-limiting examples of GCPMs are included in Table A, Table B, Table C or Table D, herein below, and include, but are not limited to, the specific group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; and the specific group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In the context of the present invention, reference to "at least one," "at least two," "at least five," etc., of the markers listed in any particular set (e.g., any signature) means any one or any and all combinations of the markers listed.

The term "prediction method" is defined to cover the broader genus of methods from the fields of statistics, machine learning, artificial intelligence, and data mining, which can be used to specify a prediction model. These are discussed further in the Detailed Description section.

The term "prediction model" refers to the specific mathematical model obtained by applying a prediction method to a collection of data. In the examples detailed herein, such data sets consist of measurements of gene activity in tissue samples taken from recurrent and non-recurrent colorectal cancer patients, for which the class (recurrent or non-recurrent) of each sample is known. Such models can be used to (1) classify a sample of unknown recurrence status as being one of recurrent or non-recurrent, or (2) make a probabilistic prediction (i.e., produce either a proportion or percentage to be interpreted as a probability) which represents the likelihood that the unknown sample is recurrent, based on the measurement of mRNA expression levels or expression products, of a specified collection of genes, in the unknown sample. The exact details of how these gene-specific measurements are combined to produce classifications and probabilistic predictions are dependent on the specific mechanisms of the prediction method used to construct the model.

The term "proliferation" refers to the processes leading to increased cell size or cell number, and can include one or more of: tumour or cell growth, angiogenesis, innervation, and metastasis.

The term "qPCR" or "QPCR" refers to quantative polymerase chain reaction as described, for example, in PCR Technique: Quantitative PCR, J.W. Larrick, ed., Eaton Publishing, 1997, and A-Z of Quantitative PCR, S. Bustin, ed., IUL Press, 2004.

The term "tumour" refers to all neoplastic cell growth and proliferation, whether malignant or benign, and all pre-cancerous and cancerous cells and tissues.

Sensitivity", "specificity" (or "selectivity"), and "classification rate", when applied to the describing the effectiveness of prediction models mean the following:
"Sensitivity" means the proportion of truly positive samples that are also *predicted* (by the model) to be positive. In a test for cancer recurrence, that would be the proportion of recurrent tumours predicted by the model to be recurrent. "Specificity" or "selectivity" means the proportion of truly negative samples that are also *predicted* (by the model) to be negative. In a test for CRC recurrence, this equates to the proportion of non-recurrent samples that are predicted to by non-recurrent by the model. "Classification Rate" is the proportion of all samples that are correctly classified by the prediction model (be that as positive or negative).
"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ a denaturing agent during hybridization, such as formamide, for example, 50% (v/v) formamide with 0.1 % bovine serum albumin/0.1 % Ficoll/0.1% polyvinylpyrrolidone/50 mM sodium phosphate buffer at pH 6.5 with 750 mM sodium chloride, 75 mM sodium citrate at 42°C; or (3) employ 50% formamide, 5X SSC (0.75 M NaCl, 0.075 M sodium citrate), 50 mM sodium phosphate (pH 6.8), 0.1% sodium pyrophosphate, 5X, Denhardt's solution, sonicated salmon sperm DNA (50 µg/ml), 0.1% SDS, and 10% dextran sulfate at 42°C, with washes at 42°C in 0.2X SSC (sodium chloride/sodium citrate) and 50% formamide at 55°C, followed by a high-stringency wash comprising 0.1X SSC containing EDTA at 55°C.
"Moderately stringent conditions" may be identified as described by Sambrook et al., Molecular Cloning: A Laboratory Manual, New York: Cold Spring Harbor Press, 1989, and include the use of washing solution and hybridization conditions (e. g., temperature, ionic strength, and % SDS) less stringent that those described above. An example of moderately stringent conditions is overnight incubation at 37°C in a solution comprising: 20% formamide, 5X SSC (150 mM NaCl, 15 mM trisodium citrate), 50 mM sodium phosphate (pH 7.6), 5X Denhardt's solution, 10% dextran sulfate, and 20 mg/ml denatured sheared salmon sperm DNA, followed by washing the filters in 1X SSC at about 37-50°C. The skilled artisan will recognize how to adjust the temperature, ionic strength, etc. as necessary to accommodate factors such as probe length and the like.

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, Molecular Cloning: A Laboratory Manual, 2nd edition, Sambrook et al., 1989; Oligonucleotide Synthesis, MJ Gait, ed., 1984; Animal Cell Culture, R.I. Freshney, ed., 1987; Methods in Enzymology, Academic Press, Inc.; Handbook of Experimental Immunology, 4th edition, D .M. Weir & CC. Blackwell, eds., Blackwell Science Inc., 1987; Gene Transfer Vectors for Mammalian Cells, J.M. Miller & M.P. Calos, eds., 1987; Current Protocols in Molecular Biology, F.M. Ausubel et al., eds., 1987; and PCR: The Polymerase Chain Reaction, Mullis et al., eds., 1994.

### Description of Embodiments of the Invention

Cell proliferation is an indicator of outcome in some malignancies. In colorectal cancer, however, discordant results have been reported. As these results are based on a single proliferation marker, the present invention discloses the use of microarrays to overcome this limitation, to reach a firmer conclusion, and to determine the prognostic role of cell proliferation in colorectal cancer. The microarray-based proliferation studies shown herein indicate that reduced rate of the proliferation signature in colorectal cancer is associated with poor outcome. The invention can therefore be used to identify patients at high risk of early death from cancer.

The present invention provides for markers for the determination of disease prognosis, for example, the likelihood of recurrence of tumours, including gastrointestinal tumours. Using the methods of the invention, it has been found that numerous markers are associated with the progression of gastrointestinal cancer, and can be used to determine the prognosis of cancer. Microarray analysis of samples taken from patients with various stages of colorectal tumours has led to the surprising discovery that specific patterns of marker expression are associated with prognosis of the cancer.

An increase in certain GCPMs, for example, markers associated with cell proliferation, is indicative of positive prognosis. This can include decreased likelihood of cancer recurrence after standard treatment, especially for gastrointestinal cancer, such as gastric or colorectal cancer. Conversely, a decrease in these markers is indicative of a negative prognosis. This can include disease progression or the increased likelihood of cancer recurrence, especially for gastrointestinal cancer, such as gastric or colorectal cancer. A decrease in expression can be determined, for example, by comparison of a test sample (e.g., tumour sample) to samples associated with a positive prognosis. An increase in expression can be determined, for example, by comparison of a test sample (e.g., tumour samples) to samples associated with a negative prognosis.

For example, to obtain a prognosis, a patient's sample (e.g., tumour sample) can be compared to samples with known patient outcome. If the patient's sample shows increased expression of GCPMs that is comparable to samples with good outcome, and/or higher than samples with poor outcome, then a positive prognosis is implicated. If the patient's sample shows decreased expression of GCPMs that is comparable to samples with poor outcome, and/or lower than samples with good outcome, then a negative prognosis is implicated. Alternatively, a patient's sample can be compared to samples of actively proliferating/non-proliferating tumour cells. If the patient's sample shows increased expression of GCPMs that is comparable to actively proliferating cells, and/or higher than non-proliferating cells, then a positive prognosis is implicated. If the patient's sample shows decreased expression of GCPMs that is comparable to non-proliferating cells, and/or lower than actively proliferating cells, then a negative prognosis is implicated.

The invention provides for a set of genes, identified from cancer patients with various stages of tumours, outlined in Table C that are shown to be prognostic for colorectal cancer. These genes are all associated with cell proliferation and establish a relationship between cell proliferation genes and their utility in cancers prognosis. It has also been found that the genes in the prognostic signature listed in Table C are also correlated with additional cell proliferation genes. Based on these finding, the invention also provides for a set of cell cycle genes, shown in Table D, that are differentially expressed between high and low proliferation groups, for use as prognostic markers. Further, based on the surprising finding of the correlation between prognosis and cell proliferation-related genes, the invention also provides for a set of proliferation-related genes differentially expressed between cell lines in high and low proliferative states (Table A) and known proliferative-related genes (Table B). The genes outlined in Table A, Table B, Table C and Table D provide for a set of gastrointestinal cancer prognostic markers (gCPMs).

As one approach, the expression of a panel of markers (e.g., GCPMs) can be analysed by techniques including Linear Discriminant Analysis (LDA) to work out a prognostic score. The marker panel selected and prognostic score calculation can be derived through extensive laboratory testing and multiple independent clinical development studies.

The disclosed GCPMs therefore provide a useful tool for determining the prognosis of cancer, and establishing a treatment regime specific for that tumour. In particular, a positive prognosis can be used by a patient to decide to pursue standard or less invasive treatment options. A negative prognosis can be used by a patient to decide to terminate treatment or to pursue highly aggressive or experimental treatments. In addition, a patient can chose treatments based on their impact on cell proliferation or the expression of cell proliferation markers (e.g., GCPMs). In accordance with the present invention, treatments that specifically target cells with high proliferation or specifically decrease expression of cell proliferation markers (e.g., GCPMs) would not be preferred for patients with gastrointestinal cancer, such as colorectal cancer or gastric cancer.

Levels of GCPMs can be detected in tumour tissue, tissue proximal to the tumour, lymph node samples, blood samples, serum samples, urine samples, or faecal samples, using any suitable technique, and can include, but is not limited to, oligonucleotide probes, quantitative PCR, or antibodies raised against the markers. The expression level of one GCPM in the sample will be indicative of the likelihood of recurrence in that subject. However, it will be appreciated that by analyzing the presence and amounts of expression of a plurality of GCPMs, and constructing a proliferation signature, the sensitivity and accuracy of prognosis will be increased. Therefore, multiple markers according to the present invention can be used to determine the prognosis of a cancer.

The present invention relates to a set of markers, in particular, GCPMs, the expression of which has prognostic value, specifically with respect to cancer-free survival. In specific aspects, the cancer is gastrointestinal cancer, particularly, gastric or colorectal cancer, and, in further aspects, the colorectal cancer is an adenocarcinoma.

In one aspect, the invention relates to a method of predicting the likelihood of long-term survival of a cancer patient without the recurrence of cancer, comprising determining the expression level of one or more proliferation markers or their expression products in a sample obtained from the patient, normalized against the expression level of all RNA transcripts or their products in the sample, or of a reference set of RNA transcripts or their expression products, wherein the proliferation marker is the transcript of one or more markers listed in Table A, Table B, Table C or Table D, herein. In particular aspects, a decrease in expression levels of one or more GCPM indicates a decreased likelihood of long-term survival without cancer recurrence, while an increase in expression levels of one or more GCPM indicates an increased likelihood of long-term survival without cancer recurrence.

In a further aspect, the expression levels one or more, for example at least two, or at least 3, or at least 4, or at least 5, or at least 10, at least 15, at least 20, at least 25, at least 30, at least 35, at least 40, at least 45, at least 50, or at least 75 of the proliferation markers or their expression products are determined, e.g., as selected from Table A, Table, B, Table C or Table D; as selected from CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as selected from CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In another aspect, the method comprises the determination of the expression levels of all proliferation markers or their expression products, e.g., as listed in Table A, Table, B, Table C or Table D; as listed for the group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as listed for the group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

The invention includes the use of archived paraffin-embedded biopsy material for assay of all markers in the set, and therefore is compatible with the most widely available type of biopsy material. It is also compatible with several different methods of tumour tissue harvest, for example, via core biopsy or fine needle aspiration. In a further aspect, RNA is isolated from a fixed, wax-embedded cancer tissue specimen of the patient. Isolation may be performed by any technique known in the art, for example from core biopsy tissue or fine needle aspirate cells.

In another aspect, the invention relates to an array comprising polynucleotides hybridizing to two or more markers as selected from Table A, Table B, Table C or Table D; as selected from CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as selected from CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In particular aspects, the array comprises polynucleotides hybridizing to at least 3, or at least 5, or at least 10, or at least 15, or at least 20, at least 25, at least 30, at least 35, at least 40, at least 45, at least 50, or at least 75 or all of the markers listed in Table A, Table B, Table C or Table D; as listed in the group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as listed in the group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

In another specific aspect, the array comprises polynucleotides hybridizing to the full set of markers listed in Table A, Table B, Table C or Table D; as listed for the group CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37; or as listed for the group CDC2, RFC4, PCNA, CCNE1, CCND1, CDK7, MCM genes (e.g., one or more of MCM3, MCM6, and MCM7), FEN1, MAD2L1, MYBL2, RRM2, and BUB3.

The polynucleotides can be cDNAs, or oligonucleotides, and the solid surface on which they are displayed can be glass, for example. The polynucleotides can hybridize to one or more of the markers as disclosed herein, for example, to the full-length sequences, any coding sequences, any fragments, or any complements thereof.

In still another aspect, the invention relates to a method of predicting the likelihood of long-term survival of a patient diagnosed with cancer, without the recurrence of cancer, comprising the steps of: (1) determining the expression levels of the RNA transcripts or the expression products of the full set or a subset of the markers listed in Table A, Table B, Table C or Table D, herein, in a sample obtained from the patient, normalized against the expression levels of all RNA transcripts or their expression products in the sample, or of a reference set of RNA transcripts or their products; (2) subjecting the data obtained in step (1) to statistical analysis; and (3) determining whether the likelihood of the long-term survival has increased or decreased.

In yet another aspect, the invention concerns a method of preparing a personalized genomics profile for a patient, e.g., a cancer patient, comprising the steps of: (a) subjecting a sample obtained from the patient to expression analysis; (b) determining the expression level of one or more markers selected from the marker set listed in any one of Table A, Table B, Table C or Table D, wherein the expression level is normalized against a control gene or genes and optionally is compared to the amount found in a reference set; and (c) creating a report summarizing the data obtained by the expression analysis. The report may, for example, include prediction of the likelihood of long term survival of the patient and/or recommendation for a treatment modality of the patient.

In additional aspects, the invention relates to a prognostic method comprising: (a) subjecting a sample obtained from a patient to quantitative analysis of the expression level of the RNA transcript of at least one marker selected from Table A, Table B, Table C or Table D, herein, or its product, and (b) identifying the patient as likely to have an increased likelihood of long-term survival without cancer recurrence if the normalized expression levels of the marker or markers, or their products, are above defined expression threshold. In alternate aspects, step (b) comprises identifying the patient as likely to have a decreased likelihood of long-term survival without cancer recurrence if the normalized expression levels of the marker or markers, or their products, are decreased below a defined expression threshold.

In particular, the relatively low expression of proliferation markers is associated with poor outcome. This can include disease progression or the increased likelihood of cancer recurrence, especially for gastrointestinal cancer, such as gastric or colorectal cancer. By contrast, the relatively high expression of proliferation markers is associated with a good outcome. This can include decreased likelihood of cancer recurrence after standard treatment, especially for gastrointestinal cancer, such as gastric or colorectal cancer. Low expression can be determined, for example, by comparison of a test sample (e.g., tumour sample) to samples associated with a positive prognosis. High expression can be determined, for example, by comparison of a test sample (e.g., tumour sample) to samples associated with a negative prognosis.

For example, to obtain a prognosis, a patient's sample (e.g., tumour sample) can be compared to samples with known patient outcome. If the patient's sample shows high expression of GCPMs that is comparable to samples with good outcome, and/or higher than samples with poor outcome, then a positive prognosis is implicated. If the patient's sample shows low expression of GCPMs that is comparable to samples with poor outcome, and/or lower than samples with good outcome, then a negative prognosis is implicated. Alternatively, a patient's sample can be compared to samples of actively proliferating/non-proliferating tumour cells. If the patient's sample shows high expression of GCPMs that is comparable to actively proliferating cells, and/or higher than non-proliferating cells, then a positive prognosis is implicated. If the patient's sample shows low expression of GCPMs that is comparable to non-proliferating cells, and/or lower than actively proliferating cells, then a negative prognosis is implicated.

As further examples, the expression levels of a prognostic signature comprising two or more GCPMs from a patient's sample (e.g., tumour sample) can be compared to samples of recurrent/non-recurrent cancer. If the patient's sample shows increased or decreased expression of CCPMs by comparison to samples of non-recurrent cancer, and/or comparable expression to samples of recurrent cancer, then a negative prognosis is implicated. If the patient's sample shows expression of GCPMs that is comparable to samples of non-recurrent cancer, and/or lower or higher expression than samples of recurrent cancer, then a positive prognosis is implicated.

As one approach, a prediction method can be applied to a panel of markers, for example the panel of GCPMs outlined in Table A, Table B Table C or Table D, in order to generate a predictive model. This involves the generation of a prognostic signature, comprising two or more GCPMs.

The disclosed GCPMs in Table A, Table B, Table C or Table Dtherefore provide a useful set of markers to generate prediction signatures for determining the prognosis of cancer, and establishing a treatment regime, or treatment modality, specific for that tumour. In particular, a positive prognosis can be used by a patient to decide to pursue standard or less invasive treatment options. A negative prognosis can be used by a patient to decide to terminate treatment or to pursue highly aggressive or experimental treatments. In addition, a patient can chose treatments based on their impact on the expression of prognostic markers (e.g., GCPMs).

Levels of GCPMs can be detected in tumour tissue, tissue proximal to the tumour, lymph node samples, blood samples, serum samples, urine samples, or faecal samples, using any suitable technique, and can include, but is not limited to, oligonucleotide probes, quantitative PCR, or antibodies raised against the markers. It will be appreciated that by analyzing the presence and amounts of expression of a plurality of GCPMs in the form of prediction signatures, and constructing a prognostic signature, the sensitivity and accuracy of prognosis will be increased. Therefore, multiple markers according to the present invention can be used to determine the prognosis of a cancer.

The invention includes the use of archived paraffin-embedded biopsy material for assay of the markers in the set, and therefore is compatible with the most widely available type of biopsy material. It is also compatible with several different methods of tumour tissue harvest, for example, via core biopsy or fine needle aspiration. In certain aspects, RNA is isolated from a fixed, wax-embedded cancer tissue specimen of the patient. Isolation may be performed by any technique known in the art, for example from core biopsy tissue or fine needle aspirate cells.

In one aspect, the invention relates to a method of predicting a prognosis, e.g., the likelihood of long-term survival of a cancer patient without the recurrence of cancer, comprising determining the expression level of one or more prognostic markers or their expression products in a sample obtained from the patient, normalized against the expression level of other RNA transcripts or their products in the sample, or of a reference set of RNA transcripts or their expression products. In specific aspects, the prognostic marker is one or more markers listed in Table A, Table B, Table C or Table D or is included as one or more of the prognostic signatures derived from the markers listed in Table A, Table B, Table C or Table D.

In further aspects, the expression levels of the prognostic markers or their expression products are determined, e.g., for the markers listed in Table A, Table B, Table C or Table D, a prognostic signature derived from the markers listed in Table A, Table B, Table C or Table D. In another aspect, the method comprises the determination of the expression levels of a full set of prognosis markers or their expression products, e.g., for the markers listed in Table A, Table B, Table C or Table D, or, a prognostic signature derived from the markers listed in Table A, Table B, Table C or Table D.

In an additional aspect; the invention relates to an array (e.g., microarray) comprising polynucleotides hybridizing to two or more markers, e.g., for the markers listed in Table A, Table B, Table C or Table D, or a prognostic signature derived from the markers listed in Table A, Table B, Table C or Table D. In particular aspects, the array comprises polynucleotides hybridizing to prognostic signature derived from the markers listed in Table A, Table B, Table C or Table D, or e.g., for a prognostic signature. In another specific aspect, the array comprises polynucleotides hybridizing to the full set of markers, e.g., for the markers listed in Table A, Table B, Table C or Table D, or, e.g., for a prognostic signature.

For these arrays, the polynucleotides can be cDNAs, or oligonucleotides, and the solid surface on which they are displayed can be glass, for example. The polynucleotides can hybridize to one or more of the markers as disclosed herein, for example, to the full-length sequences, any coding sequences, any fragments, or any complements thereof. In particular aspects, an increase or decrease in expression levels of one or more GCPM indicates a decreased likelihood of long-term survival, e.g., due to cancer recurrence, while a lack of an increase or decrease in expression levels of one or more GCPM indicates an increased likelihood of long-term survival without cancer recurrence.

In further aspects, the invention relates to a kit comprising one or more of: (1) extraction buffer/reagents and protocol; (2) reverse transcription buffer/reagents and protocol; and (3) quantitative PCR buffer/reagents and protocol suitable for performing any of the foregoing methods. Other aspects and advantages of the invention are illustrated in the description and examples included herein.

**Table A: GCPMs for cell proliferation signature**

| Unique ID | Gene Symbol | Gene Name | GenBank Acc. No. | Gene Aliases |
|---|---|---|---|---|
| A:09020 | CCND1 | cyclin D1 | NM_053056 | BCL1; PRAD1; D11S287E |
| C:0921 | CCNE1 | cyclin E1 | NM_001238, NM_057182 | CCNE |
| A:05382 | CDC2 | cell division cycle 2, G1 to S and G2 to M | NM_001786, NM_033379 | CDK1; MGC111195; DKFZp686L2022 2 |
| A:09842 | CDK7 | cyclin-dependent kinase 7 (MO15 homolog, Xenopus laevis, cdk-activating kinase) | NM_001799 | CAK1; STK1; CDKN7; p39MO15 |
| B:7793 | CHEK1 | CHK1 checkpoint homolog (S. pombe) | NM_001274 | CHK1 |
| A:03447 | CSE1L | CSE1 chromosome segregation 1-like (yeast) | NM_001316 | CAS; CSE1; XPO2; MGC117283; MGC130036; MGC130037 |
| A:05535 | DKC1 | dyskeratosis congenita 1, dyskerin | NM_001363 | DKC; NAP57; NOLA4; XAP101; dyskerin |
| A:07296 | DUT | dUTP pyrophosphatase | NM_001025248, NM_001025249, NM_001948 | dUTPase; FLJ20622 |
| C:2467 | E4F1 | E4F transcription factor 1 | NM_004424 | E4F; MGC99614 |
| B:9065 | FEN1 | flap structure-specific endonuclease 1 | NM_004111 | MF1; RAD2; FEN-1 |
| A:01437 | FH | fumarate hydratase | NM_000143 | MCL; LRCC; HLRCC; MCUL1 |
| B:9714 | XRCC6 | X-ray repair complementing defective repair in Chinese hamster cells 6 (Ku autoantigen, 70kDa) | NM_001469 | ML8; KU70; TLAA; CTC75; CTCBF; G22P1 |
| B:3553_hk-r1 | GPS1 | G protein pathway suppressor 1 | NM_004127, NM_212492 | CSN1; COPS1; MGC71287 |
| B:4036 | KPNA2 | karyopherin alpha 2 (RAG cohort 1, importin alpha 1) | NM_002266 | QIP2; RCH1; IPOA1; SRP1alpha |
| A:06387 | MAD2L1 | MAD2 mitotic arrest deficient-like 1 (yeast) | NM_002358 | MAD2; HSMAD2 |
| A:08668 | MCM3 | MCM3 minichromosome maintenance deficient 3 (S. cerevisiae) | NM_002388 | HCC5; P1.h; RLFB; MGC1157; P1-MCM3 |
| B:8147 | MCM6 | MCM6 minichromosome maintenance deficient 6 (MIS5 homolog, S. pombe) (S. cerevisiae) | NM_005915 | Mis5; P105MCM; MCG40308 |
| B:7620 | MCM7 | MCM7 minichromosome maintenance deficient 7 (S. cerevisiae) | NM_005916, NM_182776 | MCM2; CDC47; P85MCM; P1CDC47; PNAS-146; CDABP0042; P1.1-MCM3 |
| A:10600 | RAB8A | RAB8A, member RAS oncogene family | NM_005370 | MEL; RAB8 |
| A:09470 | KITLG | KIT ligand | NM_000899, NM_003994 | SF; MGF; SCF; KL-1; Kitl; DKFZp686F2250 |
| A:06037 | MYBL2 | v-myb myeloblastosis viral oncogene homolog (avian)-like 2 | NM_002466 | BMYB; MGC15600 |
| A:01677 | NME1 | non-metastatic cells 1, protein (NM23A) expressed in | NM_000269, NM_198175 | AWD; GAAD; NM23; NDPKA; NM23-H1 |
| A:03397 | PRDX1 | peroxiredoxin 1 | NM_002574, NM_181696, NM_181697 | PAG; PAGA; PAGB; MSP23; NKEFA; TDPX2 |
| A:03715 | PCNA | proliferating cell nuclear antigen | NM_002592, NM_182649 | MGC8367 |
| A:02929 | POLD2 | polymerase (DNA directed), delta 2, regulatory subunit 50kDa | NM_006230 | None |
| A:04680 | POLE2 | polymerase (DNA directed), epsilon 2 (p59 subunit) | NM_002692 | DPE2 |
| A:09169 | RAN | RAN, member RAS oncogene family | NM_006325 | TC4; Gsp1; ARA24 |
| A:09145 | RBBP8 | retinoblastoma binding protein 8 | NM_002894, NM_203291, NM_203292 | RIM; CTIP |
| A:09921 | RFC4 | replication factor C (activator 1) 4, 37kDa | NM_002916, NM_181573 | A1; RFC37; MGC27291 |
| A:10597 | RPA1 | replication protein A1, 70kDa | NM_002945 | HSSB; RF-A; RP-A; REPA1; RPA70 |
| A:00231 | RPA3 | replication protein A3, 14kDa | NM_002947 | REPA3 |
| A:09802 | RRM1 | ribonucleotide reductase M1 polypeptide | NM_001033 | R1; RR1; RIR1 |
| B:3501 | RRM2 | ribonucleotide reductase M2 polypeptide | NM_001034 | R2; RR2M |
| A:08332 | S100A5 | S100 calcium binding protein A5 | NM_002962 | S100D |
| A:07314 | FSCN1 | fascin homolog 1, actin-bundling protein (Strongylocentrotus purpuratus) | NM_003088 | SNL; p55; FLJ38511 |
| A:03507 | FOSL1 | FOS-like antigen 1 | NM_005438 | FRA1; fra-1 |
| A:09331 | CDC45L | CDC45 cell division cycle 45-like (S. cerevisiae) | NM_003504 | CDC45; CDC45L2; PORC-PI-1 |
| A:09436 | SMC3 | structural maintenance of chromosomes 3 | NM_005445 | BAM; BMH; HCAP; CSPG6; SMC3L1 |
| A:09747 | BUB3 | BUB3 budding uninhibited by benzimidazoles 3 homolog (yeast) | NM_001007793, NM_004725 | BUB3L; hBUB3 |
| A:00891 | WDR39 | WD repeat domain 39 | NM_004804 | CIAO1 |
| A:05648 | SMC4 | structural maintenance of chromosomes 4 | NM_001002799, NM_001002800, NM_005496 | CAPC; SMC4L1; hCAP-C |
| B:7911 | TOB1 | transducer of ERBB2, 1 | NM_005749 | TOB; TROB; APRO6; PIG49; TROB1; MGC34446; MGC104792 |
| A:04760 | ATG7 | ATG7 autophagy related 7 homolog (S. cerevisiae) | NM_006395 | GSA7; APG7L; DKFZp434N0735 |
| A:04950 | CCT7 | chaperonin containing TCP1, subunit 7 (eta) | NM_001009570, NM_006429 | Ccth; Nip7-1; CCT-ETA; MGC110985; TCP-1-eta |
| A:09500 | CCT2 | chaperonin containing TCP1, subunit 2 (beta) | NM_006431 | CCTB; 99D8.1; PRO1633; CCT-beta; . MGC142074; MGC142076; TCP-1-beta |
| A:03486 | CDC37 | CDC37 cell division cycle 37 homolog (S. cerevisiae) | NM_007065 | P50CDC37 |
| B:7247 | TREX1 | three prime repair exonuclease 1 | NM_016381, NM_032166, NM_033627, NM_033628, NM_033629, NM_130384 | AGS1; DRN3; ATRIP; FLJ12343; DKFZp434J0310 |
| A:01322 | PARK7 | Parkinson disease (autosomal recessive, early onset) 7 | NM_007262 | DJ1; DJ-1; FLJ27376 |
| A:09401 | PREI3 | preimplantation protein 3 | NM_015387, NM_199482 | 2C4D; MOB1; MOB3; CGI-95; MGC12264 |
| A:09724 | MLH3 | mutL homolog 3 (E. coli) | NM_001040108, NM_014381 | HNPCC7; MGC138372 |
| A:02984 | CACYBP | calcyclin binding protein | NM_001007214, NM_014412 | SIP; GIG5; MGC87971; S100A6BP; RP1-102G20.6 |
| A:09821 | MCTS1 | malignant T cell amplified sequence 1 | NM_014060 | MCT1; MCT-1 |
| A:03435 | GMNN | geminin, DNA replication inhibitor | NM_015895 | Gem; RP3-369A17.3 |
| B:1035 | GINS2 | GINS complex subunit 2 (Psf2 homolog) | NM_016095 | PSF2; Pfs2; HSPC037 |
| A:02209 | POLE3 | polymerase (DNA directed), epsilon 3 (p17 subunit) | NM_017443 | p17; YBL1; CHRAC17; CHARAC17 |
| A:05280 | ANLN | anillin, actin binding protein | NM_018685 | scra; Scraps; ANILLIN; DKFZp779A055 |
| A:07468 | SEPT11 | septin 11 | NM_018243 | None |
| A:03912 | PBK | PDZ binding kinase | NM_018492 | SPK; TOPK; Nori-3; FLJ14385 |
| B:8449 | BCCIP | BRCA2 and CDKN1A interacting protein | NM_016567, NM_078468, NM_078469 | TOK-1 |
| B:2392 | DBF4B | DBF4 homolog B (S. cerevisiae) | NM_025104, NM_145663 | DRF1; ASKL1; FLJ13087; MGC15009 |
| B:6501 | CD276 | CD276 molecule | NM_001024736, NM_025240 | B7H3; B7-H3 |
| B:5467 | LAMA1 | laminin, alpha 1 | NM_005559 | LAMA |

**Table A: Proliferation-related genes differentially expressed between cell lines in high and low proliferative states.** Genes that were differentially expressed between cell lines in confluent (low proliferation) and semi-confluent (high proliferation) states (see Figure 1) were identified by microarray analysis on 30K MWG Biotech arrays. Table A comprises the subset of these genes that were categorized by gene ontology analysis as cell proliferation-related.

**Table B: GCPMs for cell proliferation signature**

| Unique ID | Gene Description | LocusLink | GenBank Accession |
|---|---|---|---|
| B:7560 | v-abl Abelson murine leukaemia viral oncogene homolog 1 (ABL1), transcript variant a, mRNA | 25 | NM_005157 |
| A:09071 | acetylcholinesterase (YT blood group) (ACHE), transcript variant E4-E5, mRNA | 43 | NM_015831, NM_000665 |
| A:04114 | acid phosphatase 2, lysosomal (ACP2), mRNA | 53 | NM_001610 |
| A:09146 | acid phosphatase, prostate (ACPP), mRNA | 55 | NM_001099 |
| A:09585 | adrenergic, alpha-1D-, receptor (ADRA1D), mRNA | 146 | NM_000678 |
| A:08793 | adrenergic, alpha-1B-, receptor (ADRA1B), mRNA | 147 | NM_000679 |
| C:0326 | adrenergic, alpha-1A-, receptor (ADRA1A), transcript variant 4, mRNA | 148 | NM_033304 |
| A:02272 | adrenergic, alpha-2A-, receptor (ADRA2A), mRNA | 150 | NM_000681 |
| A:05807 | jagged 1 (Alagille syndrome) (JAG1), mRNA | 182 | NM_000214 |
| A:02268 | aryl hydrocarbon receptor (AHR), mRNA | 196 | NM_001621 |
| A:00978 | allograft inflammatory factor 1 (AIF1), transcript variant 2, mRNA | 199 | NM_004847 |
| A:06335 | adenylate kinase 1 (AK1), mRNA | 203 | NM_000476 |
| A:07028 | v-akt murine thymoma viral oncogene homolog 1 (AKT1), transcript variant 1, mRNA | 207 | NM_005163 |
| A:05949 | v-akt murine thymoma viral oncogene homolog 2 (AKT2), mRNA | 208 | NM_001626 |
| B:9542 | arachidonate 15-lipoxygenase, second type (ALOX15B), mRNA | 247 | NM_001141 |
| A:02569 | bridging integrator 1 (BIN1), transcript variant 8, mRNA | 274 | NM_004305 |
| C:0393 | amyloid beta (A4) precursor protein-binding, family B, member 1 (APBB1), transcript variant 1, mRNA | 322 | NM_001164 |
| B:5288 | amyloid beta (A4) precursor protein-binding, family B, member 2 (Fe65-like) (APBB2), mRNA | 323 | NM_173075 |
| A:09151 | adenomatosis polyposis coli (APC), mRNA | 324 | NM_000038 |
| B:3616 | baculoviral IAP repeat-containing 5 (survivin) (BIRC5), transcript variant 1, mRNA | 332 | NM_001168 |
| C:2007 | androgen receptor (dihydrotestosterone receptor; testicular feminization; spinal and bulbar muscular atrophy; Kennedy disease) (AR), transcript variant 2, mRNA | 367 | NM_001011645 |
| A:04819 | amphiregulin (schwannoma-derived growth factor) (AREG), mRNA | 374 | NM_001657 |
| A:01709 | ras homolog gene family, member G (rho G) (RHOG), mRNA | 391 | NM_001665 |
| B:6554 | ataxia telangiectasia mutated (includes complementation groups A, C and D) (ATM), transcript variant 1, mRNA | 472 | NM_000051 |
| A:02418 | ATPase, Cu++ transporting, beta polypeptide (ATP7B), transcript variant 1, mRNA | 545 | NM_000053 |
| A:05997 | AXL receptor tyrosine kinase (AXL), transcript variant 2, mRNA | 558 | NM_001699 |
| B:0073 | brain-specific angiogenesis inhibitor 1 (BAI1), mRNA | 575 | NM_001702 |
| A:07209 | BCL2-associated X protein (BAX), transcript variant beta, mRNA | 581 | NM_004324 |
| B:1845 | Bardet-Biedl syndrome 4 (BBS4), mRNA | 586 | NM_033028 |
| A:00571 | branched chain aminotransferase 2, mitochondrial (BCAT2), mRNA | 588 | NM_001190 |
| A:09020 | cyclin D1 (CCND1), mRNA | 595 | NM_053056 |
| A:10775 | B-cell CLL/lymphoma 2 (BCL2), nuclear gene encoding mitochondrial protein, transcript variant alpha, mRNA | 596 | NM_000633 |
| A:09014 | B-cell CLL/lymphoma 3 (BCL3), mRNA | 602 | NM_005178 |
| C:2412 | B-cell CLL/lymphoma 6 (zinc finger protein 51) (BCL6), transcript variant 1, mRNA | 604 | NM_001706 |
| A:08794 | tumour necrosis factor receptor superfamily, member 17 (TNFRSF17), mRNA | 608 | NM_001192 |
| A:01162 | Bloom syndrome (BLM), mRNA | 641 | NM_000057 |
| B:5276 | basonuclin 1 (BNC1), mRNA | 646 | NM_001717 |
| B:3766 | polymerase (RNA) III (DNA directed) polypeptide D, 44kDa (POLR3D), mRNA | 661 | NM_001722 |
| C:2188 | dystonin (DST), transcript variant 1, mRNA | 667 | NM_183380 |
| B:5103 | breast cancer 1, early onset (BRCA1), transcript variant BRCA1a, mRNA | 672 | NM_007294 |
| A:03676 | breast cancer 2, early onset (BRCA2), mRNA | 675 | NM_000059 |
| A:07404 | zinc finger protein 36, C3H type-like 1 (ZFP36L1), mRNA | 677 | NM_004926 |
| B:5146 | zinc finger protein 36, C3H type-like 2 (ZFP36L2), mRNA | 678 | NM_006887 |
| B:4758 | bone marrow stromal cell antigen 2 (BST2), mRNA | 684 | NM_004335 |
| B:4642 | betacellulin (BTC), mRNA | 685 | NM_001729 |
| C:2483 | B-cell translocation gene 1, anti-proliferative (BTG1), mRNA | 694 | NM_001731 |
| B:0618 | BUB1 budding uninhibited by benzimidazoles 1 homolog (BUB1), mRNA | 699 | NM_004336 |
| A:09398 | BUB1 budding uninhibited by benzimidazoles 1 homolog beta (yeast) (BUB1 B), mRNA | 701 | NM_001211 |
| A:01104 | chromosome 8 open reading frame 1 (C8orf1), mRNA | 734 | NM_004337 |
| B:3828 | calmodulin 2 (phosphorylase kinase, delta) (CALM2), mRNA | 805 | NM_001743 |
| B:6851 | calpain 1, (mu/l) large subunit (CAPN1), mRNA | 823 | NM_005186 |
| A:09763 | calpain, small subunit 1 (CAPNS1), transcript variant 1, mRNA | 826 | NM_001749 |
| B:0205 | core-binding factor, runt domain, alpha subunit 2; translocated to, 3 (CBFA2T3), transcript variant 2, mRNA | 863 | NM_175931 |
| B:2901 | runt-related transcription factor 3 (RUNX3), transcript variant 2, mRNA | 864 | NM_004350 |
| A:01132 | cholecystokinin B receptor (CCKBR), mRNA | 887 | NM_176875 |
| A:04253 | cyclin A2 (CCNA2), mRNA | 890 | NM_001237 |
| A:04253 | cyclin A2 (CCNA2), mRNA | 891 | NM_001237 |
| A:09352 | cyclin C (CCNC), transcript variant 1, mRNA | 892 | NM_005190 |
| A:10559 | cyclin D2 (CCND2), mRNA | 894 | NM_001759 |
| A:02240 | cyclin D3 (CCND3), mRNA | 896 | NM_001760 |
| C:0921 | cyclin E1 (CCNE1), transcript variant 1, mRNA | 898 | NM_001238 |
| C:0921 | cyclin E1 (CCNE1), transcript variant 1, mRNA | 899 | NM_001238 |
| B:5261 | cyclin G1 (CCNG1), transcript variant 1, mRNA | 900 | NM_004060 |
| A:07154 | cyclin G2 (CCNG2), mRNA | 901 | NM_004354 |
| A:07930 | cyclin H (CCNH), mRNA | 902 | NM_001239 |
| A:01253 | cyclin T1 (CCNT1), mRNA | 904 | NM_001240 |
| B:0645 | cyclin T2 (CCNT2), transcript variant b, mRNA | 905 | NM_058241 |
| C:2676 | CD3E antigen, epsilon polypeptide (TiT3 complex) (CD3E), mRNA | 916 | NM_000733 |
| A:10068 | CD5 antigen (p56-62) (CD5), mRNA | 921 | NM_014207 |
| A:07504 | tumour necrosis factor receptor superfamily, member 7 (TNFRSF7), mRNA | 939 | NM_001242 |
| A:05558 | CD28 antigen (Tp44) (CD28), mRNA | 940 | NM_006139 |
| A:07387 | CD86 antigen (CD28 antigen ligand 2, B7-2 antigen) (CD86), transcript variant 1, mRNA | 942 | NM_175862 |
| A:06344 | tumour necrosis factor receptor superfamily, member 8 (TNFRSF8), transcript variant 1, mRNA | 943 | NM_001243 |
| A:03064 | tumour necrosis factor (ligand) superfamily, member 8 (TNFSF8), mRNA | 944 | NM_001244 |
| A:03802 | CD33 antigen (gp67) (CD33), mRNA | 945 | NM_001772 |
| A:07407 | CD40 antigen (TNF receptor superfamily member 5) (CD40), transcript variant 1, mRNA | 958 | NM_001250 |
| B:9757 | CD40 ligand (TNF superfamily, member 5, hyper-IgM syndrome) (CD40LG), mRNA | 959 | NM_000074 |
| A:07070 | CD68 antigen (CD68), mRNA | 968 | NM_001251 |
| A:04715 | tumour necrosis factor (ligand) superfamily, member 7 (TNFSF7), mRNA | 970 | NM_001252 |
| A:09638 | CD81 antigen (target of antiproliferative antibody 1) (CD81), mRNA | 975 | NM_004356 |
| A:05382 | cell division cycle 2, G1 to S and G2 to M (CDC2), transcript variant 1, mRNA | 983 | NM_001786 |
| A:00282 | cell division cycle 2-like 1 (PITSLRE proteins) (CDC2L1), transcript variant 2, mRNA | 984 | NM_033486 |
| A:00282 | cell division cycle 2-like 1 (PITSLRE proteins) (CDC2L1), transcript variant 2, mRNA | 985 | NM_033486 |
| A:07718 | CDC5 cell division cycle 5-like (S. pombe) (CDC5L), mRNA | 988 | NM_001253 |
| A:00843 | septin 7 (SEPT7), transcript variant 1, mRNA | 989 | NM_001788 |
| A:05789 | CDC6 cell division cycle 6 homolog (S. cerevisiae) (CDC6), mRNA | 990 | NM_001254 |
| A:03063 | CDC20 cell division cycle 20 homolog (S. cerevisiae) (CDC20), mRNA | 991 | NM_001255 |
| B:4185 | cell division cycle 25A (CDC25A), transcript variant 1, mRNA | 993 | NM_001789 |
| A:04022 | cell division cycle 25B (CDC25B), transcript variant 3, mRNA | 994 | NM_021873 |
| B:9539 | cell division cycle 25C (CDC25C), transcript variant 1, mRNA | 995 | NM_001790 |
| B:5590 | cell division cycle 27 CDC27 | 996 | NM_001256 |
| B:9041 | cell division cycle 34 (CDC34), mRNA | 997 | NM_004359 |
| A:03518 | cyclin-dependent kinase 2 (CDK2), transcript variant 2, mRNA | 1017 | NM_052827 |
| A:02068 | cyclin-dependent kinase 3 (CDK3), mRNA | 1018 | NM_001258 |
| B:4838 | cyclin-dependent kinase 4 (CDK4), mRNA | 1019 | NM_000075 |
| A:10302 | cyclin-dependent kinase 5 (CDK5), mRNA | 1020 | NM_004935 |
| A:01923 | cyclin-dependent kinase 6 (CDK6), mRNA | 1021 | NM_001259 |
| A:09842 | cyclin-dependent kinase 7 (MO15 homolog, Xenopus laevis, cdk-activating kinase) (CDK7), mRNA | 1022 | NM_001799 |
| A:08302 | cyclin-dependent kinase 8 (CDK8), mRNA | 1024 | NM_001260 |
| A:05151 | cyclin-dependent kinase 9 (CDC2-related kinase) (CDK9), | 1025 | NM_001261 |
| A:09736 | cyclin-dependent kinase inhibitor 1A (p21, Cip1) (CDKN1A), transcript variant 2, mRNA | 1026 | NM_078467 |
| A:05571 | cyclin-dependent kinase inhibitor 1 B (p27, Kip1) (CDKN1B), mRNA | 1027 | NM_004064 |
| A:08441 | cyclin-dependent kinase inhibitor 1C (p57, Kip2) (CDKN1C), mRNA | 1028 | NM_000076 |
| B:9782 | cyclin-dependent kinase inhibitor 2A (melanoma, p16, inhibits CDK4) (CDKN2A), transcript variant 4, mRNA | 1029 | NM_058195 |
| C:6459 | cyclin-dependent kinase inhibitor 2B (p15, inhibits CDK4) (CDKN2B), transcript variant 1, mRNA | 1030 | NM_004936 |
| B:0604 | cyclin-dependent kinase inhibitor 2C (p18, inhibits CDK4) (CDKN2C), transcript variant 1, mRNA | 1031 | NM_001262 |
| A:03310 | cyclin-dependent kinase inhibitor 2D (p19, inhibits CDK4) (CDKN2D), transcript variant 2, mRNA | 1032 | NM_079421 |
| A:05799 | cyclin-dependent kinase inhibitor 3 (CDK2-associated dual specificity phosphatase) (CDKN3), mRNA | 1033 | NM_005192 |
| B:9170 | centromere protein B, 80kDa (CENPB), mRNA | 1059 | NM_001810 |
| A:07769 | centromere protein E, 312kDa (CENPE), mRNA | 1062 | NM_001813 |
| A:06471 | centromere protein F, 350/400ka (mitosin) (CENPF), mRNA | 1063 | NM_016343 |
| A:03128 | centrin, EF-hand protein, 1 (CETN1), mRNA | 1068 | NM_004066 |
| A:05554 | centrin, EF-hand protein, 2 (CETN2), mRNA | 1069 | NM_004344 |
| B:4016 | centrin, EF-hand protein, 3 (CDC31 homolog, yeast) (CETN3), mRNA | 1070 | NM_004365 |
| B:5082 | regulator of chromosome condensation 1 RCC1 | 1104 | NM_001048194, NM_001048195, NM_001269 |
| B:7793 | CHK1 checkpoint homolog (S. pombe) (CHEK1), mRNA | 1111 | NM_001274 |
| B:8504 | checkpoint suppressor 1 (CHES1), mRNA | 1112 | NM_005197 |
| A:00320 | cholinergic receptor, muscarinic 1 (CHRM1), mRNA | 1128 | NM_000738 |
| A:10168 | cholinergic receptor, muscarinic 3 (CHRM3), mRNA | 1131 | NM_000740 |
| A:06655 | cholinergic receptor, muscarinic 4 (CHRM4), mRNA | 1132 | NM_000741 |
| A:00869 | cholinergic receptor, muscarinic 5 (CHRM5), mRNA | 1133 | NM_012125 |
| C:0649 | CDC28 protein kinase regulatory subunit 1 B (CKS1 B), mRNA | 1163 | NM_001826 |
| B:6912 | CDC28 protein kinase regulatory subunit 2 (CKS2), mRNA | 1164 | NM_001827 |
| A:07840 | CDC-like kinase 1 (CLK1), transcript variant 1, | 1195 | NM_004071 |
| B:8665 | polo-like kinase 3 (Drosophila) (PLK3), mRNA | 1263 | NM_004073 |
| B:8651 | collagen, type IV, alpha 3 (Goodpasture antigen) (COL4A3), transcript variant 1, mRNA | 1285 | NM_000091 |
| B:4734 | mitogen-activated protein kinase 8 (MAP3K8), mRNA | 1326 | NM_005204 |
| B:3778 | cysteine-rich protein 1 (intestinal) (CRIP1), mRNA | 1396 | NM_001311 |
| B:3581 | cysteine-rich protein 2 (CRIP2), mRNA | 1397 | NM_001312 |
| B:5543 | v-crk sarcoma virus CT10 oncogene homolog (avian) (CRK), transcript variant I, mRNA | 1398 | NM_005206 |
| B:6254 | v-crk sarcoma virus CT10 oncogene homolog (avian)-like (CRKL), mRNA | 1399 | NM_005207 |
| A:03447 | CSE1 chromosome segregation 1-like (yeast) (CSE1 L), transcript variant 2, mRNA | 1434 | NM_177436 |
| A:10730 | colony stimulating factor 1 (macrophage) (CSF1), transcript variant 2, mRNA | 1435 | NM_172210 |
| A:05457 | colony stimulating factor 1 receptor, formerly McDonough feline sarcoma viral (v-fms) oncogene homolog (CSF1R), mRNA | 1436 | NM_005211 |
| B:1908 | colony stimulating factor 3 (granulocyte) (CSF3), transcript variant 2, mRNA | 1440 | NM_172219 |
| A:01629 | c-src tyrosine kinase (CSK), mRNA | 1445 | NM_004383 |
| A:07097 | casein kinase 2, alpha prime polypeptide (CSNK2A2), mRNA | 1459 | NM_001896 |
| B:3639 | cysteine and glycine-rich protein 2 (CSRP2), mRNA | 1466 | NM_001321 |
| B:8929 | C-terminal binding protein 1 CTBP1 | 1487 | NM_001012614, NM_001328 |
| A:08689 | C-terminal binding protein 2 (CTBP2), transcript variant 1, mRNA | 1488 | NM_001329 |
| A:02604 | cardiotrophin 1 (CTF1), mRNA | 1489 | NM_001330 |
| A:05018 | disabled homolog 2, mitogen-responsive phosphoprotein (Drosophila) (DAB2), mRNA | 1601 | NM_001343 |
| A:09374 | deleted in colorectal carcinoma (DCC), mRNA | 1630 | NM_005215 |
| A:05576 | dynactin 1 (p150, glued homolog, Drosophila) (DCTN1), transcript variant 1, mRNA | 1639 | NM_004082 |
| A:04346 | growth arrest and DNA-damage-inducible, alpha (GADD45A), mRNA | 1647 | NM_001924 |
| B:9526 | DNA-damage-inducible transcript 3 (DDIT3), mRNA | 1649 | NM_004083 |
| B:6726 | DEAD/H (Asp-Glu-Ala-Asp/His) box polypeptide 11 (CHL1-like helicase homolog, S. cerevisiae) (DDX11), transcript variant 1, mRNA | 1663 | NM_030653 |
| B:1955 | deoxyhypusine synthase (DHPS), transcript variant 1, mRNA | 1725 | NM_001930 |
| A:09887 | diaphanous homolog 2 (Drosophila) (DIAPH2), transcript variant 12C, mRNA | 1730 | NM_007309 |
| B:4704 | septin 1 (SEPT1), mRNA | 1731 | NM_052838 |
| A:05535 | dyskeratosis congenita 1, dyskerin (DKC1), mRNA | 1736 | NM_001363 |
| A:06695 | discs, large homolog 3 (neuroendocrine-dlg, Drosophila) (DLG3), mRNA | 1741 | NM_021120 |
| B:9032 | dystrophia myotonica-containing WD repeat motif (DMWD), mRNA | 1762 | NM_004943 |
| B:4936 | DNA2 DNA replication helicase 2-like (yeast) (DNA2L), mRNA | 1763 | XM_166103, XM_938629 |
| B:5286 | dynein, cytoplasmic 1, heavy chain 1 (DYNC1H1), mRNA | 1778 | NM_001376 |
| B:9089 | dynamin 2 (DNM2), transcript variant 4, mRNA | 1785 | NM_001005362 |
| A:05674 | deoxynucleotidyltransferase, terminal (DNTT), transcript variant 1, mRNA | 1791 | NM_004088 |
| A:00269 | heparin-binding EGF-like growth factor (HBEGF), mRNA | 1839 | NM_001945 |
| B:3724 | deoxythymidylate kinase (thymidylate kinase) (DTYMK), mRNA | 1841 | NM_012145 |
| A:01114 | dual specificity phosphatase 1 (DUSP1), mRNA | 1843 | NM_004417 |
| A:08044 | dual specificity phosphatase 4 (DUSP4), transcript variant 2, mRNA | 1846 | NM_057158 |
| B:0206 | dual specificity phosphatase 6 (DUSP6), transcript variant 1, mRNA | 1848 | NM_001946 |
| A:07296 | dUTP pyrophosphatase (DUT), nuclear gene encoding mitochondrial protein, transcript variant 2, mRNA | 1854 | NM_001948 |
| B:5540 | E2F transcription factor 1 (E2F1), mRNA | 1869 | NM_005225 |
| B:4216 | E2F transcription factor 2 (E2F2), mRNA | 1870 | NM_004091 |
| B:6451 | E2F transcription factor 3 (E2F3), mRNA | 1871 | NM_001949 |
| A:03567 | E2F transcription factor 4, p107/p130-binding (E2F4), mRNA | 1874 | NM_001950 |
| C:2484 | E2F transcription factor 5, p130-binding (E2F5), mRNA | 1875 | NM_001951 |
| B:9807 | E2F transcription factor 6 (E2F6), transcript variant a, mRNA | 1876 | NM_001952 |
| C:2467 | E4F transcription factor 1 (E4F1), mRNA | 1877 | NM_004424 |
| A:04592 | endothelial cell growth factor 1 (platelet-derived) (ECGF1), mRNA | 1890 | NM_001953 |
| A:00257 | endothelial differentiation, lysophosphatidic acid G-protein-coupled receptor, 2 (EDG2), transcript variant 1, mRNA | 1903 | NM_001401 |
| A:08155 | endothelin 1 (EDN1), mRNA | 1906 | NM_001955 |
| A:08447 | endothelin receptor type A (EDNRA), mRNA | 1909 | NM_001957 |
| A:09410 | epidermal growth factor (beta-urogastrone) (EGF), mRNA | 1950 | NM_001963 |
| A:10005 | epidermal growth factor receptor (erythroblastic leukaemia viral (v-erb-b) oncogene homolog, avian) (EGFR), transcript variant 1, mRNA | 1956 | NM_005228 |
| A:03312 | early growth response 4 (EGR4), mRNA | 1961 | NM_001965 |
| A:06719 | eukaryotic translation initiation factor 4 gamma, 2 (EIF4G2), mRNA | 1982 | NM_001418 |
| A:10651 | E74-like factor 5 (ets domain transcription factor) (ELF5), transcript variant 2, mRNA | 2001 | NM_001422 |
| A:07972 | ELK3, ETS-domain protein (SRF accessory protein 2) (ELK3), mRNA | 2004 | NM_005230 |
| A:06224 | elastin (supravalvular aortic stenosis, Williams-Beuren syndrome) (ELN), mRNA | 2006 | NM_000501 |
| A:10267 | epithelial membrane protein 1 (EMP1), mRNA | 2012 | NM_001423 |
| A:09610 | epithelial membrane protein 2 (EMP2), mRNA | 2013 | NM_001424 |
| A:00767 | epithelial membrane protein 3 (EMP3), mRNA | 2014 | NM_001425 |
| A:07219 | glutamyl aminopeptidase (aminopeptidase A) (ENPEP), mRNA | 2028 | NM_001977 |
| A:10199 | E1A binding protein p300 (EP300), mRNA | 2033 | NM_001429 |
| A:10325 | EPH receptor B4 (EPHB4), mRNA | 2050 | NM_004444 |
| A:04352 | glutamyl-prolyl-tRNA synthetase (EPRS), mRNA | 2059 | NM_004446 |
| A:04352 | glutamyl-prolyl-tRNA synthetase (EPRS), mRNA | 2060 | MM_004446 |
| A:08200 | nuclear receptor subfamily 2, group F, member 6 (NR2F6), | 2063 | NM_005234 |
| B:1429 | v-erb-b2 erythroblastic leukaemia viral oncogene homolog 2, neuro/glioblastoma derived oncogene homolog (avian) ERBB2 | 2064 | NM_001005862, NM_004448 |
| A:02313 | v-erb-a erythroblastic leukaemia viral oncogene homolog 4 (avian) (ERBB4), mRNA | 2066 | NM_005235 |
| A:08898 | epiregulin (EREG), mRNA | 2069 | NM_001432 |
| A:07916 | Ets2 repressor factor (ERF), mRNA | 2077 | NM_006494 |
| B:9779 | v-ets erythroblastosis virus E26 oncogene like (avian) (ERG), transcript variant 1, mRNA | 2078 | NM_182918 |
| C:2388 | enhancer of rudimentary homolog (Drosophila) (ERH), mRNA | 2079 | NM_004450 |
| B:5360 | endogenous retroviral sequence K(C4), 2 ERVK2 | 2087 | U87595 |
| C:2799 | estrogen receptor 1 (ESR1), mRNA | 2099 | NM_000125 |
| A:01596 | v-ets erythroblastosis virus E26 oncogene homolog 1 (avian) (ETS1), mRNA | 2113 | NM_005238 |
| A:07704 | v-ets erythroblastosis virus E26 oncogene homolog 2 (ETS2), mRNA | 2114 | NM_005239 |
| A:00924 | ecotropic viral integration site 2A (EVI2A), transcript variant 2, mRNA | 2123 | NM_014210 |
| A:07732 | exostoses (multiple) 1 (EXT1), mRNA | 2131 | NM_000127 |
| A:10493 | exostoses (multiple) 2 (EXT2), transcript variant 1, mRNA | 2132 | NM_000401 |
| A:07741 | coagulation factor II (thrombin) (F2), mRNA | 2147 | NM_000506 |
| A:06727 | coagulation factor II (thrombin) receptor (F2R), mRNA | 2149 | NM_001992 |
| A:10554 | fatty acid binding protein 3, muscle and heart (mammary-derived growth inhibitor) (FABP3), mRNA | 2170 | NM_004102 |
| A:10780 | fatty acid binding protein 5 (psoriasis-associated) (FABP5), mRNA | 2172 | NM_001444 |
| B:9700 | fatty acid binding protein 7, brain FABP7 | 2173 | NM_001446 |
| C:2632 | PTK2B protein tyrosine kinase 2 beta (PTK2B), transcript variant 1, mRNA | 2185 | NM_173174 |
| A:07570 | Fanconi anemia, complementation group G (FANCG), mRNA | 2189 | NM_004629 |
| A:08248 | membrane-spanning 4-domains, subfamily A, member 2 (Fc fragment of IgE, high affinity I, receptor for; beta polypeptide) (MS4A2), mRNA | 2206 | NM_000139 |
| B:9065 | flap structure-specific endonuclease 1 (FEN1), mRNA | 2237 | NM_004111 |
| A:10689 | glypican 4 (GPC4), mRNA | 2239 | NM_001448 |
| B:7897 | fer (fps/fes related) tyrosine kinase (phosphoprotein NCP94) (FER), mRNA | 2242 | NM_005246 |
| B:1852 | fibrinogen alpha chain (FGA), transcript variant alpha-E, mRNA | 2243 | NM_000508 |
| B:1909 | fibrinogen beta chain (FGB), mRNA | 2244 | NM_005141 |
| A:07894 | fibroblast growth factor 1 (acidic) (FGF1), transcript variant 1, mRNA | 2246 | NM_000800 |
| B:7727 | fibroblast growth factor 2 (basic) (FGF2), mRNA | 2247 | NM_002006 |
| A:01551 | fibroblast growth factor 3 (murine mammary tumour virus integration site (v-int-2) oncogene homolog) (FGF3), mRNA | 2248 | NM_005247 |
| A:10568 | fibroblast growth factor 4 (heparin secretory transforming protein 1, Kaposi sarcoma oncogene) (FGF4), mRNA | 2249 | NM_002007 |
| C:2679 | fibroblast growth factor 5 (FGF5), transcript variant 2, mRNA | 2250 | NM_033143 |
| A:04438 | fibroblast growth factor 6 (FGF6), mRNA | 2251 | NM_020996 |
| C:2713 | fibroblast growth factor 7 (keratinocyte growth factor) (FGF7), mRNA | 2252 | NM_002009 |
| B:8151 | fibroblast growth factor 8 (androgen-induced) (FGF8), transcript variant B, mRNA | 2253 | NM_006119 |
| A:10353 | fibroblast growth factor 9 (glia-activating factor) (FGF9), mRNA | 2254 | NM_002010 |
| A:10837 | fibroblast growth factor 10 (FGF10), mRNA | 2255 | NM_004465 |
| B:1815 | fibrinogen gamma chain (FGG), transcript variant gamma-B, mRNA | 2266 | NM_021870 |
| A:01437 | fumarate hydratase (FH), nuclear gene encoding mitochondrial protein, mRNA | 2271 | NM_000143 |
| A:04648 | fragile histidine triad gene (FHIT), mRNA | 2272 | NM_002012 |
| B:1938 | c-fos induced growth factor (vascular endothelial growth factor D) (FIGF), mRNA | 2277 | NM_004469 |
| B:5100 | fms-related tyrosine kinase 1 (vascular endothelial growth factor/vascular permeability factor receptor) FLT1 | 2321 | NM_002019 |
| A:05859 | fms-related tyrosine kinase 3 (FLT3), mRNA | 2322 | NM_004119 |
| A:05362 | fms-related tyrosine kinase 3 ligand (FLT3LG), mRNA | 2323 | NM_001459 |
| A:05281 | v-fos FBJ murine osteosarcoma viral oncogene homolog (FOS), mRNA | 2353 | NM_005252 |
| A:01965 | FBJ murine osteosarcoma viral oncogene homolog B (FOSB), mRNA | 2354 | NM_006732 |
| A:01738 | fyn-related kinase (FRK), mRNA | 2444 | NM_002031 |
| A:03614 | FK506 binding protein 12-rapamycin associated protein 1 (FRAP1), mRNA | 2475 | NM_004958 |
| A:08973 | ferritin, heavy polypeptide 1 (FTH1), mRNA | 2495 | NM_002032 |
| A:03646 | FYN oncogene related to SRC, FGR, YES (FYN), transcript variant 1, mRNA | 2534 | NM_002037 |
| B:9714 | X-ray repair complementing defective repair in Chinese hamster cells 6 (Ku autoantigen, 70kDa) (XRCC6), mRNA | 2547 | NM_001469 |
| A:02378 | GRB2-associated binding protein 1 (GAB1), transcript variant 2, mRNA | 2549 | NM_002039 |
| A:07229 | cyclin G associated kinase (GAK), mRNA | 2580 | NM_005255 |
| B:9019 | growth arrest-specific 1 (GAS1), mRNA | 2619 | NM_002048 |
| B:9019 | growth arrest-specific 1 (GAS1), mRNA | 2620 | NM_002048 |
| B:9020 | growth arrest-specific 6 (GAS6), mRNA | 2621 | NM_000820 |
| A:10093 | growth arrest-specific 8 (GAS8), mRNA | 2622 | NM_001481 |
| A:09801 | glucagon (GCG), mRNA | 2641 | NM_002054 |
| A:09968 | nuclear receptor subfamily 6, group A, member 1 (NR6A1), transcript variant 3, mRNA | 2649 | NM_033335 |
| B:4833 | growth factor, augmenter of liver regeneration (ERV1 homolog, S. cerevisiae) (GFER), mRNA | 2671 | NM_005262 |
| A:08908 | growth factor independent 1 (GFI1), mRNA | 2672 | NM_005263 |
| A:02108 | GPI anchored molecule like protein (GML), mRNA | 2765 | NM_002066 |
| A:05004 | gonadotropin-releasing hormone 1 (luteinizing-releasing hormone) (GNRH1), mRNA | 2796 | NM_000825 |
| B:4823 | stratifin (SFN), mRNA | 2810 | NM_006142 |
| B:3553_hk-r1 | G protein pathway suppressor 1 (GPS1), transcript variant 1, mRNA | 2873 | NM_212492 |
| A:04124 | G protein pathway suppressor 2 (GPS2), mRNA | 2874 | NM_004489 |
| A:05918 | granulin (GRN), transcript variant 1, mRNA | 2896 | NM_002087 |
| C:0852 | glucocorticoid receptor DNA binding factor 1 GRLF1 | 2909 | NM_004491 |
| A:04681 | chemokine (C-X-C motif) ligand 1 (melanoma growth stimulating activity, alpha) (CXCL1), mRNA | 2919 | NM_001511 |
| A:07763 | gastrin-releasing peptide receptor (GRPR), mRNA | 2925 | NM_005314 |
| B:9294 | glycogen synthase kinase 3 beta (GSK3B), mRNA | 2932 | NM_002093 |
| A:07312 | G1 to S phase transition 1 (GSPT1), mRNA | 2935 | NM_002094 |
| A:09859 | mutS homolog 6 (E. coli) (MSH6), mRNA | 2956 | NM_000179 |
| A:04525 | general transcription factor IIH, polypeptide 1 (62kD subunit) (GTF2H1), mRNA | 2965 | NM_005316 |
| B:9176 | hepatoma-derived growth factor (high-mobility group protein 1-like) (HDGF), mRNA | 3068 | NM_004494 |
| B:8961 | hepatocyte growth factor (hepapoietin A; scatter factor) (HGF), transcript variant 3, mRNA | 3082 | NM_001010932 |
| A:05880 | hematopoietically expressed homeobox (HHEX), mRNA | 3090 | NM_002729 |
| A:05673 | hexokinase 2 (HK2), mRNA | 3099 | NM_000189 |
| A:10377 | high-mobility group box 1 (HMGB1), mRNA | 3146 | NM_002128 |
| A:07252 | solute carrier family 29 (nucleoside transporters), member 2 (SLC29A2), mRNA | 3177 | NM_001532 |
| A:04416 | heterogeneous nuclear ribonucleoprotein L (HNRPL), transcript variant 1, mRNA | 3191 | NM_001533 |
| C:1926 | homeo box C10 (HOXC10), mRNA | 3226 | NM_017409 |
| A:08912 | homeo box D13 (HOXD13), mRNA | 3239 | NM_000523 |
| A:05637 | v-Ha-ras Harvey rat sarcoma viral oncogene homolog (HRAS), transcript variant 1, mRNA | 3265 | NM_005343 |
| A:08143 | heat shock 70kDa protein 1A (HSPA1A), mRNA | 3304 | NM_005345 |
| A:05469 | heat shock 70kDa protein 2 (HSPA2), mRNA | 3306 | NM_021979 |
| A:09246 | 5-hydroxytryptamine (serotonin) receptor 1A (HTR1A), mRNA | 3350 | NM_000524 |
| A:07300 | HUS1 checkpoint homolog (S. pombe) (HUS1), | 3364 | NM_004507 |
| B:7639 | interferon, gamma-inducible protein 16 IFI16 | 3428 | NM_005531 |
| A:04388 | interferon, beta 1, fibroblast (IFNB1), mRNA | 3456 | NM_002176 |
| A:02473 | interferon, omega 1 (IFNW1), mRNA | 3467 | NM_002177 |
| B:5220 | insulin-like growth factor 1 (somatomedin C) | 3479 | NM_000618 |
| C:0361 | insulin-like growth factor 1 receptor IGF1R | 3480 | NM_000875 |
| B:5688 | insulin-like growth factor 2 (somatomedin A) (IGF2), mRNA | 3481 | NM_000612 |
| A:09232 | insulin-like growth factor binding protein 4 (IGFBP4), mRNA | 3487 | NM_001552 |
| A:02232 | insulin-like growth factor binding protein 6 (IGFBP6), mRNA | 3489 | NM_002178 |
| A:03385 | insulin-like growth factor binding protein 7 (IGFBP7), mRNA | 3490 | NM_001553 |
| B:8268 | cysteine-rich, angiogenic inducer, 61 CYR61 | 3491 | NM_001554 |
| C:2817 | immunoglobulin mu binding protein 2 (IGHMBP2), mRNA | 3508 | NM_002180 |
| A:07761 | interleukin 1, alpha (IL1A), mRNA | 3552 | NM_000575 |
| A:08500 | interleukin 1, beta (IL1B), mRNA | 3553 | NM_000576 |
| A:02668 | interleukin 2 (IL2), mRNA | 3558 | NM_000586 |
| A:03791 | interleukin 2 receptor, alpha (IL2RA), mRNA | 3559 | NM_000417 |
| B:4721 | interleukin 2 receptor, gamma (severe combined immunodeficiency) (IL2RG), mRNA | 3561 | NM_000206 |
| A:09679 | interleukin 3 (colony-stimulating factor, multiple) (IL3), mRNA | 3562 | NM_000588 |
| A:05115 | interleukin 4 (IL4), transcript variant 1, mRNA | 3565 | NM_000589 |
| A:04767 | interleukin 5 (colony-stimulating factor, eosinophil) (IL5), mRNA | 3567 | NM_000879 |
| A:00154 | interleukin 5 receptor, alpha (IL5RA), transcript variant 1, mRNA | 3568 | NM_000564 |
| A:00705 | interleukin 6 (interferon, beta 2) (IL6), mRNA | 3569 | NM_000600 |
| B:6258 | interleukin 6 receptor (IL6R), transcript variant 1, mRNA | 3570 | NM_000565 |
| A:04305 | interleukin 7 (IL7), mRNA | 3574 | NM_000880 |
| A:06269 | interleukin 8 (IL8), mRNA | 3576 | NM_000584 |
| A:10396 | interleukin 9 (IL9), mRNA | 3578 | NM_000590 |
| B:9037 | interleukin 8 receptor, beta (IL8RB), mRNA | 3579 | NM_001557 |
| A:07447 | interleukin 9 receptor (IL9R), transcript variant 1, mRNA | 3581 | NM_002186 |
| A:07424 | interleukin 10 (IL10), mRNA | 3586 | NM_000572 |
| C:2709 | interleukin 11 (IL11), mRNA | 3589 | NM_000641 |
| A:02631 | interleukin 12A (natural killer cell stimulatory factor 1, cytotoxic lymphocyte maturation factor 1, p35) (IL12A), mRNA | 3592 | NM_000882 |
| A:01248 | interleukin 12B (natural killer cell stimulatory factor 2, cytotoxic lymphocyte maturation factor 2, p40) (IL12B), mRNA | 3593 | NM_002187 |
| A:02885 | interleukin 12 receptor, beta 1 (IL12RB1), transcript variant 1, mRNA | 3594 | NM_005535 |
| B:4956 | interleukin 12 receptor, beta 2 (IL12RB2), mRNA | 3595 | NM_001559 |
| C:2230 | interleukin 13 (IL13), mRNA | 3596 | NM_002188 |
| A:02144 | interleukin 13 receptor, alpha 2 (IL13RA2), mRNA | 3599 | NM_000640 |
| A:05823 | interleukin 15 (IL15), transcript variant 3, mRNA | 3600 | NM_000585 |
| A:05507 | interleukin 15 receptor, alpha (IL15RA), transcript variant 1, mRNA | 3601 | NM_002189 |
| A:09902 | tumour necrosis factor receptor superfamily, member 9 (TNFRSF9), mRNA | 3604 | NM_001561 |
| A:01751 | interleukin 18 (interferon-gamma-inducing factor) (IL18), mRNA | 3606 | NM_001562 |
| B: 1174 | interleukin enhancer binding factor 3, 90kDa (ILF3), transcript variant 1, mRNA | 3609 | NM_012218 |
| A:06560 | integrin-linked kinase (ILK), transcript variant 1, mRNA | 3611 | NM_004517 |
| A:04679 | inner centromere protein antigens 135/155kDa (INCENP), mRNA | 3619 | NM_020238 |
| B:8330 | inhibitor of growth family, member 1 (ING1), transcript variant 4, mRNA | 3621 | NM_005537 |
| A:05295 | inhibin, alpha (INHA), mRNA | 3623 | NM_002191 |
| A:02189 | inhibin, beta A (activin A, activin AB alpha polypeptide) (INHBA), mRNA | 3624 | NM_002192 |
| B:4601 | chemokine (C-X-C motif) ligand 10 (CXCL10), mRNA | 3627 | NM_001565 |
| B:3728 | insulin induced gene 1 (INSIG1), transcript variant 1, mRNA | 3638 | NM_005542 |
| A:08018 | insulin-like 4 (placenta) (INSL4), mRNA | 3641 | NM_002195 |
| A:02981 | interferon regulatory factor 1 (IRF1), mRNA | 3659 | NM_002198 |
| A:00655 | interferon regulatory factor 2 (IRF2), mRNA | 3660 | NM_002199 |
| B:4265 | interferon stimulated exonuclease gene 20kDa (ISG20), mRNA | 3669 | NM_002201 |
| C:0395 | jagged 2 (JAG2), transcript variant 1, mRNA | 3714 | NM_002226 |
| A:05470 | Janus kinase 2 (a protein tyrosine kinase) (JAK2), mRNA | 3717 | NM_004972 |
| A:04848 | v-jun sarcoma virus 17 oncogene homolog (avian) (JUN), mRNA | 3725 | NM_002228 |
| A:08730 | jun B proto-oncogene (JUNB), mRNA | 3726 | NM_002229 |
| A:06684 | kinesin family member 11 (KIF11), mRNA | 3832 | NM_004523 |
| B:4887 | kinesin family member C1 (KIFC1), mRNA | 3833 | NM_002263 |
| A:02390 | kinesin family member 22 (KIF22), mRNA | 3835 | NM_007317 |
| B:4036 | karyopherin alpha 2 (RAG cohort 1, importin alpha 1) (KPNA2), mRNA | 3838 | NM_002266 |
| B:8230 | v-Ki-ras2 Kirsten rat sarcoma viral oncogene homolog (KRAS), transcript variant b, mRNA | 3845 | NM_004985 |
| A:08264 | keratin 16 (focal non-epidermolytic palmoplantar keratoderma) (KRT16), mRNA | 3868 | NM_005557 |
| B:6112 | lymphocyte-specific protein tyrosine kinase (LCK), mRNA | 3932 | NM_005356 |
| A:02572 | leukaemia inhibitory factor (cholinergic differentiation factor) (LIF), mRNA | 3976 | NM_002309 |
| A:02207 | ligase I, DNA, ATP-dependent (LIG1), mRNA | 3978 | NM_000234 |
| A:08891 | ligase III, DNA, ATP-dependent (LIG3), nuclear gene encoding mitochondrial protein, transcript variant alpha, mRNA | 3980 | NM_013975 |
| A:05297 | ligase IV, DNA, ATP-dependent (LIG4), mRNA | 3981 | NM_206937 |
| B:8631 | LIM domain only 1 (rhombotin 1) (LMO1), mRNA | 4004 | NM_002315 |
| A:00504 | LIM domain containing preferred translocation partner in lipoma (LPP), mRNA | 4029 | NM_005578 |
| A:00504 | LIM domain containing preferred translocation partner in lipoma (LPP), mRNA | 4030 | NM_005578 |
| B:0707 | low density lipoprotein-related protein 1 (alpha-2-macroglobulin receptor) (LRP1), mRNA | 4035 | NM_002332 |
| A:09461 | low density lipoprotein receptor-related protein 5 (LRP5), mRNA | 4041 | NM_002335 |
| A:03776 | low density lipoprotein receptor-related protein associated protein 1 (LRPAP1), mRNA | 4043 | NM_002337 |
| B:7687 | latent transforming growth factor beta binding protein 2 (LTBP2), mRNA | 4053 | NM_000428 |
| C:2653 | v-yes-1 Yamaguchi sarcoma viral related oncogene homolog (LYN), mRNA | 4067 | NM_002350 |
| A:10613 | tumour-associated calcium signal transducer 2 (TACSTD2), mRNA | 4070 | NM_002353 |
| A:03716 | MAX dimerization protein 1 (MXD1), mRNA | 4084 | NM_002357 |
| A:06387 | MAD2 mitotic arrest deficient-like 1 (yeast) (MAD2L1), mRNA | 4085 | NM_002358 |
| B:5699 | v-maf musculoaponeurotic fibrosarcoma oncogene homolog (avian) (MAFG), transcript variant 1, mRNA | 4097 | NM_002359 |
| A:03848 | MAS1 oncogene (MAS1), mRNA | 4142 | NM_002377 |
| B:9275 | megakaryocyte-associated tyrosine kinase (MATK), transcript variant 1, mRNA | 4145 | NM_139355 |
| B:4426 | mutated in colorectal cancers (MCC), mRNA | 4163 | NM_002387 |
| A:08834 | MCM2 minichromosome maintenance deficient 2, mitotin (S. cerevisiae) (MCM2), mRNA | 4171 | NM_004526 |
| A:08668 | MCM3 minichromosome maintenance deficient 3 (S. cerevisiae) (MCM3), mRNA | 4172 | NM_002388 |
| B:7581 | MCM4 minichromosome maintenance deficient 4 (S. cerevisiae) (MCM4), transcript variant 1, mRNA | 4173 | NM_005914 |
| B:7805 | MCM5 minichromosome maintenance deficient 5, cell division cycle 46 (S. cerevisiae) (MCM5), mRNA | 4174 | NM_006739 |
| B:8147 | MCM6 minichromosome maintenance deficient 6 (MIS5 homolog, S. pombe) (S. cerevisiae) (MCM6), mRNA | 4175 | NM_005915 |
| B:7620 | MCM7 minichromosome maintenance deficient 7 (S. cerevisiae) MCM7 | 4176 | NM_005916 |
| B:4650 | midkine (neurite growth-promoting factor 2) (MDK), transcript variant 1, mRNA | 4192 | NM_001012334 |
| B:8649 | Mdm2, transformed 3T3 cell double minute 2, p53 binding protein (mouse) (MDM2), transcript variant MDM2a, mRNA | 4193 | NM_006878 |
| A:03964 | Mdm4, transformed 3T3 cell double minute 4, p53 binding protein (mouse) (MDM4), mRNA | 4194 | NM_002393 |
| A:10600 | RAB8A, member RAS oncogene family (RAB8A), mRNA | 4218 | NM_005370 |
| B:8222 | met proto-oncogene (hepatocyte growth factor receptor) MET | 4233 | NM_000245 |
| A:09470 | KIT ligand (KITLG), transcript variant b, mRNA | 4254 | NM_000899 |
| A:01575 | O-6-methylguanine-DNA methyltransferase (MGMT), mRNA | 4255 | NM_002412 |
| A:10388 | antigen identified by monoclonal antibody Ki-67 (MKI67), mRNA | 4288 | NM_002417 |
| A:06073 | mutL homolog 1, colon cancer, nonpolyposis type 2 (E. coli) (MLH1), mRNA | 4292 | NM_000249 |
| B:7492 | myeloid/lymphoid or mixed-lineage leukaemia (trithorax homolog, Drosophila); translocated to, 7 (MLLT7), mRNA | 4303 | NM_005938 |
| A:09644 | meningioma (disrupted in balanced translocation) 1 (MN1), mRNA | 4330 | NM_002430 |
| A:08968 | menage a trois 1 (CAK assembly factor) (MNAT1), mRNA | 4331 | NM_002431 |
| A:02100 | MAX binding protein (MNT), mRNA | 4335 | NM_020310 |
| A:02282 | v-mos Moloney murine sarcoma viral oncogene homolog (MOS), mRNA | 4342 | NM_005372 |
| A:06141 | myeloproliferative leukaemia virus oncogene (MPL), | 4352 | NM_005373 |
| A:04072 | MRE11 meiotic recombination 11 homolog A (S. cerevisiae) (MRE11A), transcript variant 1, mRNA | 4361 | NM_005591 |
| A:04072 | MRE11 meiotic recombination 11 homolog A (S. cerevisiae) (MRE11A), transcript variant 1, mRNA | 4362 | NM_005591 |
| A:04514 | mutS homolog 2, colon cancer, nonpolyposis type 1 (E. (MSH2), mRNA | 4436 | NM_000251 |
| A:06785 | mutS homolog 3 (E. coli) (MSH3), mRNA | 4437 | NM_002439 |
| A:02756 | mutS homolog 4 (E. coli) (MSH4), mRNA | 4438 | NM_002440 |
| A:09339 | mutS homolog 5 (E. coli) (MSH5), transcript variant 1, mRNA | 4439 | NM_025259 |
| A:04591 | macrophage stimulating 1 receptor (c-met-related tyrosine kinase) (MST1 R), mRNA | 4486 | NM_002447 |
| A:05992 | metallothionein 3 (growth inhibitory factor (neurotrophic)) (MT3), mRNA | 4504 | NM_005954 |
| C:2393 | mature T-cell proliferation 1 (MTCP1), nuclear gene encoding mitochondrial protein, transcript variant B1, mRNA | 4515 | NM_014221 |
| A:01898 | mutY homolog (E. coli) (MUTYH), mRNA | 4595 | NM_012222 |
| A:10478 | MAX interactor 1 (MXI1), transcript variant 1, mRNA | 4601 | NM_005962 |
| B:5181 | v-myb myeloblastosis viral oncogene homolog (avian) MYB | 4602 | NM_005375 |
| B:5429 | v-myb myeloblastosis viral oncogene homolog (avian)-like 1 (MYBL1), mRNA | 4603 | XM_034274, XM_933460, XM_938064 |
| A:06037 | v-myb myeloblastosis viral oncogene homolog (avian)-like 2 (MYBL2), mRNA | 4605 | NM_002466 |
| A:02498 | v-myc myelocytomatosis viral oncogene homolog (avian) (MYC), mRNA | 4609 | NM_002467 |
| C:2723 | myosin, heavy polypeptide 10, non-muscle (MYH10), | 4628 | NM_005964 |
| B:4239 | NGFI-A binding protein 2 (EGR1 binding protein 2) (NAB2), mRNA | 4665 | NM_005967 |
| B:1584 | nucleosome assembly protein 1-like 1 (NAP1L1), transcript variant 1, mRNA | 4673 | NM_139207 |
| A:09960 | neuroblastoma, suppression of tumourigenicity 1 (NBL1), transcript variant 1, mRNA | 4681 | NM_182744 |
| A:02361 | nucleotide binding protein 1 (MinD homolog, E. coli) (NUBP1), mRNA | 4682 | NM_002484 |
| A:10519 | nibrin (NBN), transcript variant 1, mRNA | 4683 | NM_002485 |
| A:08868 | NCK adaptor protein 1 (NCK1), mRNA | 4690 | NM_006153 |
| A:07320 | necdin homolog (mouse) (NDN), mRNA | 4692 | NM_002487 |
| B:5481 | Norrie disease (pseudoglioma) (NDP), mRNA | 4693 | NM_000266 |
| B:4761 | septin 2 (SEPT2), transcript variant 4, mRNA | 4735 | NM_004404 |
| A:04128 | neural precursor cell expressed, developmentally down-regulated 9 (NEDD9), transcript variant 1, mRNA | 4739 | NM_006403 |
| B:7542 | NIMA (never in mitosis gene a)-related kinase 1 (NEK1), mRNA | 4750 | NM_012224 |
| A:00847 | NIMA (never in mitosis gene a)-related kinase 2 (NEK2), mRNA | 4751 | NM_002497 |
| B:7555 | NIMA (never in mitosis gene a)-related kinase 3 (NEK3), transcript variant 1, mRNA | 4752 | NM_002498 |
| B:9751 | neurofibromin 1 (neurofibromatosis, von Recklinghausen disease, Watson disease) (NF1), mRNA | 4763 | NM_000267 |
| B:7527 | neurofibromin 2 (bilateral acoustic neuroma) (NF2), transcript variant 12, mRNA | 4771 | NM_181825 |
| B:8431 | nuclear factor I/A (NFIA), mRNA | 4774 | NM_005595 |
| A:03729 | nuclear factor I/B (NFIB), mRNA | 4781 | NM_005596 |
| B:5428 | nuclear factor I/C (CCAAT-binding transcription factor) (NFIC), transcript variant 1, mRNA | 4782 | NM_005597 |
| C:5826 | nuclear factor I/X (CCAAT-binding transcription factor) (NFIX), mRNA | 4784 | NM_002501 |
| B:5078 | nuclear transcription factor Y, gamma NFYC | 4802 | NM_014223 |
| A:05462 | NHP2 non-histone chromosome protein 2-like 1 (S. cerevisiae) (NHP2L1), transcript variant 1, mRNA | 4809 | NM_005008 |
| A:01677 | non-metastatic cells 1, protein (NM23A) expressed in (NME1), transcript variant 2, mRNA | 4830 | NM_000269 |
| A:04306 | non-metastatic cells 2, protein (NM23B) expressed in (NME2), transcript variant 1, mRNA | 4831 | NM_002512 |
| C:1522 | nucleolar protein 1, 120kDa (NOL1), transcript variant 2, mRNA | 4839 | NM_001033714 |
| A:06565 | neuropeptide Y (NPY), mRNA | 4852 | NM_000905 |
| A:00579 | Notch homolog 2 (Drosophila) (NOTCH2), mRNA | 4853 | NM_024408 |
| A:02787 | neuroblastoma RAS viral (v-ras) oncogene homolog (NRAS), mRNA | 4893 | NM_002524 |
| B:6139 | nuclear mitotic apparatus protein 1 (NUMA1), mRNA | 4926 | NM_006185 |
| A:04432 | opioid receptor, mu 1 (OPRM1), transcript variant MOR-1, mRNA | 4988 | NM_000914 |
| A:02654 | origin recognition complex, subunit 1-like (yeast) (ORC1L), mRNA | 4998 | NM_004153 |
| A:01697 | origin recognition complex, subunit 2-like (yeast) (ORC2L), mRNA | 4999 | NM_006190 |
| A:06724 | origin recognition complex, subunit 4-like (yeast) (ORC4L), transcript variant 2, mRNA | 5000 | NM_002552 |
| C:0244 | origin recognition complex, subunit 5-like (yeast) (ORC5L), transcript variant 2, mRNA | 5001 | NM_181747 |
| A:09399 | oncostatin M (OSM), mRNA | 5008 | NM_020530 |
| A:07058 | proliferation-associated 2G4, 38kDa (PA2G4), mRNA | 5036 | NM_006191 |
| A:04710 | platelet-activating factor acetylhydrolase, isoform Ib, alpha subunit 45kDa (PAFAH1B1), mRNA | 5048 | NM_000430 |
| A:03397 | peroxiredoxin 1 (PRDX1), transcript variant 1, mRNA | 5052 | NM_002574 |
| B:4727 | regenerating islet-derived 3 alpha (REG3A), transcript variant 1, mRNA | 5068 | NM_002580 |
| A:03215 | PRKC, apoptosis, WT1, regulator (PAWR), mRNA | 5074 | NM_002583 |
| A:03715 | proliferating cell nuclear antigen (PCNA), transcript variant 1, mRNA | 5111 | NM_002592 |
| A:09486 | PCTAIRE protein kinase 1 (PCTK1), transcript variant 1, mRNA | 5127 | NM_006201 |
| A:09486 | PCTAIRE protein kinase 1 (PCTK1), transcript variant 1, mRNA | 5128 | NM_006201 |
| C:2666 | platelet-derived growth factor alpha polypeptide (PDGFA), transcript variant 1, mRNA | 5154 | NM_002607 |
| B:7519 | platelet-derived growth factor beta polypeptide (simian sarcoma viral (v-sis) oncogene homolog) (PDGFB), transcript variant 1, mRNA | 5155 | NM_002608 |
| A:02349 | platelet-derived growth factor receptor, alpha polypeptide (PDGFRA), mRNA | 5156 | NM_006206 |
| A:00876 | PDZ domain containing 1 (PDZK1), mRNA | 5174 | NM_002614 |
| A:04139 | serpin peptidase inhibitor, clade F (alpha-2 antiplasmin, pigment epithelium derived factor), member 1 (SERPINF1), transcript variant 4, mRNA | 5176 | NM_002615 |
| B:4669 | prefoldin 1 (PFDN1), mRNA | 5201 | NM_002622 |
| A:00156 | placental growth factor, vascular endothelial growth protein (PGF), mRNA | 5228 | NM_002632 |
| B:9242 | phosphoinositide-3-kinase, catalytic, beta polypeptide (PIK3CB), mRNA | 5291 | NM_006219 |
| A:09957 | protein (peptidyl-prolyl cis/trans isomerase) NIMA-interacting 1 (PIN1), mRNA | 5300 | NM_006221 |
| A:00888 | pleiomorphic adenoma gene-like 1 (PLAGL1), transcript variant 2, mRNA | 5325 | NM_006718 |
| A:08398 | plasminogen (PLG), mRNA | 5340 | NM_000301 |
| B:3744 | polo-like kinase 1 (Drosophila) (PLK1), mRNA | 5347 | NM_005030 |
| B:4722 | peripheral myelin protein 22 (PMP22), transcript variant 1, mRNA | 5376 | NM_000304 |
| A:10286 | PMS1 postmeiotic segregation increased 1 (S. cerevisiae) (PMS1), mRNA | 5378 | NM_000534 |
| A:10286 | PMS1 postmeiotic segregation increased 1 (S. cerevisiae) (PMS1), mRNA | 5379 | NM_000534 |
| B:9336 | postmeiotic segregation increased 2-like 2 (PMS2L2), mRNA | 5380 | NM_002679 |
| B:9336 | postmeiotic segregation increased 2-like 2 (PMS2L2), mRNA | 5382 | NM_002679 |
| A:10467 | postmeiotic segregation increased 2-like 5 (PMS2L5), mRNA | 5383 | NM_174930 |
| A:10467 | postmeiotic segregation increased 2-like 5 (PMS2L5), mRNA | 5386 | NM_174930 |
| A:02096 | PMS2 postmeiotic segregation increased 2 (S. cerevisiae) (PMS2), transcript variant 1, mRNA | 5395 | NM_000535 |
| B:0731 | septin 5 (SEPT5), transcript variant 1, mRNA | 5413 | NM_002688 |
| A:09062 | septin 4 (SEPT4), transcript variant 1, mRNA | 5414 | NM_004574 |
| A:05543 | polymerase (DNA directed), alpha (POLA), mRNA | 5422 | NM_016937 |
| A:02852 | polymerase (DNA directed), beta (POLB), mRNA | 5423 | NM_002690 |
| A:09477 | polymerase (DNA directed), delta 1, catalytic subunit 125kDa (POLD1), mRNA | 5424 | NM_002691 |
| A:02929 | polymerase (DNA directed), delta 2, regulatory subunit 50kDa (POLD2), mRNA | 5425 | NM_006230 |
| B:3196 | polymerase (DNA directed), epsilon POLE | 5426 | NM_006231 |
| A:04680 | polymerase (DNA directed), epsilon 2 (p59 subunit) (POLE2), | 5427 | NM_002692 |
| A:08572 | polymerase (DNA directed), gamma (POLG), mRNA | 5428 | NM_002693 |
| A:08948 | polymerase (RNA) mitochondrial (DNA directed) (POLRMT), nuclear gene encoding mitochondrial protein, mRNA | 5442 | NM_005035 |
| A:00480 | POU domain, class 1, transcription factor 1 (Pit1, growth hormone factor 1) (POU1F1), mRNA | 5449 | NM_000306 |
| C:6960 | peroxisome proliferative activated receptor, delta (PPARD), transcript variant 1, mRNA | 5467 | NM_006238 |
| B:0695 | PPAR binding protein (PPARBP), mRNA | 5469 | NM_004774 |
| A:10622 | pro-platelet basic protein (chemokine (C-X-C motif) ligand 7) (PPBP), mRNA | 5473 | NM_002704 |
| A:08431 | protein phosphatase 1G (formerly 2C), magnesium-dependent, gamma isoform (PPM1G), transcript variant 1, mRNA | 5496 | NM_177983 |
| A:05348 | protein phosphatase 1, catalytic subunit, alpha isoform (PPP1CA), transcript variant 1, mRNA | 5499 | NM_002708 |
| B:0943 | protein phosphatase 1, catalytic subunit, beta isoform (PPP1CB), transcript variant 1, mRNA | 5500 | NM_002709 |
| A:02064 | protein phosphatase 1, catalytic subunit, gamma isoform (PPP1CC), mRNA | 5501 | NM_002710 |
| A:01231 | protein phosphatase 2 (formerly 2A), catalytic subunit, alpha isoform (PPP2CA), mRNA | 5515 | NM_002715 |
| A:03825 | protein phosphatase 2 (formerly 2A), regulatory subunit A (PR 65), alpha isoform (PPP2R1A), mRNA | 5518 | NM_014225 |
| A:01064 | protein phosphatase 2 (formerly 2A), regulatory subunit A (PR 65), beta isoform (PPP2R1 B), transcript variant 1, mRNA | 5519 | NM_002716 |
| A:00874 | protein phosphatase 2 (formerly 2A), regulatory subunit B", alpha (PPP2R3A), transcript variant 1, mRNA | 5523 | NM_002718 |
| A:07683 | protein phosphatase 3 (formerly 2B), catalytic subunit, beta isoform (calcineurin A beta) (PPP3CB), mRNA | 5532 | NM_021132 |
| A:00032 | protein phosphatase 5, catalytic subunit (PPP5C), mRNA | 5536 | NM_006247 |
| A:02880 | protein phosphatase 6, catalytic subunit (PPP6C), mRNA | 5537 | NM_002721 |
| A:07833 | primase, polypeptide 1, 49kDa (PRIM1), mRNA | 5557 | NM_000946 |
| A:08706 | primase, polypeptide 2A, 58kDa PRIM2A | 5558 | NM_000947 |
| A:00953 | protein kinase, cAMP-dependent, regulatory, type I, alpha (tissue specific extinguisher 1) (PRKAR1A), transcript variant 1, mRNA | 5573 | NM_002734 |
| A:07305 | protein kinase, cAMP-dependent, regulatory, type II, beta (PRKAR2B), mRNA | 5578 | NM_002736 |
| A:08970 | protein kinase D1 (PRKD1), mRNA | 5587 | NM_002742 |
| A:05228 | protein kinase, cGMP-dependent, type II (PRKG2), mRNA | 5593 | NM_006259 |
| B:6263 | mitogen-activated protein kinase 1 (MAPK1), transcript variant 1, mRNA | 5594 | NM_002745 |
| B:5471 | mitogen-activated protein kinase 3 (MAPK3), mRNA | 5595 | NM_002746 |
| B:9088 | mitogen-activated protein kinase 4 (MAPK4), mRNA | 5596 | NM_002747 |
| A:03644 | mitogen-activated protein kinase 6 (MAPK6), mRNA | 5597 | NM_002748 |
| A:09951 | mitogen-activated protein kinase 7 (MAPK7), transcript variant 1, mRNA | 5598 | NM_139033 |
| A:00932 | mitogen-activated protein kinase 13 (MAPK13), mRNA | 5603 | NM_002754 |
| A:06747 | mitogen-activated protein kinase 6 (MAP2K6), transcript variant 1, mRNA | 5608 | NM_002758 |
| B:4014 | mitogen-activated protein kinase 7 MAP2K7 | 5609 | NM_145185 |
| B:1372 | eukaryotic translation initiation factor 2-alpha kinase 2 (EIF2AK2), mRNA | 5610 | NM_002759 |
| B:5991 | protein-kinase, interferon-inducible double stranded RNA dependent inhibitor, repressor of (P58 repressor) (PRKRIR), mRNA | 5612 | NM_004705 |
| A:03959 | prolactin (PRL), mRNA | 5617 | NM_000948 |
| A:09385 | protamine 1 (PRM1), mRNA | 5619 | NM_002761 |
| A:02848 | protamine 2 (PRM2), mRNA | 5620 | NM_002762 |
| A:07907 | kallikrein 10 (KLK10), transcript variant 1, mRNA | 5655 | NM_002776 |
| A:01338 | proteinase 3 (serine proteinase, neutrophil, Wegener granulomatosis autoantigen) (PRTN3), mRNA | 5657 | NM_002777 |
| B:4949 | presenilin 1 (Alzheimer disease 3) PSEN1 | 5663 | NM_000021 |
| A:00037 | presenilin 2 (Alzheimer disease 4) (PSEN2), transcript variant 1, mRNA | 5664 | NM_000447 |
| A:05430 | peptide YY (PYY), mRNA | 5697 | NM_004160 |
| A:05083 | proteasome (prosome, macropain) 26S subunit, non-ATPase, 8 (PSMD8), mRNA | 5714 | NM_002812 |
| A:10847 | patched homolog (Drosophila) (PTCH), mRNA | 5727 | NM_000264 |
| A:04029 | phosphatase and tensin homolog (mutated in multiple advanced cancers 1) (PTEN), mRNA | 5728 | NM_000314 |
| A:08708 | parathyroid hormone-like hormone (PTHLH), transcript variant 2, mRNA | 5744 | NM_002820 |
| B:4775 | prothymosin, alpha (gene sequence 28) (PTMA), mRNA | 5757 | NM_002823 |
| A:05250 | parathymosin (PTMS), mRNA | 5763 | NM_002824 |
| C:2316 | pleiotrophin (heparin binding growth factor 8, neurite growth-promoting factor 1) (PTN), mRNA | 5764 | NM_002825 |
| C:2627 | quiescin Q6 (QSCN6), transcript variant 1, mRNA | 5768 | NM_002826 |
| A:10310 | protein tyrosine phosphatase, non-receptor type 6 (PTPN6), transcript variant 2, mRNA | 5777 | NM_080548 |
| A:02619 | RAD1 homolog (S. pombe) (RAD1), transcript variant 1, mRNA | 5810 | NM_002853 |
| C:2196 | purine-rich element binding protein A (PURA), mRNA | 5813 | NM_005859 |
| B:1151 | ras-related C3 botulinum toxin substrate 1 (rho family, small GTP binding protein Rac1) (RAC1), transcript variant Rac1b, mRNA | 5879 | NM_018890 |
| A:05292 | RAD9 homolog A (S. pombe) (RAD9A), mRNA | 5883 | NM_004584 |
| A:10635 | RAD17 homolog (S. pombe) (RAD17), transcript variant 8, mRNA | 5884 | NM_002873 |
| A:07580 | RAD21 homolog (S. pombe) (RAD21), mRNA | 5885 | NM_006265 |
| A:07819 | RAD51 homolog (RecA homolog, E. coli) (S. cerevisiae) (RAD51), transcript variant 1, mRNA | 5888 | NM_002875 |
| A:09744 | RAD51-like 1 (S. cerevisiae) (RAD51L1), transcript variant 1, mRNA | 5890 | NM_002877 |
| B:0346 | RAD51-like 3 (S. cerevisiae) RAD51 L3 | 5892 | NM_002878, NM_133629 |
| B:1043 | RAD52 homolog (S. cerevisiae) (RAD52), transcript variant beta, mRNA | 5893 | NM_134424 |
| C:2457 | v-raf-1 murine leukaemia viral oncogene homolog 1 (RAF1), mRNA | 5894 | NM_002880 |
| B:8341 | ral guanine nucleotide dissociation stimulator RALGDS | 5900 | NM_001042368, NM_006266 |
| A:09169 | RAN, member RAS oncogene family (RAN), mRNA | 5901 | NM_006325 |
| C:0082 | RAP1A, member of RAS oncogene family RAP1A | 5906 | NM_001010935, NM_002884 |
| A:00423 | RAP1 B, member of RAS oncogene family (RAP1 B), transcript variant 1, mRNA | 5908 | NM_015646 |
| A:09690 | retinoic acid receptor responder (tazarotene induced) 1 (RARRES1), transcript variant 2, mRNA | 5918 | NM_002888 |
| A:08045 | retinoic acid receptor responder (tazarotene induced) 3 (RARRES3), mRNA | 5920 | NM_004585 |
| B:9011 | retinoblastoma 1 (including osteosarcoma) (RB1), mRNA | 5925 | NM_000321 |
| A:04888 | retinoblastoma binding protein 4 (RBBP4), mRNA | 5928 | NM_005610 |
| C:2267 | retinoblastoma binding protein 6 (RBBP6), transcript variant 1, mRNA | 5930 | NM_006910 |
| A:06741 | retinoblastoma binding protein 7 (RBBP7), mRNA | 5931 | NM_002893 |
| A:09145 | retinoblastoma binding protein 8 (RBBP8), transcript variant 1, mRNA | 5932 | NM_002894 |
| A:10222 | retinoblastoma-like 1 (p107) (RBL1), transcript variant 1, mRNA | 5933 | NM_002895 |
| A:08246 | retinoblastoma-like 2 (p130) (RBL2), mRNA | 5934 | NM_005611 |
| B:9795 | RNA binding motif, single stranded interacting protein 1 (RBMS1), transcript variant 1, mRNA | 5937 | NM_016836 |
| B:1393 | regenerating islet-derived 1 alpha (pancreatic stone protein, pancreatic thread protein) (REG1A), mRNA | 5967 | NM_002909 |
| B:4741 | regenerating islet-derived 1 beta (pancreatic stone protein, pancreatic thread protein) (REG1B), mRNA | 5968 | NM_006507 |
| B:4741 | regenerating islet-derived 1 beta (pancreatic stone protein, pancreatic thread protein) (REG1B), mRNA | 5969 | NM_006507 |
| A:04164 | REV3-like, catalytic subunit of DNA polymerase zeta (yeast) (REV3L), mRNA | 5980 | NM_002912 |
| A:03348 | replication factor C (activator 1) 1, 145kDa (RFC1), mRNA | 5981 | NM_002913 |
| A:06693 | replication factor C (activator 1) 2, 40kDa (RFC2), transcript variant 1, mRNA | 5982 | NM_181471 |
| A:02491 | replication factor C (activator 1) 3, 38kDa (RFC3), transcript variant 1, mRNA | 5983 | NM_002915 |
| A:09921 | replication factor C (activator 1) 4, 37kDa (RFC4), transcript variant 1, mRNA | 5984 | NM_002916 |
| B:3726 | replication factor C (activator 1) 5, 36kDa (RFC5), transcript variant 1, mRNA | 5985 | NM_007370 |
| A:04896 | ret finger protein (RFP), transcript variant alpha, mRNA | 5987 | NM_006510 |
| A:04971 | regulator of G-protein signalling 2, 24kDa (RGS2), | 5997 | NM_002923 |
| B:8684 | relaxin 2 (RLN2), transcript variant 2, mRNA | 6024 | NM_005059 |
| A:10597 | replication protein A1, 70kDa (RPA1), mRNA | 6117 | NM_002945 |
| A:09203 | replication protein A2, 32kDa (RPA2), mRNA | 6118 | NM_002946 |
| A:00231 | replication protein A3, 14kDa (RPA3), mRNA | 6119 | NM_002947 |
| B:8856 | ribosomal protein S4, X-linked (RPS4X), mRNA | 6191 | NM_001007 |
| B:8856 | ribosomal protein S4, X-linked (RPS4X), mRNA | 6192 | NM_001007 |
| A:10444 | ribosomal protein S6 kinase, 70kDa, polypeptide 2 (RPS6KB2), transcript variant 1, mRNA | 6199 | NM_003952 |
| A:02188 | ribosomal protein S25 (RPS25), mRNA | 6232 | NM_001028 |
| A:08509 | related RAS viral (r-ras) oncogene homolog (RRAS), mRNA | 6237 | NM_006270 |
| A:09802 | ribonucleotide reductase M1 polypeptide (RRM1), mRNA | 6240 | NM_001033 |
| B:3501 | ribonucleotide reductase M2 polypeptide (RRM2), mRNA | 6241 | NM_001034 |
| A:08332 | S100 calcium binding protein A5 (S100A5), mRNA | 6276 | NM_002962 |
| C:1129 | S100 calcium binding protein A6 (calcyclin) (S100A6), | 6277 | NM_014624 |
| B:3690 | S100 calcium binding protein A11 (calgizzarin) (S100A11), | 6282 | NM_005620 |
| A:08910 | S100 calcium binding protein, beta (neural) (S1008), | 6285 | NM_006272 |
| A:05458 | mitogen-activated protein kinase 12 (MAPK12), mRNA | 6300 | NM_002969 |
| A:07786 | tetraspanin 31 (TSPAN31), mRNA | 6302 | NM_005981 |
| A:09884 | C-type lectin domain family 11, member A (CLEC11A), mRNA | 6320 | NM_002975 |
| A:00985 | chemokine (C-C motif) ligand 3 (CCL3), mRNA | 6348 | NM_002983 |
| A:00985 | chemokine (C-C motif) ligand 3 (CCL3), mRNA | 6349 | NM_002983 |
| B:0899 | chemokine (C-C motif) ligand 14 (CCL14), transcript variant 2, mRNA | 6358 | NM_032962 |
| B:0898 | chemokine (C-C motif) ligand 23 (CCL23), transcript variant CKbeta8, mRNA | 6368 | NM_145898 |
| B:5275 | chemokine (C-X-C motif) ligand 11 (CXCL11), mRNA | 6374 | NM_005409 |
| C:2038 | SET translocation (myeloid leukaemia-associated) (SET), mRNA | 6418 | M_003011 |
| A:00679 | SHC (Src homology 2 domain containing) transforming protein 1 (SHC1), transcript variant 1, mRNA | 6464 | NM_183001 |
| B:9295 | SCL/TAL1 interrupting locus (STIL), mRNA | 6491 | NM_003035 |
| B:7410 | signal-induced proliferation-associated gene 1 (SIPA1), transcript variant 1, mRNA | 6494 | NM_1532538 |
| C:5435 | S-phase kinase-associated protein 2 (p45) (SKP2), transcript variant 1, mRNA | 6502 | NM_005983 |
| A:09017 | signaling lymphocytic activation molecule family member 1 (SLAMF1), mRNA | 6504 | NM_003037 |
| A:06456 | solute carrier family 12 (potassium/chloride transporters), member 4 (SLC12A4), mRNA | 6560 | NM_005072 |
| A:05730 | SWI/SNF related, matrix associated, actin dependent regulator of chromatin, subfamily b, member 1 (SMARCB1), transcript variant 1, mRNA | 6598 | NM_003073 |
| A:07314 | fascin homolog 1, actin-bundling protein (Strongylocentrotus purpuratus) (FSCN1), mRNA | 6624 | NM_003088 |
| A:04540 | sparc/osteonectin, cwcv and kazallike domains proteoglycan (testican) 1 (SPOCK1), mRNA | 6695 | NM_004598 |
| A:09441 | secreted phosphoprotein 1 (osteopontin, bone sialoprotein I, early T-lymphocyte activation 1) (SPP1), mRNA | 6696 | NM_000582 |
| A:02264 | v-src sarcoma (Schmidt-Ruppin A-2) viral oncogene homolog (avian) (SRC), transcript variant 1, mRNA | 6714 | NM_005417 |
| A:04127 | single-stranded DNA binding protein 1 (SSBP1), | 6742 | NM_003143 |
| A:07245 | signal sequence receptor, alpha (translocon-associated protein alpha) (SSR1), mRNA | 6745 | NM_003144 |
| A:08350 | somatostatin (SST), mRNA | 6750 | M_001048 |
| A:03956 | somatostatin receptor 1 (SSTR1), mRNA | 6751 | NM_001049 |
| C:1740 | somatostatin receptor 2 (SSTR2), mRNA | 6752 | M_001050 |
| A:04237 | somatostatin receptor 3 (SSTR3), mRNA | 6753 | NM_001051 |
| A:04852 | somatostatin receptor 4 (SSTR4), mRNA | 6754 | NM_001052 |
| A:01484 | somatostatin receptor 5 (SSTR5), mRNA | 6755 | M_001053 |
| A:03398 | signal transducer and activator of transcription 1, 91kDa (STAT1), transcript variant alpha, mRNA | 6772 | NM_007315 |
| A:05843 | stromal interaction molecule 1 (STIM1), mRNA | 6786 | NM_003156 |
| A:04562 | NIMA (never in mitosis gene a)-related kinase 4 (NEK4), mRNA | 6787 | NM_003157 |
| A:04814 | serine/threonine kinase 6 (STK6), transcript variant 1, mRNA | 6790 | NM_198433 |
| A:01764 | aurora kinase C (AURKC), transcript variant 3, mRNA | 6795 | NM_003160 |
| A:10309 | suppressor of variegation 3-9 homolog 1 (Drosophila) (SUV39H1), mRNA | 6839 | NM_003173 |
| A:01895 | synaptonemal complex protein 1 (SYCP1), mRNA | 6847 | NM_003176 |
| A:09854 | spleen tyrosine kinase (SYK), mRNA | 6850 | NM_003177 |
| A:02589 | transcriptional adaptor 2 (ADA2 homolog; yeast)-like (TADA2L), transcript variant 1, mRNA | 6871 | NM_001488 |
| A:01355 | TAF1 RNA polymerase II, TATA box binding protein (TBP)-associated factor, 250kDa (TAF1), transcript variant 1, mRNA | 6872 | NM_004606 |
| C:1960 | T-cell acute lymphocytic leukaemia 1 (TAL1), mRNA | 6886 | NM_003189 |
| C:2789 | transcription factor 3 (E2A immunoglobulin enhancer binding factors E12/E47) (TCF3), mRNA | 6930 | NM_003200 |
| B:4738 | transcription factor 8 (represses interleukin 2 expression) (TCF8), mRNA | 6935 | NM_030751 |
| A:03967 | transcription factor 19 (SC1) (TCF19), mRNA | 6941 | NM_007109 |
| A:05964 | telomerase-associated protein 1 (TEP1), mRNA | 7011 | M_007110 |
| B:9167 | telomeric repeat binding factor (NIMA-interacting) 1 (TERF1), transcript variant 2, mRNA | 7013 | NM_003218 |
| B:7401 | telomeric repeat binding factor 2 (TERF2), mRNA | 7014 | NM_005652 |
| C:0355 | telomerase reverse transcriptase (TERT), transcript variant 1, mRNA | 7015 | NM_003219 |
| A:07625 | transcription factor A, mitochondrial (TFAM), mRNA | 7019 | NM_003201 |
| A:06784. | nuclear receptor subfamily 2, group F, member 1 (NR2F1), mRNA | 7025 | NM_005654 |
| A:06784 | nuclear receptor subfamily 2, group F, member 1 (NR2F1), mRNA | 7027 | NM_005654 |
| B:5016 | transcription factor Dp-2 (E2F dimerization partner 2) (TFDP2), mRNA | 7029 | NM_006286 |
| B:5851 | transforming growth factor, alpha (TGFA), mRNA | 7039 | NM_003236 |
| A:07050 | transforming growth factor, beta 1 (Camurati-Engelmann disease) (TGFB1), mRNA | 7040 | NM_000660 |
| B:0094 | transforming growth factor beta 1 induced transcript 1 (TGFB1I1), mRNA | 7041 | NM_015927 |
| A:09824 | transforming growth factor, beta 2 (TGFB2), mRNA | 7042 | NM_003238 |
| B:7853 | transforming growth factor, beta 3 (TGFB3), mRNA | 7043 | NM_003239 |
| B:4156 | transforming growth factor, beta-induced, 68kDa (TGFBI), mRNA | 7045 | NM_000358 |
| A:03732 | transforming growth factor, beta receptor II (70/80kDa) (TGFBR2), transcript variant 2, mRNA | 7048 | NM_003242 |
| B:0258 | thrombopoietin (myeloproliferative leukaemia virus oncogene ligand, megakaryocyte growth and development factor) (THPO), transcript variant 3, mRNA | 7066 | NM_199356 |
| B:4371 | thyroid hormone receptor, alpha (erythroblastic leukaemia viral (verb-a) oncogene homolog, avian) (THRA), transcript variant 1, mRNA | 7067 | NM_199334 |
| A:06139 | Kruppel-like factor 10 (KLF10), transcript variant 1, mRNA | 7071 | NM_005655 |
| A:08048 | TIMP metallopeptidase inhibitor 1 (TIMP1), mRNA | 7076 | NM_003254 |
| B:3686 | transmembrane 4 L six family member 4 (TM4SF4), mRNA | 7104 | NM_004617 |
| B:5451 | topoisomerase (DNA) I (TOP1), mRNA | 7150 | NM_003286 |
| B:7145 | topoisomerase (DNA) II alpha 170kDa (TOP2A), mRNA | 7153 | NM_001067 |
| A:04487 | topoisomerase (DNA) II beta 180kDa (TOP2B), mRNA | 7155 | NM_001068 |
| A:05345 | topoisomerase (DNA) III alpha (TOP3A), mRNA | 7156 | NM_004618 |
| A:07597 | tumour protein p53 (Li-Fraumeni syndrome) (TP53), mRNA | 7157 | NM_000546 |
| B:6951 | tumour protein p53 binding protein, 2 (TP53BP2), transcript variant 1, mRNA | 7159 | NM_001031685 |
| A:10089 | tumour protein p73 (TP73), mRNA | 7161 | NM_005427 |
| A:07179 | tumour protein D52-like 1 (TPD52L1), transcript variant 4, mRNA | 7165 | NM_001003397 |
| A:00700 | tuberous sclerosis 1 (TSC1), transcript variant 1, mRNA | 7248 | NM_000368 |
| C:2440 | tuberous sclerosis 2 (TSC2), transcript variant 2, | 7249 | NM_021055 |
| A:06571 | thyroid stimulating hormone receptor (TSHR), transcript variant 1, mRNA | 7253 | NM_000369 |
| A:02759 | testis specific protein, Y-linked 1 (TSPY1), mRNA | 7258 | NM_003308 |
| A:09121 | tumour suppressing subtransferable candidate 1 (TSSC1), mRNA | 7260 | NM_003310 |
| A:07936 | TTK protein kinase (TTK), mRNA | 7272 | NM_003318 |
| A:05365 | tumour necrosis factor (ligand) superfamily, member 4 (tax-transcriptionally activated glycoprotein 1, 34kDa) (TNFSF4), mRNA | 7292 | NM_003326 |
| B:0763 | thioredoxin TXN | 7295 | NM_003329 |
| B:4917 | ubiquitin-activating enzyme E1 (A1S9T and BN75 temperature sensitivity complementing) (UBE1), transcript variant 1, mRNA | 7317 | NM_003334 |
| A:08169 | ubiquitin-conjugating enzyme E2D 1 (UBC4/5 homolog, yeast) (UBE2D1), mRNA | 7321 | NM_003338 |
| A:07196 | ubiquitin-conjugating enzyme E2D 3 (UBC4/5 homolog, yeast) (UBE2D3), transcript variant 1, mRNA | 7323 | NM_003340 |
| A:04972 | ubiquitin-conjugating enzyme E2 variant 1 (UBE2V1), transcript variant 1, mRNA | 7335 | NM_021988 |
| B:0648 | ubiquitin-conjugating enzyme E2 variant 2 (UBE2V2), mRNA | 7336 | NM_003350 |
| C:2659 | uromodulin (uromucoid, Tamm-Horsfall glycoprotein) (UMOD), transcript variant 2, mRNA | 7369 | M_001008389 |
| A:06855 | vav 1 oncogene (VAV1), mRNA | 7409 | NM_005428 |
| A:08040 | vav 2 oncogene VAV2 | 7410 | NM_003371 |
| C:1128 | vascular endothelial growth factor (VEGF), transcript variant 5, mRNA | 7422 | NM_001025369 |
| B:5229 | vascular endothelial growth factor B (VEGFB), mRNA | 7423 | NM_003377 |
| A:06320 | vascular endothelial growth factor C (VEGFC), mRNA | 7424 | NM_005429 |
| A:06488 | von Hippel-Lindau tumour suppressor (VHL), transcript variant 2, mRNA | 7428 | NM_198156 |
| C:2407 | vasoactive intestinal peptide (VIP), transcript variant 1, mRNA | 7432 | NM_003381 |
| B:8107 | vasoactive intestinal peptide receptor 1 (VIPR1), mRNA | 7433 | NM_004624 |
| A:08324 | tryptophanyl-tRNA synthetase (WARS), transcript variant 1, mRNA | 7453 | NM_004184 |
| A:06953 | WEE1 homolog (S. pombe) (WEE1), mRNA | 7465 | NM_003390 |
| B:5487 | Wilms tumour 1 (WT1), transcript variant D, mRNA | 7490 | NM_024426 |
| C:0172 | X-ray repair complementing defective repair in Chinese hamster cells 2 (XRCC2), mRNA | 7516 | NM_005431 |
| A:02526 | v-yes-1 Yamaguchi sarcoma viral oncogene homolog 1 (YES1), mRNA | 7525 | NM_005433 |
| B:5702 | ecotropic viral integration site 5 (EV15), mRNA | 7813 | NM_005665 |
| B:5523 | BTG family, member 2 (BTG2), mRNA | 7832 | NM_006763 |
| A:03788 | interferon-related developmental regulator 2 (IFRD2), mRNA | 7866 | NM_006764 |
| A:09614 | v-maf musculoaponeurotic fibrosarcoma oncogene homolog K (avian) (MAFK), mRNA | 7975 | NM_002360 |
| A:02920 | frizzled homolog 3 (Drosophila) (FZD3), mRNA | 7976 | NM_017412 |
| A:03507 | FOS-like antigen 1 (FOSL1), mRNA | 8061 | NM_005438 |
| A:00218 | cullin 5 (CUL5), mRNA | 8065 | NM_003478 |
| A:08128 | CDK2-associated protein 1 (CDK2AP1), mRNA | 8099 | NM_004642 |
| A:09843 | melanoma inhibitory activity (MIA), mRNA | 8190 | NM_006533 |
| A:09310 | chromatin assembly factor 1, subunit B (p60) (CHAF1 B), mRNA | 8208 | NM_005441 |
| A:05798 | SMC1 structural maintenance of chromosomes 1-like 1 (yeast) (SMC1L1), mRNA | 8243 | NM_006306 |
| C:0317 | axin 1 (AXIN1), transcript variant 1, mRNA | 8312 | NM_003502 |
| B:0065 | BRCA1 associated protein-1 (ubiquitin carboxy-terminal hydrolase) (BAP1), mRNA | 8314 | NM_004656 |
| A:08801 | CDC7 cell division cycle 7 (S. cerevisiae) (CDC7), mRNA | 8317 | NM_003503 |
| A:09331 | CDC45 cell division cycle 45-like (S. cerevisiae) (CDC45L), | 8318 | NM_003504 |
| A:01727 | growth factor independent 1 B (potential regulator of CDKN1A, translocated in CML) (GFI1B), mRNA | 8328 | NM_004188 |
| A:10009 | MAD1 mitotic arrest deficient-like 1 (yeast) (MAD1L1), transcript variant 1, mRNA | 8379 | NM_003550 |
| A:06561 | breast cancer anti-estrogen resistance 3 (BCAR3), mRNA | 8412 | NM_003567 |
| A:06461 | reversion-inducing-cysteine-rich protein with kazal motifs (RECK), mRNA | 8434 | NM_021111 |
| A:06991 | RAD54-like (S. cerevisiae) (RAD54L), mRNA | 8438 | NM_003579 |
| A:04140 | NCK adaptor protein 2 (NCK2), transcript variant 1, mRNA | 8440 | NM_003581 |
| B:6523 | DEAH (Asp-Glu-Ala-His) box polypeptide 16 DHX16 | 8449 | NM_003587 |
| A:09834 | cullin 4B (CUL4B), mRNA | 8450 | NM_003588 |
| A:06931 | cullin 4A (CUL4A), transcript variant 1, mRNA | 8451 | NM_001008895 |
| A:05012 | cullin 3 (CUL3), mRNA | 8452 | NM_003590 |
| A:05211 | cullin 2 (CUL2), mRNA | 8453 | NM_003591 |
| A:01673 | cullin 1 (CUL1), mRNA | 8454 | NM_003592 |
| C:0388 | Kruppel-like factor 11 (KLF11), mRNA | 8462 | NM_003597 |
| A:01318 | suppressor of Ty 3 homolog (S. cerevisiae) (SUPT3H), transcript variant 2, mRNA | 8464 | NM_181356 |
| A:01318 | suppressor of Ty 3 homolog (S. cerevisiae) (SUPT3H), transcript variant 2, mRNA | 8465 | NM_181356 |
| A:09841 | protein phosphatase 1 D magnesium-dependent, delta isoform (PPM1D), mRNA | 8493 | NM_003620 |
| B:3627 | interferon induced transmembrane protein 1 (9-27) (IFITM1), mRNA | 8519 | NM_003641 |
| A:06665 | growth arrest-specific 7 (GAS7), transcript variant a, mRNA | 8522 | NM_003644 |
| A:10603 | basic leucine zipper nuclear factor 1 (JEM-1) (BLZF1), mRNA | 8548 | NM_003666 |
| A:10266 | CDC14 cell division cycle 14 homolog A (S. cerevisiae) (CDC14A), transcript variant 2, mRNA | 8556 | NM_033312 |
| A:09697 | cyclin-dependent kinase (CDC2-like) 10 (CDK10), transcript variant 1, mRNA | 8558 | NM_003674 |
| A:10520 | protein kinase, interferon-inducible double stranded RNA dependent activator (PRKRA), mRNA | 8575 | NM_003690 |
| A:00630 | phosphatidic acid phosphatase type 2A (PPAP2A), transcript variant 2, mRNA | 8611 | NM_176895 |
| B:9227 | cell division cycle 2-like 5 (cholinesterase-related cell division controller) (CDC2L5), transcript variant 1, mRNA | 8621 | NM_003718 |
| A:08282 | tumour protein p73-like TP73L | 8626 | NM_003722 |
| B:8989 | aldo-keto reductase family 1, member C3 (3-alpha hydroxysteroid dehydrogenase, type II) (AKR1C3), mRNA | 8644 | NM_003739 |
| B:1328 | insulin receptor substrate 2 (IRS2), mRNA | 8660 | NM_003749 |
| B:4001 | CDC23 (cell division cycle 23, yeast, homolog) CDC23 | 8697 | NM_004661 |
| A:00144 | tumour necrosis factor (ligand) superfamily, member 14 (TNFSF14), transcript variant 1, mRNA | 8740 | NM_003807 |
| B:8481 | tumour necrosis factor (ligand) superfamily, member 13 (TNFSF13), transcript variant alpha, mRNA | 8741 | NM_003808 |
| A:09478 | tumour necrosis factor (ligand) superfamily, member 9 (TNFSF9), mRNA | 8744 | NM_003811 |
| B:8202 | CD164 antigen, sialomucin (CD164), mRNA | 8763 | NM_006016 |
| A:01775 | RIO kinase 3 (yeast) (RIOK3), transcript variant 2, mRNA | 8780 | NM_145906 |
| A:01775 | RIO kinase 3 (yeast) (RIOK3), transcript variant 2, mRNA | 8781 | NM_145906 |
| C:0356 | tumour necrosis factor receptor superfamily, member 11a, NFKB activator (TNFRSF11A), mRNA | 8792 | NM_003839 |
| A:03645 | cellular repressor of E1A-stimulated genes 1 (CREG1), | 8804 | NM_003851 |
| A:08261 | galanin receptor 2 (GALR2), mRNA | 8812 | NM_003857 |
| A:03558 | cyclin-dependent kinase-like 1 (CDC2-related kinase) (CDKL1), mRNA | 8814 | NM_004196 |
| B:0089 | fibroblast growth factor 18 (FGF18), transcript variant 2, mRNA | 8817 | NM_033649 |
| B:5592 | sin3-associated polypeptide, 30kDa SAP30 | 8819 | NM_003864 |
| B:4763 | IQ motif containing GTPase activating protein 1 (IQGAP1), mRNA | 8827 | NM_003870 |
| C:0673 | neuropilin 1 NRP1 | 8829 | NM_001024628, NM_001024629, NM_003873 |
| A:09407 | histone deacetylase 3 (HDAC3), mRNA | 8841 | NM_003883 |
| A:07011 | alkB, alkylation repair homolog (E. coli) (ALKBH), mRNA | 8847 | NM_006020 |
| A:06184 | p300/CBP-associated factor (PCAF), mRNA | 8850 | NM_003884 |
| A:06285 | cyclin-dependent kinase 5, regulatory subunit 1 (p35) (CDK5R1), mRNA | 8851 | NM_003885 |
| B:3696 | chromosome 10 open reading frame 7 (C10orf7), mRNA | 8872 | NM_006023 |
| C:2264 | sphingosine kinase 1 (SPHK1), transcript variant 1, mRNA | 8877 | NM_021972 |
| A:06721 | CDC16 cell division cycle 16 homolog (S. cerevisiae) (CDC16), mRNA | 8881 | NM_003903 |
| A:04142 | zinc finger protein 259 (ZNF259), mRNA | 8882 | NM_003904 |
| A:10737 | MCM3 minichromosome maintenance deficient 3 (S. cerevisiae) associated protein (MCM3AP), mRNA | 8888 | NM_003906 |
| A:03854 | cyclin A1 (CCNA1), mRNA | 8900 | NM_003914 |
| B:0704 | B-cell CLL/lymphoma 10 (BCL10), mRNA | 8915 | NM_003921 |
| A:03168 | topoisomerase (DNA) III beta (TOP3B), mRNA | 8940 | NM_003935 |
| B:9727 | cyclin-dependent kinase 5, regulatory subunit 2 (p39) (CDK5R2), mRNA | 8941 | NM_003936 |
| A:06189 | protein regulator of cytokinesis 1 (PRC1), transcript variant 1, mRNA | 9055 | NM_003981 |
| A:01168 | DIRAS family, GTP-binding RAS-like 3 (DIRAS3), mRNA | 9077 | NM_004675 |
| A:06043 | protein kinase, membrane associated tyrosine/threonine 1 (PKMYT1), transcript variant 1, mRNA | 9088 | NM_004203 |
| B:4778 | ubiquitin specific peptidase 8 (USP8),mRNA | 9101 | NM_005154 |
| B:8108 | LATS, large tumour suppressor, homolog 1 (Drosophila) (LATS1), mRNA | 9113 | NM_004690 |
| A:09436 | chondroitin sulfate proteoglycan 6 (bamacan) (CSPG6), mRNA | 9126 | NM_005445 |
| A:03606 | cyclin B2 (CCNB2), mRNA | 9133 | NM_004701 |
| A:10498 | cyclin E2 (CCNE2), transcript variant 1, mRNA | 9134 | NM_057749 |
| A:00971 | Rho guanine nucleotide exchange factor (GEF) 1 (ARHGEF1), transcript variant 2, mRNA | 9138 | NM_004706 |
| B:3843 | hepatocyte growth factor-regulated tyrosine kinase substrate (HGS), mRNA | 9146 | NM_004712 |
| A:03143 | exonuclease 1 (EXO1), transcript variant 1, mRNA | 9156 | NM_006027 |
| A:07881 | oncostatin M receptor (OSMR), mRNA | 9180 | NM_003999 |
| A:00335 | ZW10, kinetochore associated, homolog (Drosophila) (ZW10), mRNA | 9183 | NM_004724 |
| A:09747 | BUB3 budding uninhibited by benzimidazoles 3 homolog (yeast) (BUB3), transcript variant 1, mRNA | 9184 | NM_004725 |
| B:0692 | leucine-rich, glioma inactivated 1 (LGI1), mRNA | 9211 | NM_005097 |
| B:0692 | leucine-rich, glioma inactivated 1 (LGI1), mRNA | 9212 | NM_005097 |
| A:03609 | nucleolar and coiled-body phosphoprotein 1 (NOLC1), mRNA | 9221 | NM_004741 |
| A:04043 | discs, large homolog 5 (Drosophila) (DLG5), mRNA | 9231 | NM_004747 |
| A:05954 | pituitary tumour-transforming 1 (PTTG1), mRNA | 9232 | NM_004219 |
| B:0420 | transforming growth factor beta regulator 4 (TBRG4), transcript variant 1, mRNA | 9238 | NM_004749 |
| A:02479 | endothelial differentiation, sphingolipid G-protein-coupled receptor, 5 (EDG5), mRNA | 9294 | NM_004230 |
| A:06066 | Kruppel-like factor 4 (gut) (KLF4), mRNA | 9314 | NM_004235 |
| A:05541 | glucagon-like peptide 2 receptor (GLP2R), mRNA | 9340 | NM_004246 |
| A:00891 | WD repeat domain 39 (WDR39), mRNA | 9391 | NM_004804 |
| A:00519 | lymphocyte antigen 86 (LY86), mRNA | 9450 | NM_004271 |
| A:01180 | Rho-associated, coiled-coil containing protein kinase 2 (ROCK2), mRNA | 9475 | NM_004850 |
| A:01080 | kinesin family member 23 (KIF23), transcript variant 2, mRNA | 9493 | NM_004856 |
| A:04266 | ADAM metallopeptidase with thrombospondin type 1 motif, 1 (ADAMTS1), mRNA | 9510 | NM_006988 |
| B:9060 | tumour protein p53 inducible protein 11 (TP53I11), mRNA | 9537 | NM_006034 |
| A:04813 | breast cancer anti-estrogen resistance 1 (BCAR1), mRNA | 9564 | NM_014567 |
| A:09885 | M-phase phosphoprotein 1 (MPHOSPH1), mRNA | 9585 | NM_016195 |
| B:8184 | mediator of DNA damage checkpoint 1 (MDC1), mRNA | 9656 | NM_014641 |
| C:1135 | extra spindle poles like 1 (S. cerevisiae) (ESPL1), mRNA | 9700 | NM_012291 |
| C:0186 | histone deacetylase 9 (HDAC9), transcript variant 4, mRNA | 9734 | NM_178423 |
| A:05391 | kinetochore associated 1 (KNTC1), mRNA | 9735 | NM_014708 |
| B:0082 | histone deacetylase 4 (HDAC4), mRNA | 9759 | NM_006037 |
| B:0891 | metastasis suppressor 1 (MTSS1), mRNA | 9788 | NM_014751 |
| B:0062 | Rho guanine nucleotide exchange factor (GEF) 11 (ARHGEF11), transcript variant 1, mRNA | 9826 | NM_014784 |
| A:03269 | tousled-like kinase 1 (TLK1), mRNA | 9874 | NM_012290 |
| B:9335 | RAB GTPase activating protein 1-like (RABGAP1 L), transcript variant 1, mRNA | 9910 | NM_014857 |
| A:08624 | chromosome condensation-related SMC-associated protein 1 (CNAP1), mRNA | 9918 | NM_014865 |
| B:8937 | deleted in lung and esophageal cancer 1 (DLEC1), transcript variant DLEC1-L1, mRNA | 9940 | NM_007338 |
| B:8656 | major vault protein (MVP), transcript variant 1, mRNA | 9961 | NM_017458 |
| A:02173 | tumour necrosis factor (ligand) superfamily, member 15 (TNFSF15), mRNA | 9966 | NM_005118 |
| A:05257 | fibroblast growth factor binding protein 1 (FGFBP1), mRNA | 9982 | NM_005130 |
| A:00752 | REC8-like 1 (yeast) (REC8L1), mRNA | 9985 | NM_005132 |
| A:01592 | solute carrier family 12 (potassium/chloride transporters), member 6 (SLC12A6), mRNA | 9990 | NM_005135 |
| A:04645 | abl-interactor 1 (ABI1), transcript variant 1, mRNA | 10006 | NM_005470 |
| A:10156 | histone deacetylase 6 (HDAC6), mRNA | 10013 | NM_006044 |
| B:2818 | histone deacetylase 5 HDAC5 | 10014 | NM_001015053, NM_005474 |
| A:10510 | chromatin assembly factor 1, subunit A (p150) (CHAF1A), mRNA | 10036 | NM_005483 |
| A:05648 | SMC4 structural maintenance of chromosomes 4-like 1 (yeast) (SMC4L1), transcript variant 3, mRNA | 10051 | NM_001002799 |
| B:0675 | tetraspanin 5 (TSPAN5), mRNA | 10098 | NM_005723 |
| B:0685 | tetraspanin 3 (TSPAN3), transcript variant 1, mRNA | 10099 | NM_005724 |
| A:08229 | tetraspanin 2 (TSPAN2), mRNA | 10100 | NM_005725 |
| A:02634 | tetraspanin 1 (TSPAN1), mRNA | 10103 | NM_005727 |
| A:07852 | RAD50 homolog (S. cerevisiae) (RAD50), transcript variant 1, mRNA | 10111 | NM_005732 |
| B:4820 | pre-B-cell colony enhancing factor 1 (PBEF1), transcript variant 1, mRNA | 10135 | NM_005746 |
| B:7911 | transducer of ERBB2, 1 (TOB1), mRNA | 10140 | NM_005749 |
| B:0969 | odz, odd Oz/ten-m homolog 1(Drosophila) (ODZ1), mRNA | 10178 | NM_014253 |
| A:06242 | RNA binding motif protein 7 (RBM7), mRNA | 10179 | NM_016090 |
| A:03840 | RNA binding motif protein 5 (RBM5), mRNA | 10181 | NM_005778 |
| B:8194 | M-phase phosphoprotein 9 MPHOSPH9 | 10198 | NM_022782 |
| A:09658 | M-phase phosphoprotein 6 (MPHOSPH6), mRNA | 10200 | NM_005792 |
| A:04009 | ret finger protein 2 (RFP2), transcript variant 1, mRNA | 10206 | NM_005798 |
| A:03270 | proteoglycan 4 (PRG4), mRNA | 10216 | NM_005807 |
| A:01614 | A kinase (PRKA) anchor protein 8 (AKAP8), mRNA | 10270 | NM_005858 |
| B:5575 | stromal antigen 1 (STAG1), mRNA | 10274 | NM_005862 |
| B:8332 | aortic preferentially expressed gene 1 APEG1 | 10290 | XM_001131579, XM_001128413 |
| A:04828 | DnaJ (Hsp40) homolog, subfamily A, member 2 (DNAJA2), mRNA | 10294 | NM_005880 |
| B:0667 | katanin p80 (WD repeat containing) subunit B 1 (KATNB1), mRNA | 10300 | NM_005886 |
| A:04635 | deleted in lymphocytic leukaemia, 1 (DLEU1) on chromosome 13 | 10301 | NR_002605 |
| B:2626 | uracil-DNA glycosylase 2 (UNG2), transcript variant 1, mRNA | 10309 | NM_021147 |
| A:09675 | T-cell, immune regulator 1, ATPase, H+ transporting, lysosomal protein a isoform 3 (TCIRG1), transcript variant 1, mRNA | 10312 | NM_006019 |
| A:09047 | nucleophosmin/nucleoplasmin, 3 (NPM3), mRNA | 10361 | NM_006993 |
| A:04517 | synaptonemal complex protein 2 (SYCP2), mRNA | 10388 | NM_014258 |
| A:06405 | anaphase promoting complex subunit 10 (ANAPC10), mRNA | 10393 | NM_014885 |
| A:04338 | phosphatidylethanolamine N-methyltransferase (PEMT), nuclear gene encoding mitochondrial protein, transcript variant 2, mRNA | 10400 | NM_007169 |
| A:10053 | kinetochore associated 2 (KNTC2), mRNA | 10403 | NM_006101 |
| A:08539 | Rap guanine nucleotide exchange factor (GEF) 3 (RAPGEF3), mRNA | 10411 | NM_006105 |
| A:01717 | SKB1 homolog (S. pombe) (SKB1), mRNA | 10419 | NM_006109 |
| B:6182 | RNA binding motif protein 14 (RBM14), mRNA | 10432 | NM_006328 |
| B:4641 | glycoprotein (transmembrane) nmb GPNMB | 10457 | NM_001005340, NM_002510 |
| A:10829 | MAD2 mitotic arrest deficient-like 2 (yeast) (MAD2L2), mRNA | 10459 | NM_006341 |
| A:01067 | transcriptional adaptor 3 (NGG1 homolog, yeast)-like (TADA3L), transcript variant 1, mRNA | 10474 | NM_006354 |
| A:00010 | vesicle transport through interaction with t-SNAREs homolog 1 B (VTI1B), mRNA | 10490 | NM_006370 |
| B:1984 | cartilage associated protein (CRTAP), mRNA | 10491 | NM_006371 |
| A:07616 | Sjogren's syndrome/scleroderma autoantigen 1 (SSSCA1), mRNA | 10534 | NM_006396 |
| A:04760 | ribonuclease H2, large subunit (RNASEH2A), mRNA | 10535 | NM_006397 |
| A:10701 | dynactin 2 (p50) (DCTN2), mRNA | 10540 | NM_006400 |
| A:04950 | chaperonin containing TCP1, subunit 7 (eta) (CCT7), transcript variant 1, mRNA | 10574 | NM_006429 |
| A:04081 | chaperonin containing TCP1, subunit 4 (delta) (CCT4), mRNA | 10575 | NM_006430 |
| A:09500 | chaperonin containing TCP1, subunit 2 (beta) (CCT2), mRNA | 10576 | NM_006431 |
| A:09726 | chromosome 6 open reading frame 108 (C6orf108), transcript variant 1, mRNA | 10591 | NM_006443 |
| A:10196 | SMC2 structural maintenance of chromosomes 2-like 1 (yeast) (SMC2L1), mRNA | 10592 | NM_006444 |
| B:1048 | ubiquitin specific peptidase 16 (USP16), transcript variant 1, mRNA | 10600 | NM_006447 |
| A:08296 | MAX dimerization protein 4 (MXD4), mRNA | 10608 | NM_006454 |
| A:05163 | synaptonemal complex protein SC65 (SC65), mRNA | 10609 | NM_006455 |
| A:04356 | STAM binding protein (STAMBP), transcript variant 1, mRNA | 10617 | NM_006463 |
| B:3717 | growth arrest-specific 2 like 1 (GAS2L1), transcript variant 1, mRNA | 10634 | NM_006478 |
| A:01918 | S-phase response (cyclin-related) (SPHAR), mRNA | 10638 | NM_006542 |
| A:04374 | KH domain containing, RNA binding, signal transduction associated 1 (KHDRBS1), mRNA | 10657 | NM_006559 |
| A:08738 | CCCTC-binding factor (zinc finger protein) (CTCF), mRNA | 10664 | NM_006565 |
| A:08733 | cell growth regulator with ring finger domain 1 (CGRRF1), mRNA | 10668 | NM_006568 |
| A:07876 | cell growth regulator with EF-hand domain 1 (CGREF1), mRNA | 10669 | NM_006569 |
| A:05572 | tumour necrosis factor (ligand) superfamily, member (TNFSF13B), mRNA | 10673 | NM_006573 |
| B:4752 | polymerase (DNA-directed), delta 3, accessory subunit (POLD3), mRNA | 10714 | NM_006591 |
| B:3500 | polymerase (DNA directed), theta (POLQ), mRNA | 10721 | NM_199420 |
| A:03035 | nuclear distribution gene C homolog (A. nidulans) (NUDC), mRNA | 10726 | NM_006600 |
| A:00069 | transcription factor-like 5 (basic helix-loop-helix) (TCFL5), mRNA | 10732 | NM_006602 |
| B:7543 | polo-like kinase 4 (Drosophila) (PLK4), mRNA | 10733 | NM_014264 |
| B:2404 | stromal antigen 3 (STAG3), mRNA | 10734 | NM_012447 |
| A:10760 | stromal antigen 2 (STAG2), mRNA | 10735 | NM_006603 |
| B:5933 | transducer of ERBB2, 2 (TOB2), mRNA | 10766 | NM_016272 |
| A:02195 | polo-like kinase 2 (Drosophila) (PLK2), mRNA | 10769 | NM_006622 |
| A:04982 | zinc finger, MYND domain containing 11 (ZMYND11), transcript variant 1, mRNA | 10771 | NM_006624 |
| B:2320 | septin 9 (SEPT9), mRNA | 10801 | NM_006640 |
| A:07660 | thioredoxin-like 4A (TXNL4A), mRNA | 10907 | NM_006701 |
| B:9218 | SGT1, suppressor of G2 allele of SKP1 (S. cerevisiae) (SUGT1), mRNA | 10910 | NM_006704 |
| A:08320 | DBF4 homolog (S. cerevisiae) (DBF4), mRNA | 10926 | NM_006716 |
| A:08852 | spindlin (SPIN), mRNA | 10927 | NM_006717 |
| A:00006 | BTG family, member 3 (BTG3), mRNA | 10950 | NM_006806 |
| A:01860 | cytoskeleton-associated protein 4 (CKAP4), mRNA | 10971 | NM_006825 |
| A:01595 | microtubule-associated protein, RP/EB family, member 2 (MAPRE2), transcript variant 5, mRNA | 10982 | NM_014268 |
| A:05220 | cyclin 1 (CCNI), mRNA | 10983 | NM_006835 |
| B:4359 | kinesin family member 2C (KIF2C), mRNA | 11004 | NM_006845 |
| A:09969 | tousled-like kinase 2 (TLK2), mRNA | 11011 | NM_006852 |
| A:04957 | polymerase (DNA directed) sigma (POLS), mRNA | 11044 | NM_006999 |
| A:01776 | ubiquitin-conjugating enzyme E2C (UBE2C), transcript variant 1, mRNA | 11065 | NM_007019 |
| A:09200 | cytochrome b-561 domain containing 2 (CYB561D2), mRNA | 11068 | NM_007022 |
| A:00904 | topoisomerase (DNA) II binding protein 1 (TOPBP1), mRNA | 11073 | NM_007027 |
| B:1407 | ADAM metallopeptidase with thrombospondin type 1 motif, 8 (ADAMTS8), mRNA | 11095 | NM_007037 |
| A:09918 | katanin p60 (ATPase-containing) subunit A 1 (KATNA1), | 11104 | NM_007044 |
| A:09825 | PR domain containing 4 (PRDM4), mRNA | 11108 | NM_012406 |
| B:7528 | FGFR1 oncogene partner (FGFR1OP), transcript variant 1, mRNA | 11116 | NM_007045 |
| A:04279 | CD160 antigen (CD160), mRNA | 11126 | NM_007053 |
| C:4275 | TBC1 domain family, member 8 (with GRAM domain) (TBC1D8), mRNA | 11138 | NM_007063 |
| A:03486 | CDC37 cell division cycle 37 homolog (S. cerevisiae) (CDC37), mRNA | 11140 | NM_007065 |
| A:06143 | MYST histone acetyltransferase 2 (MYST2), mRNA | 11143 | NM_007067 |
| A:06472 | DMC1 dosage suppressor of mck1 homolog, meiosis-specific homologous recombination (yeast) (DMC1), mRNA | 11144 | NM_007068 |
| A:07181 | coronin, actin binding protein, 1A (CORO1A), mRNA | 11151 | NM_007074 |
| A:04421 | Huntingtin interacting protein E (HYPE), mRNA | 11153 | NM_007076 |
| A:03200 | PC4 and SFRS1 interacting protein 1 (PSIP1), transcript variant 2, mRNA | 11168 | NM_033222 |
| C:0370 | centrosomal protein 2 (CEP2), transcript variant 1, | 11190 | NM_007186 |
| C:0370 | centrosomal protein 2 (CEP2), transcript variant 1, mRNA | 11191 | NM_007186 |
| A:02177 | CHK2 checkpoint homolog (S. pombe) (CHEK2), transcript variant 1, mRNA | 11200 | NM_007194 |
| A:09335 | polymerase (DNA directed), gamma 2, accessory subunit (POLG2), mRNA | 11232 | NM_007215 |
| A:08008 | dynactin 3 (p22) (DCTN3), transcript variant 2, mRNA | 11258 | NM_024348 |
| B:7247 | three prime repair exonuclease 1 (TREX1), transcript variant 2, mRNA | 11277 | NM_033627 |
| A:03276 | polynucleotide kinase 3'-phosphatase (PNKP), mRNA | 11284 | NM_007254 |
| A:01322 | Parkinson disease (autosomal recessive, early onset) 7 (PARK7), mRNA | 11315 | NM_007262 |
| B:5525 | PDGFA associated protein 1 (PDAP1), mRNA | 11333 | NM_014891 |
| A:05117 | tumour suppressor candidate 2 (TUSC2), mRNA | 11334 | NM_007275 |
| A:08584 | activating transcription factor 5 (ATF5), mRNA | 22809 | NM_012068 |
| A:10029 | KIAA0971 (KIAA0971), mRNA | 22868 | NM_014929 |
| C:4180 | DENN/MADD domain containing 3 (DENND3), mRNA | 22898 | NM_014957 |
| A:07655 | microtubule-associated protein, RP/EB family, member 1 (MAPRE1), mRNA | 22919 | NM_012325 |
| A:02013 | sirtuin (silent mating type information regulation 2 homolog) 2 (S. cerevisiae) (SIRT2), transcript variant 2, mRNA | 22933 | NM_030593 |
| A:07965 | TPX2, microtubule-associated, homolog (Xenopus laevis) (TPX2), mRNA | 22974 | NM_012112 |
| B:1032 | apoptotic chromatin condensation inducer 1 ACIN1 | 22985 | NM_014977 |
| A:10375 | androgen-induced proliferation inhibitor (APRIN), transcript variant 1, mRNA | 23047 | NM_015032 |
| A:04696 | nuclear receptor coactivator 6 (NCOA6), mRNA | 23054 | NM_014071 |
| A:09165 | KIAA0676 protein (KIAA0676), transcript variant 1, mRNA | 23061 | NM_198868 |
| B:4976 | KIAA0261 (KIAA0261), mRNA | 23063 | NM_015045 |
| B:8950 | KIAA0241 protein (KIAA0241), mRNA | 23080 | NM_015060 |
| C:2458 | p53-associated parkin-like cytoplasmic protein (PARC), mRNA | 23113 | NM_015089 |
| B:9549 | SMC5 structural maintenance of chromosomes 5-like 1 (yeast) (SMC5L1), mRNA | 23137 | NM_015110 |
| B:4428 | septin 6 (SEPT6), transcript variant I, mRNA | 23157 | NM_145799 |
| B:6278 | KIAA0882 protein (KIAA0882), mRNA | 23158 | NM_015130 |
| B:1443 | septin 8 (SEPT8), mRNA | 23176 | XM_034872 |
| B:8136 | ankyrin repeat domain 15 (ANKRD15), transcript variant 1, mRNA | 23189 | NM_015158 |
| B:4969 | KIAA1086 (KIAA1086), mRNA | 23217 | XM_001130130, XM_001130674 |
| A:10369 | phospholipase C, beta 1 (phosphoinositide-specific) (PLCB1), transcript variant 2, mRNA | 23236 | NM_182734 |
| B:0524 | RAB6 interacting protein 1 (RAB6IP1), mRNA | 23258 | NM_015213 |
| B:0230 | inducible T-cell co-stimulator ligand ICOSLG | 23308 | NM_015259 |
| B:0327 | SAM and SH3 domain containing 1 (SASH1), mRNA | 23328 | NM_015278 |
| B:5714 | KIAA0650 protein (KIAA0650), mRNA | 23347 | XM_113962, XM_938891 |
| B:8897 | formin binding protein 4 (FNBP4), mRNA | 23360 | NM_015308 |
| B:8228 | barren homolog 1 (Drosophila) (BRRN1), mRNA | 23397 | NM_015341 |
| B:9601 | ATPase type 13A2 (ATP13A2), mRNA | 23401 | NM_022089 |
| B:7418 | TAR DNA binding protein (TARDBP), mRNA | 23435 | NM_007375 |
| B:7878 | microtubule-actin crosslinking factor 1 (MACF1), transcript variant 1, mRNA | 23499 | NM_012090 |
| A:09105 | RNA binding motif protein 9 (RBM9), transcript variant 2, mRNA | 23543 | NM_014309 |
| B:1165 | origin recognition complex, subunit 6 homolog-like (yeast) (ORC6L), mRNA | 23594 | NM_014321 |
| B:3180 | origin recognition complex, subunit 3-like (yeast) (ORC3L), transcript variant 2, mRNA | 23595 | NM_012381 |
| A:00473 | SPO11 meiotic protein covalently bound to DSB-like (S. cerevisiae) (SPO11), transcript variant 1, mRNA | 23626 | NM_012444 |
| A:02179 | RAB GTPase activating protein 1 (RABGAP1), mRNA | 23637 | NM_012197 |
| A:06494 | leucine zipper, down-regulated in cancer 1 (LDOC1), mRNA | 23641 | NM_012317 |
| B:2198 | protein phosphatase 1, regulatory (inhibitor) subunit 15A (PPP1R15A), mRNA | 23645 | NM_014330 |
| C:3173 | polymerase (DNA directed), alpha 2 (70kD subunit) (POLA2), mRNA | 23649 | NM_002689 |
| A:03098 | SH3-domain binding protein 4 (SH3BP4), mRNA | 23677 | NM_014521 |
| C:1904 | N-acetyltransferase 6 (NAT6), mRNA | 24142 | NM_012191 |
| C:2118 | unc-84 homolog B (C. elegans) (UNC84B), mRNA | 25777 | NM_015374 |
| A:05344 | RAD54 homolog B (S. cerevisiae) (RAD54B), transcript variant 1, mRNA | 25788 | NM_012415 |
| A:06762 | CDKN1A interacting zinc finger protein 1 (CIZ1), mRNA | 25792 | NM_012127 |
| C:4297 | Nipped-B homolog (Drosophila) (NIPBL), transcript variant B, mRNA | 25836 | NM_015384 |
| A:09401 | preimplantation protein 3 (PREI3), transcript variant 1, mRNA | 25843 | NM_015387 |
| B:3103 | breast cancer metastasis suppressor 1 (BRMS1), transcript variant 1, mRNA | 25855 | NM_015399 |
| A:01151 | protein kinase D2 (PRKD2), mRNA | 25869 | NM_016457 |
| A:07688 | EGF-like-domain, multiple 6 (EGFL6), mRNA | 25975 | NM_015507 |
| B:6248 | ankyrin repeat domain 17 (ANKRD17), transcript variant 1, mRNA | 26057 | NM_032217 |
| A:02605 | adaptor protein containing pH domain, PTB domain and leucine zipper motif 1 (APPL), mRNA | 26060 | NM_012096 |
| A:02500 | ets homologous factor (EHF), mRNA | 26298 | NM_012153 |
| A:09724 | mutL homolog 3 (E. coli) (MLH3), mRNA | 27030 | NM_014381 |
| A:06200 | lysosomal-associated membrane protein 3 (LAMP3), mRNA | 27074 | NM_014398 |
| A:00686 | tetraspanin 13 (TSPAN13), mRNA | 27075 | NM_014399 |
| A:02984 | calcyclin binding protein (CACYBP), transcript variant 1, mRNA | 27101 | NM_014412 |
| A:00435 | eukaryotic translation initiation factor 2-alpha kinase 1 (EIF2AK1), mRNA | 27104 | NM_014413 |
| C:8169 | SMC1 structural maintenance of chromosomes 1-like 2 (yeast) (SMC1L2), mRNA | 27127 | NM_148674 |
| A:00927 | sestrin 1 (SESN1), mRNA | 27244 | NM_014454 |
| A:01831 | RNA binding motif, single stranded interacting protein (RBMS3), transcript variant 2, mRNA | 27303 | NM_014483 |
| A:06053 | zinc finger protein 330 (ZNF330), mRNA | 27309 | NM_014487 |
| A:03501 | down-regulated in metastasis (DRIM), mRNA | 27340 | NM_014503 |
| B:3842 | polymerase (DNA directed), lambda (POLL), mRNA | 27343 | NM_013274 |
| B:6569 | polymerase (DNA directed), mu (POLM), mRNA | 27434 | NM_013284 |
| B:4351 | echinoderm microtubule associated protein like 4 (EML4), mRNA | 27436 | NM_019063 |
| B:1612 | cat eye syndrome chromosome region, candidate 4 CECR4 | 27443 | AF307448 |
| A:08058 | protein phosphatase 2 (formerly 2A), regulatory subunit B", beta (PPP2R3B), transcript variant 1, mRNA | 28227 | NM_013239 |
| A:09647 | response gene to complement 32 (RGC32), mRNA | 28984 | NM_014059 |
| A:09821 | malignant T cell amplified sequence 1 (MCTS1), mRNA | 28985 | NM_014060 |
| B:6485 | HSPC135 protein (HSPC135), transcript variant 1, mRNA | 29083 | NM_014170 |
| A:09945 | PYD and CARD domain containing (PYCARD), transcript variant 1, mRNA | 29108 | NM_013258 |
| C:1944 | lectin, galactoside-binding, soluble, 13 (galectin 13) (LGALS13), mRNA | 29124 | NM_013268 |
| A:02160 | CD274 antigen (CD274), mRNA | 29126 | NM_014143 |
| A:08075 | replication initiator 1 (REPIN1), transcript variant 1, mRNA | 29803 | NM_013400 |
| B:1479 | anaphase promoting complex subunit 2 (ANAPC2), mRNA | 29882 | NM_013366 |
| A:08657 | protein predicted by clone 23882 (HSU79303), mRNA | 29903 | NM_013301 |
| A:10453 | replication protein A4, 34kDa (RPA4), mRNA | 29935 | NM_013347 |
| A:02862 | anaphase promoting complex subunit 4 (ANAPC4), mRNA | 29945 | NM_013367 |
| A:10100 | SERTA domain containing 1 (SERTAD1), | 29950 | NM_013376 |
| A:05316 | striatin, calmodulin binding protein 3 (STRN3), mRNA | 29966 | NM_014574 |
| A:06440 | G0/G1switch 2 (G0S2), mRNA | 50486 | NM_015714 |
| A:08113 | deleted in esophageal cancer 1 (DEC1), mRNA | 50514 | NM_017418 |
| B:7919 | hepatoma-derived growth factor, related protein 3 (HDGFRP3), mRNA | 50810 | NM_016073 |
| A:07482 | par-6 partitioning defective 6 homolog alpha (C. elegans) (PARD6A), transcript variant 1, mRNA | 50855 | NM_016948 |
| A:03435 | geminin, DNA replication inhibitor (GMNN), mRNA | 51053 | NM_015895 |
| A:00171 | ribosomal protein S27-like (RPS27L), mRNA | 51065 | NM_015920 |
| B:1459 | EGF-like-domain, multiple 7 (EGFL7), transcript variant 1, mRNA | 51162 | NM_016215 |
| A:09081 | tubulin, epsilon 1 (TUBE1), mRNA | 51175 | NM_016262 |
| A:08522 | hect domain and RLD 5 (HERC5), mRNA | 51191 | NM_016323 |
| A:05174 | phospholipase C, epsilon 1 (PLCE1), mRNA | 51196 | NM_016341 |
| B:3533 | dual specificity phosphatase 13 DUSP13 | 51207 | NM_001007271, NM_001007272, NM_001007273, NM_001007274, NM_001007275, NM_016364 |
| A:06537 | ABI gene family, member 3 (ABI3), mRNA | 51225 | NM_016428 |
| A:03107 | transcription factor Dp family, member 3 (TFDP3), mRNA | 51270 | NM_016521 |
| A:09430 | SCAN domain containing 1 (SCAND1), transcript variant 1, mRNA | 51282 | NM_016558 |
| B:9657 | CD320 antigen (CD320), mRNA | 51293 | NM_016579 |
| A:07215 | fizzy/cell division cycle 20 related 1 (Drosophila) (FZR1), mRNA | 51343 | NM_016263 |
| A:06101 | Wilms tumour upstream neighbor 1 (WIT1), mRNA | 51352 | NM_015855 |
| A:10614 | E3 ubiquitin protein ligase, HECT domain containing, 1 (EDD1), mRNA | 51366 | NM_015902 |
| B:9794 | anaphase promoting complex subunit 5 (ANAPC5), mRNA | 51433 | NM_016237 |
| B:1481 | anaphase promoting complex subunit 7 (ANAPC7), mRNA | 51434 | NM_016238 |
| A:08459 | G-2 and S-phase expressed 1 (GTSE1), mRNA | 51512 | NM_016426 |
| A:02842 | APC11 anaphase promoting complex subunit 11 homolog (yeast) (ANAPC11), transcript variant 2, mRNA | 51529 | NM_0164760 |
| B:2670 | histone deacetylase 7A HDAC7A | 51564 | NM_015401, |
| A:07829 | ubiquitin-conjugating enzyme E2D 4 (putative) (UBE2D4), mRNA | 51619 | NM_015983 |
| A:09440 | CDK5 regulatory subunit associated protein 1 (CDK5RAP1), transcript variant 2, mRNA | 51654 | NM_016082 |
| B:1035 | DNA replication complex GINS protein PSF2 (Pfs2), mRNA | 51659 | NM_016095 |
| B:9464 | sterile alpha motif and leucine zipper containing kinase AZK (ZAK), transcript variant 2, mRNA | 51776 | NM_133646 |
| B:7871 | ZW10 interactor antisense ZWINTAS | 53588 | X98261 |
| B:3431 | RNA binding motif protein 11 (RBM11), mRNA | 54033 | NM_144770 |
| A:02209 | polymerase (DNA directed), epsilon 3 (p17 subunit) (POLE3), mRNA | 54107 | NM_017443 |
| A:04070 | DKFZp434A0131 protein DKFZP434A0131 | 54441 | NM_018991 |
| A:05280 | anillin, actin binding protein (scraps homolog, Drosophila) (ANLN), mRNA | 54443 | NM_018685 |
| A:06475 | spindlin family, member 2 (SPIN2), mRNA | 54466 | NM_019003 |
| A:03960 | cyclin J (CCNJ), mRNA | 54619 | NM_019084 |
| B:3841 | M-phase phosphoprotein, mpp8 (HSMPP8), mRNA | 54737 | NM_017520 |
| B:8673 | ropporin, rhophilin associated protein 1 (ROPN1), | 54763 | NM_017578 |
| A:02474 | B-cell translocation gene 4 (BTG4), mRNA | 54766 | NM_017589 |
| B:2084 | G patch domain containing 4 (GPATC4), transcript variant 2, mRNA | 54865 | NM_182679 |
| A:06639 | hypothetical protein FLJ20422 (FLJ20422), mRNA | 54929 | NM_017814 |
| C:2265 | thioredoxin-like 4B (TXNL4B), mRNA | 54957 | NM_017853 |
| B:7809 | PIN2-interacting protein 1 (PINX1), mRNA | 54984 | NM_017884 |
| B:8204 | polybromo 1 (PB1), transcript variant 2, mRNA | 55193 | NM_018313 |
| A:03321 | hypothetical protein FLJ10781 (FLJ10781), mRNA | 55228 | NM_018215 |
| B:2270 | MOB1, Mps One Binder kinase activator-like 1 B (yeast) MOBK1 B | 55233 | NM_018221 |
| A:08002 | signal-regulatory protein beta 2 (SIRPB2), transcript variant 1, mRNA | 55423 | NM_018556 |
| A:03524 | tripartite motif-containing 36 (TRIM36), transcript variant 1, mRNA | 55522 | NM_018700 |
| A:09474 | chromosome 2 open reading frame 29 (C2orf29), mRNA | 55571 | NM_017546 |
| A:05414 | hypothetical protein H41 (H41), mRNA | 55573 | NM_017548 |
| B:2133 | CDC37 cell division cycle 37 homolog (S. cerevisiae)-like 1 (CDC37L1), mRNA | 55664 | NM_017913 |
| B:8413 | Nedd4 binding protein 2 (N4BP2), mRNA | 55728 | NM_018177 |
| A:02898 | checkpoint with forkhead and ring finger domains (CHFR), mRNA | 55743 | NM_018223 |
| A:07468 | septin 11 (SEPT11), mRNA | 55752 | NM_018243 |
| B:2252 | chondroitin beta1,4 N-acetylgalactosaminyltransferase (ChGn), mRNA | 55790 | NM_018371 |
| C:0033 | B double prime 1, subunit of RNA polymerase III transcription initiation factor IIIB BDP1 | 55814 | NM_018429 |
| A:03912 | PDZ binding kinase (PBK), mRNA | 55872 | NM_018492 |
| A:10308 | unc-45 homolog A (C. elegans) (UNC45A), transcript variant 1, mRNA | 55898 | NM_017979 |
| A:02027 | bridging integrator 3 (BIN3), mRNA | 55909 | NM_018688 |
| C:0655 | erbb2 interacting protein ERBB2IP | 55914 | NM_001006600, NM_018695 |
| B:1503 | septin 3 (SEPT3), transcript variant C, mRNA | 55964 | NM_145734 |
| B:8446 | gastrokine 1 (GKN1), mRNA | 56287 | NM_019617 |
| A:00073 | par-3 partitioning defective 3 homolog (C. elegans) (PARD3), mRNA | 56288 | NM_019619 |
| A:03990 | CTP synthase II (CTPS2), transcript variant 1, mRNA | 56475 | NM_019857 |
| B:8449 | BRCA2 and CDKN1A interacting protein (BCCIP), transcript variant B, mRNA | 56647 | NM_078468 |
| B:1203 | interferon, kappa (IFNK), mRNA | 56832 | NM_020124 |
| B:1205 | SLAM family member 8 (SLAMF8), mRNA | 56833 | NM_020125 |
| A:00149 | sphingosine kinase 2 (SPHK2), mRNA | 56848 | NM_020126 |
| A:04220 | Werner helicase interacting protein 1 (WRNIP1), transcript variant 1, mRNA | 56897 | NM_020135 |
| A:09095 | latexin (LXN), mRNA | 56925 | NM_020169 |
| A:02450 | dual specificity phosphatase 22 (DUSP22), mRNA | 56940 | NM_020185 |
| C:0975 | DC13 protein (DC13), mRNA | 56942 | NM_020188 |
| A:04008 | 5',3'-nucleotidase, mitochondrial (NT5M), nuclear gene encoding mitochondrial protein, mRNA | 56953 | NM_020201 |
| A:01586 | kinesin family member 15 (KIF15), mRNA | 56992 | NM_020242 |
| B:0396 | catenin, beta interacting protein 1 (CTNNBIP1), transcript variant 1, mRNA | 56998 | NM_020248 |
| B:3508 | cyclin L1 (CCNL1), mRNA | 57018 | NM_020307 |
| A:06501 | cholinergic receptor, nicotinic, alpha polypeptide 10 (CHRNA10), mRNA | 57053 | NM_020402 |
| B:7311 | poly(rC) binding protein 4 (PCBP4), transcript variant 1, mRNA | 57060 | NM_020418 |
| A:08184 | chromosome 1 open reading frame 128 (C1orf128), mRNA | 57095 | NM_020362 |
| B:3446 | S100 calcium binding protein A14 (S100A14), mRNA | 57402 | NM_020672 |
| C:5669 | odz, odd Oz/ten-m homolog 2 (Drosophila) (ODZ2), mRNA | 57451 | XM_047995, XM_931456, XM_942208, XM_945786, XM_945788 |
| B:8403 | membrane-associated ring finger (C3HC4) 4 (MARCH4), mRNA | 57574 | NM_020814 |
| B:1442 | polymerase (DNA-directed), delta 4 (POLD4), mRNA | 57804 | NM_021173 |
| B:1448 | prokineticin 2 (PROK2), mRNA | 60675 | NM_021935 |
| B:4091 | CTF18, chromosome transmission fidelity factor 18 homolog (S. cerevisiae) (CHTF18), mRNA | 63922 | NM_022092 |
| C:0644 | TSPY-like 2 (TSPYL2), mRNA | 64061 | NM_022117 |
| B:6809 | chromosome 10 open reading frame 54 (C10orf54), mRNA | 64115 | NM_022153 |
| A:10488 | chromosome condensation protein G (HCAP-G), mRNA | 64151 | NM_022346 |
| A:10186 | spermatogenesis associated 1 (SPATA1), mRNA | 64173 | NM_022354 |
| A:02978 | DNA cross-link repair 1C (PSO2 homolog, S. cerevisiae) (DCLRE1C), transcript variant b, mRNA | 64421 | NM_022487 |
| A:10112 | anaphase promoting complex subunit 1 (ANAPC1), mRNA | 64682 | NM_022662 |
| A:10470 | FLJ20859 gene (FLJ20859), transcript variant 1, mRNA | 64745 | NM_001029991 |
| B:3988 | interferon stimulated exonuclease gene 20kDa-like 1 (ISG20L1), mRNA | 64782 | NM_022767 |
| A:06358 | DNA cross-link repair 1 B (PSO2 homolog, S. cerevisiae) (DCLRE1B), mRNA | 64858 | NM_022836 |
| A:10073 | centromere protein H (CENPH), mRNA | 64946 | NM_022909 |
| A:05903 | chromosome 16 open reading frame 24 (C16orf24), mRNA | 65990 | NM_023933 |
| A:07975 | spermatogenesis associated 5-like 1 (SPATA5L1), mRNA | 79029 | NM_024063 |
| A:01368 | hypothetical protein MGC5297 (MGC5297), | 79072 | NM_024091 |
| C:1382 | basic helix-loop-helix domain containing, class B, 3 (BHLHB3), mRNA | 79365 | NM_030762 |
| A:00699 | NADPH oxidase, EF-hand calcium binding domain 5 (NOX5), mRNA | 79400 | NM_024505 |
| A:05363 | SMC6 structural maintenance of SMC6 structural chromosomes 6-like 1 (yeast) (SMC6L1), mRNA | 79677 | NM_024624 |
| A:09775 | V-set domain containing T cell activation inhibitor 1 (VTCN1), mRNA | 79679 | NM_024626 |
| B:6021 | hypothetical protein FLJ21125 (FLJ21125), mRNA | 79680 | NM_024627 |
| A:06447 | Sin3A associated protein p30-like (SAP30L), mRNA | 79685 | NM_024632 |
| A:08767 | suppressor of variegation 3-9 homolog 2 (Drosophila) (SUV39H2), mRNA | 79723 | NM_024670 |
| A:01156 | chromosome 15 open reading frame 29 (C15orf29), mRNA | 79768 | NM_024713 |
| A:03654 | hypothetical protein FLJ 13273 (FLJ13273), transcript variant 1, mRNA | 79807 | NM_001031720 |
| A:10726 | hypothetical protein FLJ13265 (FLJ13265), mRNA | 79935 | NM_024877 |
| B:2392 | Dbf4-related factor 1 (DRF1), transcript variant 2, mRNA | 80174 | NM_025104 |
| B:2358 | SMP3 mannosyltransferase (SMP3), mRNA | 80235 | NM_025163 |
| A:02900 | CDK5 regulatory subunit associated protein 3 (CDK5RAP3), transcript variant 2, mRNA | 80279 | NM_025197 |
| C:0025 | leucine rich repeat containing 27 (LRRC27), mRNA | 80313 | NM_030626 |
| B:9631 | ADAM metallopeptidase domain 33 (ADAM33), transcript variant 1, mRNA | 80332 | NM_025220 |
| B:6501 | CD276 antigen (CD276), transcript variant 2, mRNA | 80381 | NM_025240 |
| A:05386 | hypothetical protein MGC10334 (MGC10334), mRNA | 80772 | NM_001029885 |
| A:08918 | collagen, type XVIII, alpha 1 (COL18A1), transcript variant 1, mRNA | 80781 | NM_030582 |
| C:0358 | EGF-like-domain, multiple 8 (EGFL8), mRNA | 80864 | NM_030652 |
| B:1020 | C/EBP-induced protein (LOC81558), mRNA | 81558 | NM_030802 |
| B:3550 | DNA replication factor (CDT1), mRNA | 81620 | NM_030928 |
| B:5661 | cyclin L2 (CCNL2), mRNA | 81669 | NM_030937 |
| B:1735 | exonuclease NEF-sp (LOC81691), mRNA | 81691 | NM_030941 |
| B:2768 | ring finger protein 146 (RNF146), mRNA | 81847 | NM_030963 |
| B:2350 | interferon stimulated exonuclease gene 20kDa-like 2 (ISG20L2), mRNA | 81875 | NM_030980 |
| B:3823 | Cdk5 and Abl enzyme substrate 2 (CABLES2), mRNA | 81928 | NM_031215 |
| B:8839 | leucine rich repeat containing 48 (LRRC48), mRNA | 83450 | NM_031294 |
| B:9709 | katanin p60 subunit A-like 2 (KATNAL2), mRNA | 83473 | NM_031303 |
| B:8709 | sestrin 2 (SESN2), mRNA | 83667 | NM_031459 |
| B:8721 | CD99 antigen-like 2 (CD99L2), transcript variant 1, mRNA | 83692 | NM_031462 |
| C:0565 | regenerating islet-derived family, member 4 (REG4), mRNA | 83998 | NM_032044 |
| B:3599 | katanin p60 subunit A-like 1 (KATNAL1), transcript variant 1, mRNA | 84056 | NM_032116 |
| B:3492 | GAJ protein (GAJ), mRNA | 84057 | NM_032117 |
| A:00224 | IQ motif containing G (IQCG), mRNA | 84223 | NM_032263 |
| C:1051 | hypothetical protein MGC10911 (MGC10911), mRNA | 84262 | NM_032302 |
| B:1756 | prokineticin 1 (PROK1), mRNA | 84432 | NM_032414 |
| B:3029 | MCM8 minichromosome maintenance deficient 8 (S. cerevisiae) (MCM8), transcript variant 1, mRNA | 84515 | NM_032485 |
| C:0555 | RNA binding motif protein 13 (RBM13), mRNA | 84552 | NM_032509 |
| C:1586 | par-6 partitioning defective 6 homolog beta (C. elegans) (PARD6B), mRNA | 84612 | NM_032521 |
| C:1872 | resistin like beta (RETNLB), mRNA | 84666 | NM_032579 |
| B:9569 | protein phosphatase 1, regulatory subunit 9B, spinophilin (PPP1R9B), mRNA | 84687 | NM_032595 |
| B:3610 | hepatoma-derived growth factor-related protein 2 (HDGF2), transcript variant 2, mRNA | 84717 | NM_032631 |
| B:4127 | lamin B2 (LMNB2), mRNA | 84823 | NM_032737 |
| B:2733 | apoptosis-inducing factor (AIF)-like mitochondrion-associated inducer of death (AMID), mRNA | 84883 | NM_032797 |
| B:4273 | RAS-like, estrogen-regulated, growth inhibitor (RERG), mRNA | 85004 | NM_032918 |
| B:9560 | cyclin B3 (CCNB3), transcript variant 1, mRNA | 85417 | NM_033670 |
| C:0075 | leucine rich repeat and coiled-coil domain containing 1 (LRRCC1), mRNA | 85444 | NM_033402 |
| B:8110 | tripartite motif-containing 4 (TRIM4), transcript variant alpha, mRNA | 89765 | NM_033017 |
| B:6017 | hypothetical gene CG018, CG018 | 90634 | NM_052818 |
| C:0238 | NIMA (never in mitosis gene a)-related kinase 9 (NEK9), mRNA | 91754 | NM_033116 |
| B:3862 | Cdk5 and Abl enzyme substrate 1 (CABLES1), mRNA | 91768 | NM_138375 |
| B:3802 | chordin-like 1 (CHRDL1), mRNA | 91860 | NM_145234 |
| B:3730 | family with sequence similarity 58, member A (FAM58A), mRNA | 92002 | NM_152274 |
| B:6762 | secretoglobin, family 3A, member 1 (SCGB3A1), mRNA | 92304 | NM_052863 |
| B:4458 | membrane-associated ring finger (C3HC4) 9 MARCH9 | 92979 | NM_138396 |
| B:9351 | immunoglobulin superfamily, member 8 (IGSF8), | 93185 | NM_052868 |
| B:1687 | acid phosphatase, testicular (ACPT), transcript variant A, mRNA | 93650 | NM_033068 |
| B:3540 | RAS guanyl releasing protein 4 (RASGRP4), transcript variant 1, mRNA | 115727 | NM_170603 |
| C:4836 | topoisomerase (DNA) I, mitochondrial (TOP1 MT), nuclear gene encoding mitochondrial protein, mRNA | 116447 | NM_052963 |
| B:9435 | mediator of RNA polymerase II transcription, subunit 12 homolog (yeast)-like (MED12L), mRNA | 116931 | NM_053002 |
| C:3793 | amyotrophic lateral sclerosis 2 (juvenile) chromosome region, candidate 19 (ALS2CR19), transcript variant b, mRNA | 117583 | NM_152526 |
| C:3467 | KIAA1977 protein (KIAA1977), mRNA | 124404 | NM_133450 |
| C:3112 | ubiquitin specific protease 43 (USP43), mRNA | 124817 | XM_945578 |
| C:5265 | hypothetical protein BC009732 (LOC133308), mRNA | 133396 | NM_178833 |
| A:07401 | myosin light chain 1 slow a (MLC1SA), mRNA | 140466 | NM_002475 |
| C:1334 | CCCTC-binding factor (zinc finger protein)-like (CTCFL), mRNA | 140690 | NM_080618 |
| B:5293 | chromosome 20 open reading frame 181 C20orf181 | 140849 | U63828 |
| B:9316 | hypothetical protein MGC20470 (MGC20470), mRNA | 143686 | NM_145053 |
| B:9599 | septin 10 (SEPT10), transcript variant 1, mRNA | 151011 | NM_144710 |
| C:0962 | similar to hepatocellular carcinoma-associated antigen HCA557b (LOC151194), mRNA | 151195 | NM_145280 |
| C:1752 | connexin40 (CX40), mRNA | 219771 | NM_153368 |
| B:3031 | kinesin family member 6 (KIF6), mRNA | 221527 | NM_145027 |
| B:1737 | chromosome Y open reading frame 15A (CYorf15A), | 246176 | NM_001005852 |
| B:8632 | DNA directed RNA polymerase II polypeptide J-related gene (POLR2J2), transcript variant 3, mRNA | 246778 | NM_032959 |
| A:08544 | zinc finger, DHHC-type containing 24 (ZDHHC24), mRNA | 254394 | NM_207340 |
| C:3659 | growth arrest-specific 2 like 3 (GAS2L3), mRNA | 283431 | NM_174942 |
| B:5467 | laminin, alpha 1 (LAMA1), mRNA | 284217 | NM_005559 |
| C:2399 | hypothetical protein MGC26694 (MGC26694), mRNA | 284439 | NM_178526 |
| C:5315 | cation channel, sperm associated 3 (CATSPER3), mRNA | 347733 | NM_178019 |
| B:0631 | polymerase (DNA directed) nu (POLN), mRNA | 353497 | NM_181808 |

**Table B: Known cell proliferation-related genes.** All genes categorized as cell proliferation-related by gene ontology analysis and present on the Affymetrix HG-U133 platform.

### General Approaches to Prognostic Marker Detection

The following approaches are non-limiting methods that can be used to detect the proliferation markers, including GCPM family members: microarray approaches using oligonucleotide probes selective for a GCPM; real-time qPCR on tumour samples using GCPM specific primers and probes; real-time qPCR on lymph node, blood, serum, faecal, or urine samples using GCPM specific primers and probes; enzyme-linked immunological assays (ELISA); immunohistochemistry using anti-marker antibodies; and analysis of array or qPCR data using computers.

Other useful methods include northern blotting and *in situ* hybridization (Parker and Barnes, Methods in Molecular Biology 106: 247-283 (1999)); RNase protection assays (Hod, BioTechniques 13: 852-854 (1992)); reverse transcription polymerase chain reaction (RT-PCR; Weis et al., Trends in Genetics 8: 263-264 (1992)); serial analysis of gene expression (SAGE; Velculescu et al., Science 270: 484-487 (1995); and Velculescu et al., Cell 88: 243-51 (1997)), MassARRAY technology (Sequenom, San Diego, CA), and gene expression analysis by massively parallel signature sequencing (MPSS; Brenner et al., Nature Biotechnology 18: 630-634 (2000)). Alternatively, antibodies may be employed that can recognize specific complexes, including DNA duplexes, RNA duplexes, and DNA-RNA hybrid duplexes or DNA-protein duplexes.

Primary data can be collected and fold change analysis can be performed, for example, by comparison of marker expression levels in tumour tissue and non-tumour tissue; by comparison of marker expression levels to levels determined in recurring tumours and non-recurring tumours; by comparison of marker expression levels to levels determined in tumours with or without metastasis; by comparison of marker expression levels to levels determined in differently staged tumours; or by comparison of marker expression levels to levels determined in cells with different levels of proliferation. A negative or positive prognosis is determined based on this analysis. Further analysis of tumour marker expression includes matching those markers exhibiting increased or decreased expression with expression profiles of known gastrointestinal tumours to provide a prognosis.

A threshold for concluding that expression is increased is provided as, for example, at least a 1.5-fold or 2-fold increase, and in alternative embodiments, at least a 3-fold increase, 4-fold increase, or 5-fold increase. A threshold for concluding that expression is decreased is provided as, for example, at least a 1.5-fold or 2-fold decrease, and in alternative embodiments, at least a 3-fold decrease, 4-fold decrease, or 5-fold decrease. It can be appreciated that other thresholds for concluding that increased or decreased expression has occurred can be selected without departing from the scope of this invention.

It will also be appreciated that a threshold for concluding that expression is increased will be dependent on the particular marker and also the particular predictive model that is to be applied. The threshold is generally set to achieve the highest sensitivity and selectivity with the lowest error rate, although variations may be desirable for a particular clinical situation. The desired threshold is determined by analysing a population of sufficient size taking into account the statistical variability of any predictive model and is calculated from the size of the sample used to produce the predictive model. The same applies for the determination of a threshold for concluding that expression is decreased. It can be appreciated that other thresholds, or methods for establishing a threshold, for concluding that increased or decreased expression has occurred can be selected without departing from the scope of this invention.

It is also possible that a prediction model may produce as it's output a numerical value, for example a score, likelihood value or probability. In these instances, it is possible to apply thresholds to the results produced by prediction models, and in these cases similar principles apply as those used to set thresholds for expression values

Once the expression level of one or more proliferation markers in a tumour sample has been obtained the likelihood of the cancer recurring can then be determined. In accordance with the invention, a negative prognosis is associated with decreased expression of at least one proliferation marker, while a positive prognosis is associated with increased expression of at least one proliferation marker. In various aspects, an increase in expression is shown by at least 1, 2, 3, 4, 5, 10, 15, 20, 25, 30, 35, 40, 45, 50, or 75 of the markers disclosed herein. In other aspects, a decrease in expression is shown by at least 1, 2, 3, 4, 5, 10, 15, 20, 25, 30, 35, 40, 45, 50, or 75 of the markers disclosed herein

From the genes identified, proliferation signatures comprising one or more GCPMs can be used to determine the prognosis of a cancer, by comparing the expression level of the one or more genes to the disclosed proliferation signature. By comparing the expression of one or more of the GCPMs in a tumour sample with the disclosed proliferation signature, the likelihood of the cancer recurring can be determined. The comparison of expression levels of the prognostic signature to establish a prognosis can be done by applying a predictive model as described previously.

Determining the likelihood of the cancer recurring is of great value to the medical practitioner. A high likelihood of reoccurrence means that a longer or higher dose treatment should be given, and the patient should be more closely monitored for signs of recurrence of the cancer. An accurate prognosis is also of benefit to the patient. It allows the patient, along with their partners, family, and friends to also make decisions about treatment, as well as decisions about their future and lifestyle changes. Therefore, the invention also provides for a method establishing a treatment regime for a particular cancer based on the prognosis established by matching the expression of the markers in a tumour sample with the differential proliferation signature.

It will be appreciated that the marker selection, or construction of a proliferation signature, does not have to be restricted to the GCPMs disclosed in Table A, Table B, Table C or Table D, herein, but could involve the use of one or more GCPMs from the disclosed signature, or a new signature may be established using GCPMs selected from the disclosed marker lists. The requirement of any signature is that it predicts the likelihood of recurrence with enough accuracy to assist a medical practitioner to establish a treatment regime.

Surprisingly, it was discovered that many of the GCPM were associated with increased levels of cell proliferation, and were also associated with a positive prognosis. It has similarly been found that there is a close correlation between the decreased expression level of GCPMs and a negative prognosis, e.g., an increased likelihood of gastrointestinal cancer recurring. Therefore, the present invention also provides for the use of a marker associated with cell proliferation, e.g., a cell cycle component, as a GCPM.

As described herein, determination of the likelihood of a cancer recurring can be accomplished by measuring expression of one or more proliferation-specific markers. The methods provided herein also include assays of high sensitivity. In particular, qPCR is extremely sensitive, and can be used to detect markers in very low copy number (e.g., 1 - 100) in a sample. With such sensitivity, prognosis of gastrointestinal cancer is made reliable, accurate, and easily tested.

### Reverse Transcription PCR (RT-PCR)

Of the techniques listed above, the most sensitive and most flexible quantitative method is RT-PCR, which can be used to compare RNA levels in different sample populations, in normal and tumour tissues, with or without drug treatment, to characterize patterns of expression, to discriminate between closely related RNAs, and to analyze RNA structure.

For RT-PCR, the first step is the isolation of RNA from a target sample. The starting material is typically total RNA isolated from human tumours or tumour cell lines, and corresponding normal tissues or cell lines, respectively. RNA can be isolated from a variety of samples, such as tumour samples from breast, lung, colon (e.g., large bowel or small bowel), colorectal, gastric, esophageal, anal, rectal, prostate, brain, liver, kidney, pancreas, spleen, thymus, testis, ovary, uterus, etc., tissues, from primary tumours, or tumour cell lines, and from pooled samples from healthy donors. If the source of RNA is a tumour, RNA can be extracted, for example, from frozen or archived paraffin-embedded and fixed (e.g., formalin-fixed) tissue samples.

The first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukaemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan (g) PCR typically utilizes the 5' nuclease activity of Taq or Tth polymerase to hydrolyze a hybridization probe bound to its target amplicon, but any enzyme with equivalent 5' nuclease activity can be used.

Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan RT-PCR can be performed using commercially available equipment, such as, for example, ABI PRISM 7700tam Sequence Detection System (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700tam Sequence Detection System. The system consists of a thermocycler, laser, charge-coupled device (CCD), camera, and computer. The system amplifies samples in a 96-well format on a thermocycler. During amplification, laser-induced fluorescent signal is collected in real-time through fibre optics cables for all 96 wells, and detected at the CCD. The system includes software for running the instrument and for analyzing the data.

5' nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle.

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and-actin.

### Real-time quantitative PCR (qPCR)

A more recent variation of the RT-PCR technique is the real time quantitative PCR, which measures PCR product accumulation through a dual-labeled fluorigenic probe (i.e., TaqMan@ probe). Real time PCR is compatible both with quantitative competitive PCR and with quantitative comparative PCR. The former uses an internal competitor for each target sequence for normalization, while the latter uses a normalization gene contained within the sample, or a housekeeping gene for RT-PCR. For further details see, e.g., Held et al., Genome Research 6: 986-994 (1996).

Expression levels can be determined using fixed, paraffin-embedded tissues as the RNA source. According to one aspect of the present invention, PCR primers and probes are designed based upon intron sequences present in the gene to be amplified. In this embodiment, the first step in the primer/probe design is the delineation of intron sequences within the genes. This can be done by publicly available software, such as the DNA BLAT software developed by Kent, W. J., Genome Res. 12 (4): 656-64 (2002), or by the BLAST software including its variations. Subsequent steps follow well established methods of PCR primer and probe design.

In order to avoid non-specific signals, it is useful to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers in: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tₘ), and G/C content, specificity, complementary primer sequences, and 3' end sequence. In general, optimal PCR primers are generally 17-30 bases in length, and contain about 20-80%, such as, for example, about 50-60% G+C bases. Tₘs between 50 and 80°C, e.g., about 50 to 70°C are typically preferred. For further guidelines for PCR primer and probe design see, e.g., Dieffenbach, C. W. et al., General Concepts for PCR Primer Design in: PCR Primer, A Laboratory Manual, Cold Spring Harbor Laboratory Press, New York, 1995, pp. 133-155; Innis and Gelfand, Optimization of PCRs in: PCR Protocols, A Guide to Methods and Applications, CRC Press, London, 1994, pp. 5-11; and Plasterer, T. N. Primerselect: Primer and probe design. Methods Mol. Biol. 70: 520-527 (1997), the entire disclosures of which are hereby expressly incorporated by reference.

### Microarray analysis

Differential gene expression can also be identified, or confirmed using the microarray technique. Thus, the expression profile of GCPMs can be measured in either fresh or paraffin-embedded tumour tissue, using microarray technology. In this method, polynucleotide sequences of interest (including cDNAs and oligonucleotides) are plated, or arrayed, on a microchip substrate. The arrayed sequences (i.e., capture probes) are then hybridized with specific polynucleotides from cells or tissues of interest (i.e., targets). Just as in the RT-PCR method, the source of RNA typically is total RNA isolated from human tumours or tumour cell lines, and corresponding normal tissues or cell lines. Thus RNA can be isolated from a variety of primary tumours or tumour cell lines. If the source of RNA is a primary tumour, RNA can be extracted, for example, from frozen or archived paraffin-embedded and fixed (e.g., formalin-fixed) tissue samples, which are routinely prepared and preserved in everyday clinical practice.

In a specific embodiment of the microarray technique, PCR amplified inserts of cDNA clones are applied to a substrate. The substrate can include up to 1, 2, 3, 4, 5, 10, 15, 20, 25, 30, 35, 40, 45, 50, or 75 nucleotide sequences. In other aspects, the substrate can include at least 10,000 nucleotide sequences. The microarrayed sequences, immobilized on the microchip, are suitable for hybridization under stringent conditions. As other embodiments, the targets for the microarrays can be at least 50, 100, 200, 400, 500, 1000, or 2000 bases in length; or 50-100, 100-200, 100-500, 100-1000, 100-2000, or 500-5000 bases in length. As further embodiments, the capture probes for the microarrays can be at least 10, 15, 20, 25, 50, 75, 80, or 100 bases in length; or 10-15, 10-20, 10-25, 10-50, 10-75, 10-80, or 20-80 bases in length.

Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual colour fluorescence, separately labeled cDNA probes generated from two sources of RNA are hybridized pairwise to the array. The relative abundance of the transcripts from the two sources corresponding to each specified gene is thus determined simultaneously.

The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93 (2): 106-149 (1996)). Microarray analysis can be performed by commercially available equipment, following manufacturer's protocols, such as by using the Affymetrix GenChip technology, or Incyte's microarray technology. The development of microarray methods for large-scale analysis of gene expression makes it possible to search systematically for molecular markers of cancer classification and outcome prediction in a variety of tumour types.

### RNA isolation, purification, and amplification

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56: A67 (1987), and De Sandres et al., BioTechniques 18: 42044 (1995). In particular, RNA isolation can be performed using purification kit, buffer set, and protease from commercial manufacturers, such as Qiagen, according to the manufacturer's instructions. For example, total RNA from cells in culture can be isolated using Qiagen RNeasy mini-columns. Other commercially available RNA isolation kits include MasterPure Complete DNA and RNA Purification Kit (EPICENTRE (D, Madison, WI), and Paraffin Block RNA Isolation Kit (Ambion, Inc.). Total RNA from tissue samples can be isolated using RNA Stat-60 (Tel-Test). RNA prepared from tumour can be isolated, for example, by cesium chloride density gradient centrifugation.

The steps of a representative protocol for profiling gene expression using fixed, paraffin-embedded tissues as the RNA source, including mRNA isolation, purification, primer extension and amplification are given in various published journal articles (for example: T. E. Godfrey et al. J. Molec. Diagnostics 2: 84-91 (2000); K. Specht et al., Am. J. Pathol. 158: 419-29 (2001)). Briefly, a representative process starts with cutting about 10 µm thick sections of paraffin-embedded tumour tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR. Finally, the data are analyzed to identify the best treatment option(s) available to the patient on the basis of the characteristic gene expression pattern identified in the tumour sample examined.

### Immunohistochemistry and proteomics

Immunohistochemistry methods are also suitable for detecting the expression levels of the proliferation markers of the present invention. Thus, antibodies or antisera, preferably polyclonal antisera, and most preferably monoclonal antibodies specific for each marker, are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

Proteomics can be used to analyze the polypeptides present in a sample (e.g., tissue, organism, or cell culture) at a certain point of time. In particular, proteomic techniques can be used to asses the global changes of protein expression in a sample (also referred to as expression proteomics). Proteomic analysis typically includes: (1) separation of individual proteins in a sample by 2-D gel electrophoresis (2-D PAGE); (2) identification of the individual proteins recovered from the gel, e.g., my mass spectrometry or N-terminal sequencing, and (3) analysis of the data using bioinformatics. Proteomics methods are valuable supplements to other methods of gene expression profiling, and can be used, alone or in combination with other methods, to detect the products of the proliferation markers of the present invention.

### Selection of Differentially Expressed Genes.

An early approach to the selection of genes deemed significant involved simply looking at the "fold change" of a given gene between the two groups of interest. While this approach hones in on genes that seem to change the most spectacularly, consideration of basic statistics leads one to realize that if the variance (or noise level) is quite high (as is often seen in microarray experiments), then seemingly large fold-change can happen frequently by chance alone.

Microarray experiments, such as those described here, typically involve the simultaneous measurement of thousands of genes. If one is comparing the expression levels for a particular gene between two groups (for example recurrent and non-recurrent tumours), the typical tests for significance (such as the t-test) are not adequate. This is because, in an ensemble of thousands of experiments (in this context each gene constitutes an "experiment"), the probability of at least one experiment passing the usual criteria for significance by chance alone is essentially unity. In a test for significance, one typically calculates the probability that the "null hypothesis" is correct. In the case of comparing two groups, the null hypothesis is that there is no difference between the two groups. If a statistical test produces a probability for the null hypothesis below some threshold (usually 0.05 or 0.01), it is stated that we can reject the null hypothesis, and accept the hypothesis that the two groups are *significantly* different. Clearly, in such a test, a rejection of the null hypothesis by chance alone could be expected 1 in 20 times (or 1 in 100). The use of t-tests, or other similar statistical tests for significance, fail in the context of microarrays, producing far too many false positives (or type I errors)

In this type of situation, where one is testing multiple hypotheses at the same time, one applies typical multiple comparison procedures, such as the Bonferroni Method (43). However such tests are too conservative for most microarray experiments, resulting in too many false negative (type II) errors.

A more recent approach is to do away with attempting to apply a probability for a given test being significant, and establish a means for selecting a subset of experiments, such that the expected proportion of Type I errors (or false discovery rate; 47) is controlled for. It is this approach that has been used in this investigation, through various implementations, namely the methods provided with BRB Array Tools (48), and the limma (11,42) package of Bioconductor (that uses the R statistical environment; 10,39).

### General methodology for Data Mining: Generation of Prognostic Signatures

Data Mining is the term used to describe the extraction of "knowledge", in other words the "know-how", or predictive ability from (usually) large volumes of data (the dataset). This is the approach used in this study to generate prognostic signatures. In the case of this study the "know-how" is the ability to accurately predict prognosis from a given set of gene expression measurements, or "signature" (as described generally in this section and in more detail in the examples section).

The specific details used for the methods used in this study are described in Examples 17-20. However, application of any of the data mining methods (both those described in the Examples, and those described here) can follow this general protocol.

Data mining (49), and the related topic machine learning (40) is a complex, repetitive mathematical task that involves the use of one or more appropriate computer software packages (see below). The use of software is advantageous on the one hand, in that one does not need to be completely familiar with the intricacies of the theory behind each technique in order to successfully use data mining techniques, provided that one adheres to the correct methodology. The disadvantage is that the application of data mining can often be viewed as a "black box": one inserts the data and receives the answer. How this is achieved is often masked from the end-user (this is the case for many of the techniques described, and can often influence the statistical method chosen for data mining. For example, neural networks and support vector machines have a particularly complex implementation that makes it very difficult for the end user to extract out the "rules" used to produce the decision. On the other hand, k-nearest neighbours and linear discriminant analysis have a very transparent process for decision making that is not hidden from the user.

There are two types of approach used in data mining: supervised and unsupervised approaches. In the supervised approach, the information that is being linked to the data is known, such as categorical data (e.g. recurrent vs. non recurrent tumours). What is required is the ability to link the observed response (e.g. recurrence vs. non-recurrence) to the input variables. In the unsupervised approach, the classes within the dataset are not known in advance, and data mining methodology is employed to attempt to find the classes or structure within the dataset.

In the present example the supervised approach was used and is discussed in detail here, although it will be appreciated that any of the other techniques could be used.

The overall protocol involves the following steps:
- Data representation. This involves transformation of the data into a form that is most likely to work successfully with the chosen data mining technique. In where the data is numerical, such as in this study where the data being investigated represents relative levels of gene expression, this is fairly simple. If the data covers a large dynamic range (i.e. many orders of magnitude) often the log of the data is taken. If the data covers many measurements of separate samples on separate days by separate investigators, particular care has to be taken to ensure systematic error is minimised. The minimisation of systematic error (i.e. errors resulting from protocol differences, machine differences, operator differences and other quantifiable factors) is the process referred to here as "normalisation".
- Feature Selection. Typically the dataset contains many more data elements than would be practical to measure on a day-to-day basis, and additionally many elements that do not provide the information needed to produce a prediction model. The actual ability of a prediction model to describe a dataset is derived from some subset of the full dimensionality of the dataset. These dimensions the most important components (or features) of the dataset. Note in the context of microarray data, the dimensions of the dataset are the individual genes. Feature selection, in the context described here, involves finding those genes which are most "differentially expressed". In a more general sense, it involves those groups which pass some statistical test for significance, i.e. is the level of a particular variable consistently higher or lower in one or other of the groups being investigated. Sometimes the features are those variables (or dimensions) which exhibit the greatest variance.
   The application of feature selection is completely independent of the method used to create a prediction model, and involves a great deal of experimentation to achieve the desired results. Within this invention, the selection of significant genes, and those which correlated with the earlier successful model (the NZ classifier), entailed feature selection. In addition, methods of data reduction (such as principal component analysis) can be applied to the dataset.
- Training. Once the classes (e.g. recurrence/non-recurrence) and the features of the dataset have been established, and the data is represented in a form that is acceptable as input for data mining, the reduced dataset (as described by the features) is applied to the prediction model of choice. The input for this model is usually in the form a multi-dimensional numerical input,(known as a vector), with associated output information (a class label or a response). In the training process, selected data is input into the prediction model, either sequentially (in techniques such as neural networks) or as a whole (in techniques that apply some form of regression, such as linear models, linear discriminant analysis, support vector machines). In some instances (e.g. k-nearest neighbours) the dataset (or subset of the dataset obtained after feature selection) is itself the model. As discussed, effective models can be established with minimal understanding of the detailed mathematics, through the use of various software packages where the parameters of the model have been pre-determined by expert analysts as most likely to lead to successful results.
- Validation. This is a key component of the data-mining protocol, and the incorrect application of this frequently leads to errors. Portions of the dataset are to be set aside, apart from feature selection and training, to test the success of the prediction model. Furthermore, if the results of validation are used to effect feature selection and training of the model, then one obtains a further validation set to test the model before it is applied to real-life situations. If this process is not strictly adhered to the model is likely to fail in real-world situations. The methods of validation are described in more detail below.
- Application. Once the model has been constructed, and validated, it must be packaged in some way as it is accessible to end users. This often involves implementation of some form a spreadsheet application, into which the model has been imbedded, scripting of a statistical software package, or refactoring of the model into a hard-coded application by information technology staff.

Examples of software packages that are frequently used are:
- Spreadsheet plugins, obtained from multiple vendors.
- The R statistical environment.
- The commercial packages MatLab, S-plus, SAS, SPSS, STATA.
- Free open-source software such as Octave (a MatLab clone)
- many and varied C++ libraries, which can be used to implement prediction models in a commercial, closed-source setting.

### Examples of Data Mining Methods.

The methods can be by first performing the step of data mining process (above), and then applying the appropriate known software packages. Further description of the process of data mining is described in detail in many extremely well-written texts.(49)
- Linear models (49, 50): The data is treated as the input of a linear regression model, of which the class labels or responses variables are the output. Class labels, or other categorical data, must be transformed into numerical values (usually integer). In generalised linear models, the class labels or response variables are not themselves linearly related to the input data, but are transformed through the use of a "link function". Logistic regression is the most common form of generalized linear model.
- Linear Discriminant analysis (49, 51, 52). Provided the data is linearly separable (i.e. the groups or classes of data can be separated by a hyperplane, which is an n-dimensional extension of a threshold), this technique can be applied. A combination of variables is used to separate the classes, such that the between group variance is maximised, and the within-group variance is minimised. The byproduct of this is the formation of a classification rule. Application of this rule to samples of unknown class allows predictions or classification of class membership to be made for that sample. There are variations of linear discriminant analysis such as nearest shrunken centroids which are commonly used for microarray analysis.
- Support vector machines (53): A collection of variables is used in conjunction with a collection of weights to determine a model that maximizes the separation between classes in terms of those weighted variables. Application of this model to a sample then produces a classification or prediction of class membership for that sample.
- Neural networks (52): The data is treated as input into a network of nodes, which superficially resemble biological neurons, which apply the input from all the nodes to which they are connected, and transform the input into an output. Commonly, neural networks use the "multiply and sum" algorithm, to transform the inputs from multiple connected input nodes into a single output. A node may not necessarily produce an output unless the inputs to that node exceed a certain threshold. Each node has as its input the output from several other nodes, with the final output node usually being linked to a categorical variable. The number of nodes, and the topology of the nodes can be varied in almost infinite ways, providing for the ability to classify extremely noisy data that may not be possible to categorize in other ways. The most common implementation of neural networks is the multi-layer perceptron.
- Classification and regression trees (54): In these. variables are used to define a hierarchy of rules that can be followed in a stepwise manner to determine the class of a sample. The typical process creates a set of rules which lead to a specific class output, or a specific statement of the inability to discriminate. A example classification tree is an implementation of an algorithm such as:
   if gene A> x and gene Y > x and gene Z = z
   then
   class A
   else if geneA = q
   then
   class B
- Nearest neighbour methods (51, 52). Predictions or classifications are made by comparing a sample (of unknown class) to those around it (or known class), with closeness defined by a distance function. It is possible to define many different distance functions. Commonly used distance functions are the Euclidean distance (an extension of the Pythagorean distance, as in triangulation, to n-dimensions), various forms of correlation (including Pearson Correlation co-efficient). There are also transformation functions that convert data points that would not normally be interconnected by a meaningful distance metric into euclidean space, so that Euclidean distance can then be applied (e.g. Mahalanobis distance). Although the distance metric can be quite complex, the basic premise of k-nearest neighbours is quite simple, essentially being a restatement of "find the k-data vectors that are most similar to the unknown input, find out which class they correspond to, and vote as to which class the unknown input is".
- Other methods:
   - Bayesian networks. A directed acyclic graph is used to represent a collection of variables in conjunction with their joint probability distribution, which is then used to determine the probability of class membership for a sample.
   - Independent components analysis, in which independent signals (e.g., class membership) re isolated (into components) from a collection of variables. These components can then be used to produce a classification or prediction of class membership for a sample.
      Ensemble learning methods in which a collection of prediction methods are combined to produce a joint classification or prediction of class membership for a sample

There are many variations of these methodologies that can be explored (49), and many new methodologies are constantly being defined and developed. It will be appreciated that any one of these methodologies can be applied in order to obtain an acceptable result. Particular care must be taken to avoid overfitting, by ensuring that all results are tested via a comprehensive validation scheme.

### Validation

Application of any of the prediction methods described involves both training and cross-validation (43, 55) before the method can be applied to new datasets (such as data from a clinical trial). Training involves taking a subset of the dataset of interest (in this case gene expression measurements from colorectal tumours), such that it is stratified across the classes that are being tested for (in this case recurrent and non-recurrent tumours). This training set is used to generate a prediction model (defined above), which is tested on the remainder of the data (the testing set).

It is possible to alter the parameters of the prediction model so as to obtain better performance in the testing set, however, this can lead to the situation known as overfitting, where the prediction model works on the training dataset but not on any external dataset. In order to circumvent this, the process of validation is followed. There are two major types of validation typically applied, the first (hold-out validation) involves partitioning the dataset into three groups: testing, training, and validation. The validation set has no input into the training process whatsoever, so that any adjustment of parameters or other refinements must take place during application to the testing set (but not the validation set). The second major type is cross-validation, which can be applied in several different ways, described below.

There are two main sub-types of cross-validation: K-fold cross-validation, and leave-one-out cross-validation

K-fold cross-validation: .The dataset is divided into **K** subsamples, each subsample containing approximately the same proportions of the class groups as the original.

In each round of validation, one of the **K** subsamples is set aside, and training is accomplished using the remainder of the dataset. The effectiveness of the training for that round is guaged by how correctly the classification of the left-out group is. This procedure is repeated **K-** times, and the overall effectiveness ascertained by comparison of the predicted class with the known class.

Leave-one-out cross-validation: A commonly used variation of **K**-fold cross validation, in which **K**=*n*, where *n* is the number of samples.

Combinations of CCPMS, such as those described above in Tables 1 and 2, can be used to construct predictive models for prognosis.

### Prognostic Signatures

Prognostic signatures, comprising one or more of these markers, can be used to determine the outcome of a patient, through application of one or more predictive models derived from the signature. In particular, a clinician or researcher can determine the differential expression (e.g., increased or decreased expression) of the one or more markers in the signature, apply a predictive model, and thereby predict the negative prognosis, e.g., likelihood of disease relapse, of a patient, or alternatively the likelihood of a positive prognosis (continued remission).

In still further aspects, the invention includes a method of determining a treatment regime for a cancer comprising: (a) providing a sample of the cancer; (b) detecting the expression level of a GgCPM family member in said sample; (c) determining the prognosis of the cancer based on the expression level of a CCPM family member; and (d) determining the treatment regime according to the prognosis.

In still further aspects, the invention includes a device for detecting a GCPM, comprising: a substrate having a GCPM capture reagent thereon; and a detector associated with said substrate, said detector capable of detecting a GCPM associated with said capture reagent. Additional aspects include kits for detecting cancer, comprising: a substrate; a GCPM capture reagent; and instructions for use. Yet further aspects of the invention include method for detecting aGCPM using qPCR, comprising: a forward primer specific for said CCPM; a reverse primer specific for said GCPM; PCR reagents; a reaction vial; and instructions for use.

Additional aspects of this invention comprise a kit for detecting the presence of a GCPM polypeptide or peptide, comprising: a substrate having a capture agent for said GCPM polypeptide or peptide; an antibody specific for said GCPM polypeptide or peptide; a reagent capable of labeling bound antibody for said GCPM polypeptide or peptide; and instructions for use.

In yet further aspects, this invention includes a method for determining the prognosis of colorectal cancer, comprising the steps of: providing a tumour sample from a patient suspected of having colorectal cancer; measuring the presence of a GCPM polypeptide using an ELISA method. In specific aspects of this invention the GCPM of the invention is selected from the markers set forth in Table A, Table B, Table C or Table D. In still further aspects, the GCPM is included in a prognostic signature

While exemplified herein for gastrointestinal cancer, e.g., gastric and colorectal cancer, the GCPMs of the invention also find use for the prognosis of other cancers, e.g., breast cancers, prostate cancers, ovarian cancers, lung cancers (such as adenocarcinoma and, particularly, small cell lung cancer), lymphomas, gliomas, blastomas (e.g., medulloblastomas), and mesothelioma, where decreased or low expression is associated with a positive prognosis, while increased or high expression is associated with a negative prognosis.

### EXAMPLES

The examples described herein are for purposes of illustrating embodiments of the invention. Other embodiments, methods, and types of analyses are within the scope of persons of ordinary skill in the molecular diagnostic arts and need not be described in detail hereon. Other embodiments within the scope of the art are considered to be part of this invention.

### EXAMPLE 1: Cell cultures

The experimental scheme is shown in FIG. 1. Ten colorectal cell lines were cultured and harvested at semi- and full-confluence. Gene expression profiles of the two growth stages were analyzed on 30,000 oligonucleotide arrays and a gene proliferation signature (GPS; Table C) was identified by gene ontology analysis of differentially expressed genes. Unsupervised clustering was then used to independently dichotomize two cohorts of clinical colorectal samples (Cohort A: 73 stage I-IV on oligo arrays, Cohort B: 55 stage 11 on Affymetrix chips) based on the similarities of the GPS expression. Ki-67 immunostaining was also performed on tissue sections from Cohort A tumours. Following this, the correlation between proliferation activity and clinico-pathologic parameters was investigated.

Ten colorectal cancer cell lines derived from different disease stages were included in this study: DLD-1, HCT-8, HCT-116, HT-29, LoVo, Ls174T, SK-CO-1, SW48, SW480, and SW620 (ATCC, Manassas, VA). Cells were cultivated in a 5% CO₂ humidified atmosphere at 37°C in alpha minimum essential medium supplemented with 10% fetal bovine serum, 100 IU/ml penicillin and 100 µg/ml streptomycin (GIBCO-Invitrogen, CA). Two cell cultures were established for each cell line. The first culture was harvested upon reaching semi-confluence (50-60%). When cells in the second culture reached full-confluence (determined both microscopically and macroscopically), media was replaced, and cells were harvested twenty-four hours later to prepare RNA from the growth-inhibited cells. Array experiments were carried out on RNA extracted from each cell culture. In addition, a second culturing experiment was done following the same procedure and extracted RNA was used for dye-reversed hybridizations.

### EXAMPLE 2: Patients

Two cohorts of patients were analysed. Cohort A included 73 New Zealand colorectal cancer patients who underwent surgery at Dunedin and Auckland hospitals between 1995 and 2000. These patients were part of a prospective cohort study and included all disease stages. Tumour samples were collected fresh from the operation theatre, snap frozen in liquid nitrogen and stored at -80°C. Specimens were reviewed by a single pathologist (H-S Y) and tumours were staged according to the TNM system (34). Of the 73 patients, 32 developed disease recurrence and 41 remained recurrence-free after a minimum of five years follow up. The median overall survival was 29.5 and 66 months for recurrent and recurrent-free patients, respectively. Twenty patients received 5-FU-based post-operative adjuvant chemotherapy and 12 patients received radiotherapy (7 pre- and 5 post-operative).

Cohort B included a group of 55 German colorectal patients who underwent surgery at the Technical University of Munich between 1995 and 2001 and had fresh frozen samples stored in a tissue bank. All 55 had stage II disease, 26 developed disease recurrence (median survival 47 months) and 29 remained recurrence-free (median survival 82 months). None of patients received chemotherapy or radiotherapy. Clinico-pathologic variables of both cohorts are summarised as part of Table 2.

**Table 2: Clinico-pathologic parameters and their association with the GPS expression and Ki-67 PI**

| | | Number of patients | | GPS | | Ki-67 PI* | |
|---|---|---|---|---|---|---|---|
| Parameters | | cohort A | cohort B | cohortA (p-value)^{§} | cohort B (p-value)^{§} | Mean ± SD | p-value^{§} |
| Age^{¶} | < Mean | 34 | 31 | 1 | 0.79 | 74.4±17.9 | 0.6 |
| | >Mean | 39 | 24 | | | 77.9±17.3 | |
| Sex | Male | 35 | 33 | 0.16 | 1 | 77.3±15.3 | 1 |
| | Female | 38 | 22 | | | 75.3±19.5 | |
| Sife^{£} | Right side | 30 | 12 | 1 | 0.2 | 80.4±13.3 | 0.2 |
| | Left side | 43 | 43 | | | 73.1±19.7 | |
| Grade | Well | 9 | 0 | 0.22 | 0.2 | 75.6±18.1 | |
| | Moderate | 50 | 33 | | | 73.9±18.9 | 0.98 |
| | Poor | 14 | 22 | | | 84.3±9.3 | |
| Dukes stage | A | 10 | 0 | **0.006** | NA | 78.8±17.3 | 0.73 |
| | B | 27 | 55 | | | 75.7±18.4 | |
| | C | 28 | 0 | | | 76±16.1 | |
| | D | 8 | 0 | | | 75.9±22 | |
| T stage | T1 | 5 | 0 | 0.16 | 0.62 | 71.3±22.4 | 0.16 |
| | T2 | 11 | 11 | | | 85.4±7.4 | |
| | T3 | 50 | 41 | | | 76±17 | |
| | T4 | 7 | 3 | | | 66.2±26.3 | |
| N stage | N0 | 38 | 55 | **0.03** | NA | 76.5±17.9 | 1 |
| | N1+N2 | 35 | 0 | | | 76±17.4 | |
| Vascular invasion | Yes | 5 | 1 | 0.67 | NA | 54.4±31.5 | 0.32 |
| | No | 68 | 54 | | | 78±15 | |
| Lymphatic invasion | Yes | 32 | 5 | **0.06** | 0.35 | 76.5±18.3 | 0.6 |
| | No | 41 | 50 | | | 75.1±17.3 | |
| Lymphocyte infiltration | Mild | 35 | 15 | 0.89 | 1 | 75±18.6 | 0.85 |
| | Moderate | 27 | 25 | | | 79.4±16.5 | |
| | Prominent | 11 | 15 | | | 73.5±18.3 | |
| Margin | Infiltrative | 45 | | 0.47 | NA | 75.8±18.9 | 1 |
| | Expansive | 28 | NA | | | 77.1±15.7 | |
| Recurrence | Yes | 32 | 26 | **0.03** | **<0.001** | 75.6±19 | 0.79 |
| | No | 41 | 29 | | | 76.8±16.2 | |
| Total | | 73 | 55 | | | 76.3±17.5 | |

| | | | | | | | |
|---|---|---|---|---|---|---|---|
| § A Fisher's Exact Test or Kruskal-Wallis Test were used for testing association between clinico-pathologic parameters and GPS expression or Ki-67 PI, as appropriate. * Ki-67 immunostaining was performed on tumor sections from cohort A patients. £Proximal and distal to splenic flexure, respectively ¶ Average age 68 and 63 years for cohort A and B patients, respectively NA: not applicable | | | | | | | |

### EXAMPLE 3: Array preparation and gene expression analysis

Cohort A tumours and cell lines: Tissue samples and cell lines were homogenised and RNA was extracted using Tri-Reagent (Progenz, Auckland, NZ). The RNA was then purified using RNeasy mini column (Qiagen, Victoria, Australia) according to the manufacture's protocol. Ten micrograms of total RNA extracted from each culture or tumour sample was oligo-dT primed and cDNA synthesis was carried out in the presence of aa-dUTP and Superscript II RNase H-Reverse Transcriptase (Invitrogen). Cy dyes were incorporated into cDNA using the indirect amino-allyl cDNA labelling method. cDNA derived from a pool of 12 different cell lines was used as the reference for all hybridizations. The Cy5-dUTP-tagged cDNA from an individual colorectal cell line or tissue sample was combined with Cy3-dUTP-tagged cDNA from reference sample. The mixture was then purified using a QiaQuick PCR purification Kit (Qiagen, Victoria, Australia) and co-hybridized to a microarray spotted with the MWG 30K Oligo Set (MWG Biotech, NC). cDNA samples from the second culturing experiment were additionally analysed on microarrays using reverse labelling.

Arrays were scanned with a GenePix 4000B Microarray Scanner and data were analysed using GenePix Pro 4.1 Microarray Acquisition and Analysis Software (Axon, CA). The foreground intensities from each channel were log₂ transformed and normalised using the SNOMAD software (35) Normalised values were collated and filtered using BRB-Array Tools Version 3.2 (developed by Dr. Richard Simon and Amy Peng Lam, Biometric Research Branch, National Cancer Institute). Low intensity genes, and genes for which over 20% of measurements across tissue samples or cell lines were missing, were excluded from further analysis.

Cohort B tumours: Total RNA was extracted from each tumour using RNeasy Mini Kit and purified on RNeasy Columns (Qiagen, Hilden, Germany). Ten micrograms of total RNA was used to synthesize double-stranded cDNA with SuperScript II reverse transcriptase (GIBCO-Invitrogen, NY) and an oligo-dT-T7 primer (Eurogentec, Koeln, Germany). Biotinylated cRNA was synthesized from the double-stranded cDNA using the Promega RiboMax T7-kit (Promega, Madison, WI) and Biotin-NTP labelling mix (Loxo, Dossenheim, Germany). Then, the biotinylated cRNA was purified and fragmented. The fragmented cRNA was hybridized to Affymetrix HGU133A GeneChips (Affymetrix, Santa Clara, CA) and stained with streptavidin-phycoerythrin. The arrays were then scanned with a HP-argon-ion laser confocal microscope and the digitized image data were processed using the Affymetrix® Microarray Suite 5.0 Software. All Affymetrix U133A GeneChips passed quality control to eliminate scans with abnormal characteristics. Background correction and normalization were performed in the R computing environment using the robust multi-array average function implemented in the Bioconductor package affy.

### EXAMPLE 4: Quantitative real-time PCR (QPCR)

The expression of eleven genes (MAD2L1, POLE2, CDC2, MCM6, MCM7, RANSEH2A, TOPK, KPNA2, G22P1, PCNA, and GMNN) was validated using the cDNA from the cell cultures. Total RNA (2 µg) was reverse transcribed using Superscript II RNase H-Reverse Transcriptase kit (Invitrogen) and oligo dT primer (Invitrogen). QPCR was performed on an ABI Prism 7900HT Sequence Detection System (Applied Biosystems) using Taqman Gene Expression Assays (Applied Biosystems). Relative fold changes were calculated using the 2^{-ΔΔCT} method36 with Topoisomerase 3A as the internal control. Reference RNA was used as the calibrator to enable comparison between different experiments.

### EXAMPLE 5: Immunohistochemical analysis

Immunohistochemical expression of Ki-67 antigen (MIB-1; DakoCytomation, Denmark) was investigated on 4 µm sections of 73 paraffin-embedded primary colorectal tumours from Cohort A. Endogenous peroxidase activity was blocked with 0.3% hydrogen peroxidase in methanol and antigens were retrieved in boiling citrate buffer (pH 6). Nonspecific binding sites were blocked with 5% normal goat serum containing 1% BSA. Primary antibody (dilution 1:50) was detected using the EnVision system (Dako EnVision, CA) and the DAB substrate kit (Vector laboratories, CA). Five high-power fields were selected using a 10 x 10 microscope grid and cell counts were performed manually in a blind fashion without knowledge of the clinico-pathologic data. The Ki-67 proliferation index (PI) was presented as the percentage of positively stained nuclei for each tumour.

### EXAMPLE 6: Statistical analysis

Statistical analyses were performed using SPSS® version 14.0.0 (SPSS Inc., Chicago, IL). Ki-67 proliferation indices were presented as mean ± SD. A Fisher's Exact Test or Kruskal-Wallis Test was used to evaluate the differences between categorized groups based on the expression of the GPS or the Ki-67 PI versus the clinico-pathologic parameters. A P value ≤ 0.05 was considered significant. Overall survival (OS) and recurrence-free survival (RFS) were plotted using the method of Kaplan and Meier (37). A log-rank test was used to test for differences in survival time between the categorized groups. Relative risk and associated confidence intervals were also estimated for each variable using the Cox univariate model, and a multivariate Cox proportional hazard model was developed using forward stepwise regression with predictive variables that were significant in the univariate analysis. K-means clustering method was used to classify clinical samples based on the expression level of GPS.

### EXAMPLE 7: Identification of a gene proliferation signature (GPS) using a colorectal cell line model

An overview of the approach used to derive and apply a gene proliferation signature (GPS) is summarised in FIG. 1. The GPS, including 38 mitotic cell cycle genes (Table C), was relatively over-expressed in cycling cells in semi-confluent cultures. Low proliferation, defined by low GPS expression, was associated with unfavourable clinico-pathologic variables, shorter overall and recurrence-free survival (p<0.05). No association was found between Ki-67 proliferation index and clinico-pathologic variables or clinical outcome.

**Table C: GCPMs for cell proliferation signature**

| Unique ID | Average Fold change EP/SP | Gene Symbol | Gene Name | GenBank Acc. No. | Gene Aliases |
|---|---|---|---|---|---|
| A:05382 | 1.91 | CDC2 | cell division cycle 2, G1 to S and G2 to M | NM_001786, NM_033379 | CDK1; MGC111195; DKFZp686L2 0222 |
| B:8147 | 1.89 | MCM6 | MCM6 minichromosome maintenance deficient 6 (MIS5 homolog, S. pombe) (S. cerevisiae) | NM_005915 | Mis5; P105MCM; MCG40308 |
| A:00231 | 1.75 | RPA3 | replication protein A3, 14kDa | NM_002947 | REPA3 |
| B:7620 | 1.69 | MCM7 | MCM7 minichromosome maintenance deficient 7 (S. cerevisiae) | NM_005916, NM_182776 | MCM2; CDC47; P85MCM; P1CDC47; PNAS-146; CDABP0042; P1.1-MCM3 |
| A:03715 | 1.68 | PCNA | proliferating cell nuclear antigen | NM_002592, NM_182649 | MGC8367 |
| B:9714 | 1.59 | XRCC6 | X-ray repair complementing defective repair in Chinese hamster cells 6 (Ku autoantigen, 70kDa) | NM_001469 | ML8; KU70; TLAA; CTC75; CTCBF; G22P1 |
| B:4036 | 1.56 | KPNA2 | karyopherin alpha 2 (RAG cohort 1, importin alpha 1) | NM_002266 | QIP2; RCH1; IPOA1; SRP1alpha |
| A:05280 | 1.56 | ANLN | anillin, actin binding protein | NM_018685 | scra; Scraps; ANILLIN; DKFZp779A0 55 |
| A:04760 | 1.52 | APG7L | ATG7 autophagy related 7 homolog (S. cerevisiae) | NM_006395 | GSA7; APG7L; DKFZp434N0 735; ATG7 |
| A:03912 | 1.52 | PBK | PDZ binding kinase | M_018492 | SPK; TOPK; Nori-3; FLJ14385 |
| A:03435 | 1.51 | GMNN | geminin, DNA replication inhibitor | NM_015895 | Gem; RP3-369A17.3 |
| A:09802 | 1.51 | RRM1 | ribonucleotide reductase M1 polypeptide | NM_001033 | R1; RR1; RIR1 |
| A:09331 | 1.49 | CDC45L | CDC45 cell division cycle 45-like (S. cerevisiae) | NM_003504 | CDC45; CDC45L2; PORC-PI-1 |
| A:06387 | 1.46 | MAD2L1 | MAD2 mitotic arrest deficient-like 1 (yeast) | NM_002358 | MAD2; HSMAD2 |
| A:09169 | 1.45 | RAN | RAN, member RAS oncogene family | NM_006325 | TC4; Gsp1; ARA24 |
| A:07296 | 1.43 | DUT | dUTP pyrophosphatase | NM_001025248, NM_001025249, NM_001948 | dUTPase; FLJ20622 |
| B:3501 | 1.42 | RRM2 | ribonucleotide reductase M2 polypeptide | NM_001034 | R2; RR2M |
| A:09842 | 1.41 | CDK7 | cyclin-dependent kinase 7 (MO15 homolog, Xenopus laevis, cdk-activating kinase) | NM_001799 | CAK1; STK1; CDKN7; p39MO15 |
| A:09724 | 1.40 | MLH3 | mutL homolog 3 (E. coli) | NM_001040108, NM_014381 | HNPCC7; MGC138372 |
| A:05648 | 1.39 | SMC4 | structural maintenance of chromosomes 4 | NM_001002799, NM_001002800, NM_005496 | CAPC; SMC4L1; hCAP-C |
| A:09436 | 1.39 | SMC3 | structural maintenance of chromosomes 3 | NM_005445 | BAM; BMH; HCAP; CSPG6; SMC3L1 |
| A:02929 | 1.39 | POLD2 | polymerase (DNA directed), delta 2, regulatory subunit 50kDa | NM_006230 | None |
| A:04680 | 1.38 | POLE2 | polymerase (DNA directed), epsilon 2 (p59 subunit) | NM_002692 | DPE2 |
| B:8449 | 1.38 | BCCIP | BRCA2 and CDKN1A interacting protein | NM_016567, NM_078468, NM_078469 | TOK-1 |
| B:1035 | 1.37 | GINS2 | GINS complex subunit 2 (Psf2 homolog) | NM_016095 | PSF2; Pfs2; HSPC037 |
| B:7247 | 1.37 | TREX1 | three prime repair exonuclease 1 | NM_016381, NM_032166, NM_033627, NM_033628, NM_033629, NM_130384 | AGS1; DRN3; ATRIP; FLJ12343; DKFZp434J0 310 |
| A:09747 | 1.35 | BUB3 | BUB3 budding uninhibited by benzimidazoles 3 homolog (yeast) | NM_001007793, NM_004725 | BUB3L; hBUB3 |
| B:9065 | 1.32 | FEN1 | flap structure-specific endonuclease 1 | NM_004111 | MF1; RAD2; FEN-1 |
| B:2392 | 1.32 | DBF4B | DBF4 homolog B (S. cerevisiae) | NM_025104, NM_145663 | DRF1; ASKL1; FLJ13087; MGC15009 |
| A:09401 | 1.31 | PREI3 | preimplantation protein 3 | NM_015387, NM_199482 | 2C4D; MOB1; MOB3; CGI-95; MGC12264 |
| C:0921 | 1.30 | CCNE1 | cyclin E1 | NM_001238, NM_057182 | CCNE |
| A:10597 | 1.30 | RPA1 | replication protein A1, 70kDa | NM_002945 | HSSB; RF-A; RP-A; REPA1; RPA70 |
| A:02209 | 1.29 | POLE3 | polymerase (DNA directed), epsilon 3 (p17 subunit) | NM_017443 | p17; YBL1; CHRAC17; CHARAC17 |
| A:09921 | 1.26 | RFC4 | replication factor C (activator 1) 4, 37kDa | NM_002916, NM_181573 | A1; RFC37; MGC27291 |
| A:08668 | 1.26 | MCM3 | MCM3 minichromosome maintenance deficient 3 (S. cerevisiae) | NM_002388 | HCC5; P1.h; RLFB; MGC1157; P1-MCM3 |
| B:7793 | 1.25 | CHEK1 | CHK1 checkpoint homolog (S. pombe) | NM_001274 | CHK1 |
| A:09020 | 1.22 | CCND1 | cyclin D1 | NM_053056 | BCL1; U21B31; D11S287E |
| A:03486 | 1.22 | CDC37 | CDC37 cell division cycle 37 homolog (S. cerevisiae) | NM_007065 | P50CDC37 |

The GPS was identified as a subset of genes whose expression correlates with CRC cell proliferation rate. Statistical Analysis of Microarray (SAM; Reference 38) was used to identify genes differentially expressed (DE) between exponentially growing (semi-confluent) and non-cycling (fully-confluent) CRC cell lines (FIG. 1, stage 1). To adjust for gene specific dye bias and other sources of variation, each culture set was analysed independently. Analyses were limited to 502 DE genes for which a significant expression difference was observed between two growth stages in both sets of cultures (false discovery rate < 1 %). Gene Ontology (GO) analysis was carried out using EASE39 to identify the biological process categories that were significantly reflected in the DE genes. Cell-proliferation related categories were over-represented mainly due to genes upregulated in exponentially growing cells. The mitotic cell cycle category (GO:0000278) was defined as the GPS because (i) this biological process was the most over-represented GO term (EASE score=5.5211); and (ii) all 38 mitotic cell cycle genes (Table C) were expressed at higher levels in rapidly growing compared to growth-inhibited cells. The expression of eleven genes from the GPS was assessed by QPCR and correlated with corresponding values obtained from the array data. Therefore, QPCR confirmed that elevated expression of the proliferation signature genes correlates with the increased proliferation in CRC cell lines (FIG. 5).

### EXAMPLE 8: Classification of CRC samples according to the expression level of gene proliferation signature

In order to examine the relative proliferation state of CRC tumours and the utility of the GPS for clinical application, CRC tumours from two cohorts were stratified into two clusters based on the expression of GPS (FIG. 1, stage 2). Expression values of the 38 genes defining the GPS were first obtained from the microarray-generated expression profiles of tumours. Tumours from each cohort were then separately classified into two clusters (K=2) based on their GPS expression level similarities using K-means unsupervised clustering. Analysis of DE genes between two defined clusters using all filtered genes revealed that the GPS was contained within the list of genes upregulated in cluster 1 (FIG. 2A, upper panel) relative to cluster 2 (lower panel) in both cohorts. Thus, the tumours in cluster 1 are characterised by high GPS expression, while the tumours in cluster 2 are characterised by low GPS expression.

### EXAMPLE 9: Low gene proliferation signature is associated with unfavourable clinico-pathologic variables

Table 2 summarises the association between GPS expression levels and clinico-pathologic variables. An association was observed between low proliferation activity, defined by low GPS expression, and an increased risk of recurrence in both cohorts (P=0.03 and <0.001 for Cohort A and B, respectively). In Cohort A, low GPS expression was also associated with a higher disease stage and lymph node metastasis (P=0.006 and 0.03 respectively). In addition, tumours with lymphatic invasion from Cohort A tended to be less proliferative than tumours without lymphatic invasion, albeit without reaching statistical significance (P=0.06). No association was found between the GPS expression level and tumour site, age, sex, degree of differentiation, T-stage, vascular invasion, degree of lymphocyte infiltration and tumour margin.

### EXAMPLE 10: Gene proliferation signature predicts clinical outcome

To examine the performance of the GPS in predicting patient outcome, Kaplan-Meier survival analysis was used to compare RFS and OS between low and high GPS tumours (FIG. 3). All patients were censored at 60 months post-operation. In colorectal cancer Cohort A, OS and RFS were shorter in patients with low GPS expression (Log rank test P=0.04 and 0.01, respectively). In colorectal cancer Cohort B, low GPS expression was also associated with decreased OS (P=0.0004) and RFS (P=0.0002). When the parameters predicting OS and RFS in univariate analysis were investigated in a multivariate model, disease stage was the only independent predictor of 5-year OS, while disease stage and T-stage were independent predictors of RFS in Cohort A. In Cohort B, low GPS expression and lymphatic invasion showed an independent contribution to both OS and RFS. If survival analysis was limited to Cohort B patients without lymphatic invasion, low GPS was still associated with shorter OS and RFS, confirming the independence of the GPS as a predictor. Analyses of single and multiple-variable associations with survival are summarized in Table 3.

Low GPS expression was also associated with decreased 5-year overall survival in patients with gastric cancer (p=0.008). A Kaplan-Meier survival plot comparing the overall survival of low and high GPS gastric tumours is shown in Fig. 4.

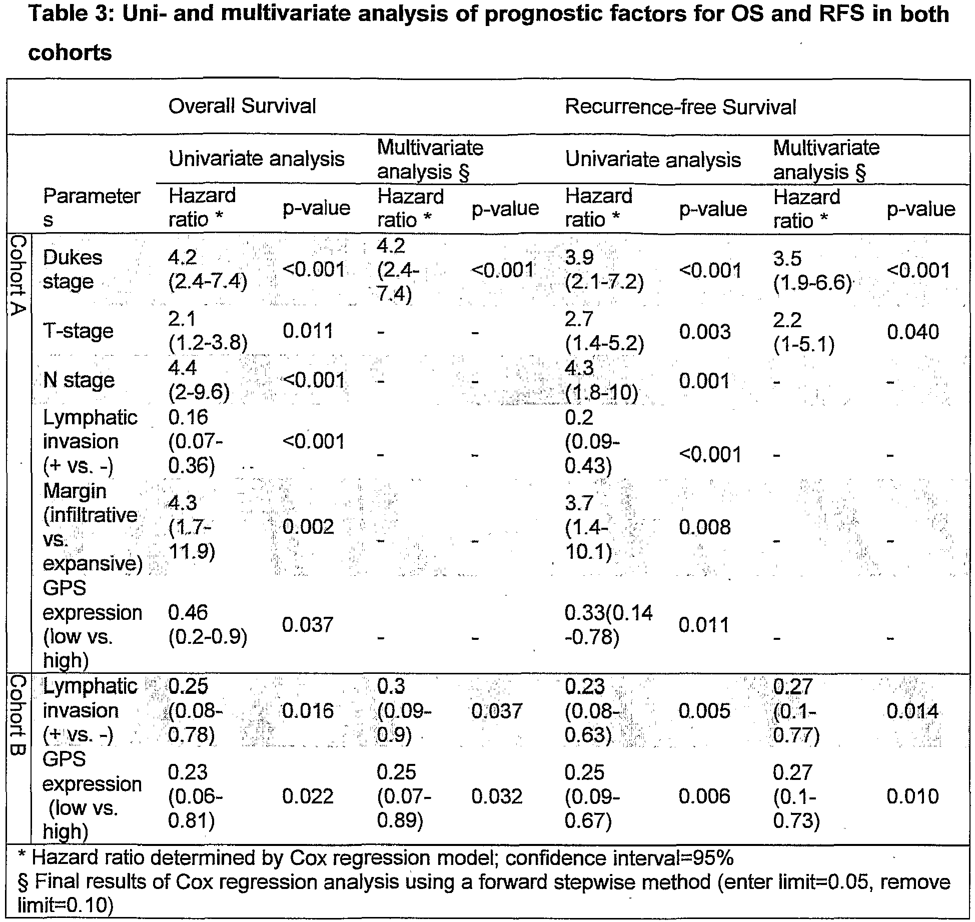

### EXAMPLE 11: Ki-67 is not associated with clinico-pathologic variables or survival

Ki-67 immunostaining was performed on tissue sections from Cohort A tumours only as paraffin-embedded samples were unavailable for Cohort B (FIG. 1, stage 3). Nuclear staining was detected in all 73 CRC tumours. Ki-67 PI ranged from 25 to 96 %, with a mean value of 76.3±17.5. Using the mean Ki-67 value as a cut-off point, tumours were assigned into two groups with low or high PI. Ki-67 PI was neither associated with clinico-pathologic variables (Table 2) nor survival (FIG. 3). When the survival analysis was limited to the patients with the highest and lowest Ki-67 values, no statistical difference was observed (data not shown). The sum of these results indicates that the low expression of growth-related genes is associated with poor outcome in colorectal cancer, and Ki-67 was not sensitive enough to detect an association. These findings can be used as additional criteria for identifying patients at high risk of early death from cancer.

### EXAMPLE 12: Selection of correlated cell proliferation genes

Cohort B (55 German CRC patients; Table 2) were first classified into low and high proliferation groups using the 38 gene cell proliferation signature (Table C) and the K-means clustering method (Pearson uncentered, 1000 permutations, threshold of occurrence in the same cluster sat at 80%). Statistical Analysis of Microarrays (SAM) was then applied to identify differentially expressed genes between low and high proliferation groups (FDR=0) when all filtered genes (16041 genes) were included for the analysis. 754 genes were found to be over-expressed in high proliferation group. The GATHER gene ontology program was then used to identify the most over-represented gene ontology categories within the list of differentially expressed genes. The cell cycle category was the most over-represented category within the list of differentially expressed genes. 102 cell cycle genes which are differentially expressed between the low and high proliferation groups (in addition to the original 38 gene signature) are shown in Table D.

**Table D: Cell Cycle Genes that are Differentially Expressed in Low and High Proliferation**

| **Gene Title** | **Gene Symbol** | **Chromosomal Location** | **Probe Set ID** | **Representative Public ID** |
|---|---|---|---|---|
| asp (abnormal spindle) homolog, microcephaly associated (Drosophila) | ASPM | chr1 q31 | 219918_s_at | NM_018123 |
| aurora kinase A | AURKA | chr20q13.2-q13.3 | 204092_s_at | NM_003600 |
| | | | 208079_s_at | NM_003158 |
| aurora kinase B | AURKB | chr17p13.1 | 209464_at | AB011446 |
| baculoviral IAP repeat-containing 5 (survivin) | BIRC5 | chr17q25 | 202094_at | AA648913 |
| | | | 202095_s_at | NM_001168 |
| | | | 210334_x_at | AB028869 |
| Bloom syndrome | BLM | chr15q26.1 | 205733_at | NM_000057 |
| breast cancer 1, early onset | BRCA1 | chr17q21 | 204531_s_at | NM_007295 |
| | | | 211851_x_at | AF005068 |
| BUB1 budding uninhibited by benzimidazoles 1 homolog (yeast) | BUB1 | chr2q14 | 209642_at | AF043294 |
| | | | 215509_s_at | AL137654 |
| BUB1 budding uninhibited by benzimidazoles 1 homolog beta (yeast) | BUB1B | chr15q15 | 203755_at | NM_001211 |
| cyclin A2 | CCNA2 | chr4q25-q31 | 203418_at | NM_001237 |
| | | | 213226_at | AI346350 |
| cyclin B1 | CCNB1 | chr5q12 | 214710_s_at | BE407516 |
| cyclin B2 | CCNB2 | chr15q22.2 | 202705_at | NM_004701 |
| cyclin E2 | CCNE2 | chr8q22.1 | 205034_at | NM_004702 |
| | | | 211814_s_at | AF112857 |
| cyclin F | CCNF | chr16p13.3 | 204826_at | NM_001761 |
| | | | 204827_s_at | U17105 |
| cyclin J | CCNJ | chr10pter-q26.12 | 219470_x_at | NM_019084 |
| cyclin T2 | CCNT2 | chr2q21.3 | 204645_at | NM_001241 |
| chaperonin containing TCP1, subunit 2 (beta) | CCT2 | chr12q15 | 201946_s_at | AL545982 |
| cell division cycle 20 homolog (S. cerevisiae) | CDC20 | chr1 p34.1 | 202870_s_at | NM_001255 |
| cell division cycle 25 homolog A (S. pombe) | CDC25A | chr3p21 | 204695_at | AI343459 |
| cell division cycle 25 homolog C (S. pombe) | CDC25C | chr5q31 | 205167_s_at | NM_001790 |
| | | | 217010_s_at | AF277724 |
| cell division cycle 27 homolog (S. cerevisiae) | CDC27 | chr17q12-q23.2 | 217879_at | AL566824 |
| cell division cycle 6 homolog (S. cerevisiae) | CDC6 | chr17q21.3 | 203968_s_at | NM_001254 |
| cyclin-dependent kinase 2 | CDK2 | chr12q13 | 204252_at | M68520 |
| | | | 211804_s_at | AB012305 |
| cyclin-dependent kinase 4 | CDK4 | chr12q14 | 202246_s_at | NM_000075 |
| cyclin-dependent kinase inhibitor 3 (CDK2-associated dual specificity phosphatase) | CDKN3 | chr14q22 | 209714_s_at | AF213033 |
| chromatin licensing and DNA replication factor 1 | CDT1 | chr16q24.3 | 209832_s_at | AF321125 |
| centromere protein E, 312kDa | CENPE | chr4q24-q25 | 205046_at | NM_001813 |
| centromere protein F, 350/400ka (mitosin) | CENPF | chr1q32-q41 | 207828_s_at | NM_005196 |
| | | | 209172_s_at | U30872 |
| chromatin assembly factor 1, subunit A (p150) | CHAF1A | chr19p13.3 | 203975_s_at | BF000239 |
| | | | 203976_s_at | NM_005483 |
| | | | 214426_x_at | BF062223 |
| CHK2 checkpoint homolog (S. pombe) | CHEK2 | chr22q11\|22q12. 1 | 210416_s_at | BC004207 |
| CDC28 protein kinase regulatory subunit 1 B | CKS1B | chr1q21.2 | 201897_s_at | NM_001826 |
| CDC28 protein kinase regulatory subunit 2 | CKS2 | chr9q22 | 204170_s_at | NM_001827 |
| DEAD/H (Asp-Glu-Ala-Asp/His) box polypeptide 11 (CHL1-like helicase homolog, S. cerevisiae) | DDX11 | chr12p11 | 210206_s_at | U33833 |
| extra spindle pole bodies homolog 1 (S. cerevisiae) | ESPL1 | chr12q | 38158_at | D79987 |
| exonuclease 1 | EXO1 | chr1 q42-q43 | 204603_at | NM_003686 |
| fumarate hydratase | FH | chr1q42.1 | 203032_s_at | AI363836 |
| fyn-related kinase | FRK | chr6q21-q22.3 | 207178_s_at | NM_002031 |
| G-2 and S-phase expressed 1 | GTSE1 | chr22q13.2-q13.3 | 204318_s_at | NM_016426 |
| | | | 215942_s_at | BF973178 |
| high mobility group AT-hook 1 | HMGA1 | chr6p21 | 206074_s_at | NM_002131 |
| high-mobility group box 2 | HMGB2 | chr4q31 | 208808_s_at | BC000903 |
| interleukin enhancer binding factor 3, 90kDa | ILF3 | chr19p13.2 | 208931_s_at | AF147209 |
| | | | 211375_s_at | AF141870 |
| kinesin family member 11 | KIF11 | chr10q24.1 | 204444_at | NM_004523 |
| kinesin family member 22 | KIF22 | chr16p11.2 | 202183_s_at | NM_007317 |
| | | | 216969_s_at | AC002301 |
| kinesin family member 23 | KIF23 | chr15q23 | 204709_s_at | NM_004856 |
| kinesin family member 2C | KIF2C | chr1p34.1 | 209408_at | U63743 |
| | | | 211519_s_at | AY026505 |
| kinesin family member C1 | KIFC1 | chr6p21.3 | 209680_s_at | BC000712 |
| kinetochore associated 1 | KNTC1 | chr12q24.31 | 206316_s_at | NM_014708 |
| ligase I, DNA, ATP-dependent | LIG1 | chr19q13.2-q13.3 | 202726_at | NM_000234 |
| mitogen-activated protein kinase 1 | MAPK1 | chr22q11.2\|22q1 1.21 | 208351_s_at | NM_002745 |
| minichromosome maintenance complex component 2 | MCM2 | chr3q21 | 202107_s_at | NM_004526 |
| minichromosome maintenance complex component 4 | MCM4 | chr8q11.2 | 212141_at | AA604621 |
| | | | 212142_at | AI936566 |
| | | | 222036_s_at | AI859865 |
| | | | 222037_at | AI859865 |
| minichromosome maintenance complex component 5 | MCM5 | chr22q13.1 | 201755_at | NM_006739 |
| | | | 216237_s_at | AA807529 |
| antigen identified by monoclonal antibody Ki-67 | MKI67 | chr10q25-qter | 212020_s_at | AU152107 |
| | | | 212021_s_at | AU132185 |
| | | | 212022_s_at | BF001806 |
| | | | 212023_s_at | AU147044 |
| M-phase phosphoprotein 1 | MPHOS PH1 | chr10q23.31 | 205235_s_at | NM_016195 |
| M-phase phosphoprotein 9 | MPHOS PH9 | chr12q24.31 | 206205_at | NM_022782 |
| mutS homolog 6 (E. coli) | MSH6 | chr2p16 | 202911_at | NM_000179 |
| | | | 211450_s_at | D89646 |
| non-SMC condensin I complex, subunit D2 | NCAPD2 | chr12p13.3 | 201774_s_at | AK022511 |
| non-SMC condensin I complex, subunit G | NCAPG | chr4p15.33 | 218662_s_at | NM_022346 |
| | | | 218663_at | NM_022346 |
| non-SMC condensin I complex, subunit H | NCAPH | chr2q11.2 | 212949_at | D38553 |
| NDC80 homolog, kinetochore complex component (S. cerevisiae) | NDC80 | chr18p11.32 | 204162_at | M_006101 |
| NIMA (never in mitosis gene a)-related kinase 2 | NEK2 | chr1q32.2-q41 | 204641_at | NM_002497 |
| | | chr1q32.2-q41 | 211080_s_at | Z25425 |
| NIMA (never in mitosis gene a)-related kinase 4 | NEK4 | chr3p21.1 | 204634_at | NM_003157 |
| non-metastatic cells 1, protein (NM23A) expressed in | NME1 | chr17q21.3 | 201577_at | NM_000269 |
| nucleolar and coiled-body phosphoprotein 1 | NOLC1 | chr10q24.32 | 205895_s_at | NM_004741 |
| nucleophosmin (nucleolar phosphoprotein B23, numatrin) | NPM1 | chr5q35 | 221691_x_at | AB042278 |
| | | | 221923_s_at | AA191576 |
| nucleoporin 98kDa | NUP98 | chr11p15.5 | 203194_s_at | AA527238 |
| origin recognition complex, subunit 1-like (yeast) | ORC1 L | chr1p32 | 205085_at | NM_004153 |
| origin recognition complex, subunit 4-like (yeast) | ORC4L | chr2q22-q23 | 203351_s_at | AF047598 |
| origin recognition complex, subunit 6 like (yeast) | ORC6L | chr16q12 | 219105_x_at | NM_014321 |
| protein kinase, membrane associated tyrosine/threonine 1 | PKMYT1 | chr16p13.3 | 204267_x_at | NM_004203 |
| polo-like kinase 1 (Drosophila) | PLK1 | chr16p12.1 | 202240_at | NM_005030 |
| polo-like kinase 4 (Drosophila) | PLK4 | chr4q28 | 204886_at | AL043646 |
| | | | 204887_s_at | NM_014264 |
| | | | 211088_s_at | Z25433 |
| PMS1 postmeiotic segregation increased 1 (S. cerevisiae) | PMS1 | chr2q31-q33\|2q31.1 | 213677_s_at | BG434893 |
| polymerase (DNA directed), theta | POLQ | chr3q13.33 | 219510_at | NM_006596 |
| protein phosphatase 1D magnesium-dependent, delta isoform | PPM1D | chr17q23.2 | 204566_at | NM_003620 |
| protein phosphatase 2 (formerly 2A), regulatory subunit A, beta isoform | PPP2R1 B | chr11 q23.2 | 202886_s_at | M65254 |
| protein phosphatase 6, catalytic subunit | PPP6C | chr9q33.3 | 206174_s_at | NM_002721 |
| protein regulator of cytokinesis 1 | PRC1 | chr15q26.1 | 218009_s_at | NM_003981 |
| primase, DNA, polypeptide 1 (49kDa) | PRIM1 | chr12q13 | 205053_at | NM_000946 |
| primase, DNA, polypeptide 2 (58kDa) | PRIM2 | chr6p12-p11.1 | 205628_at | NM_000947 |
| protein arginine methyltransferase 5 | PRMT5 | chr14q11.2-q21 | 217786_at | NM_006109 |
| pituitary tumor-transforming 1 | PTTG1 | chr5q35.1 | 203554_x_at | NM_004219 |
| pituitary tumor-transforming 3 | PTTG3 | chr8q13.1 | 208511_at | NM_021000 |
| RAD51 homolog (RecA homolog, E. coli) (S. cerevisiae) | RAD51 | chr15q15.1 | 205024_s_at | NM_002875 |
| RAD54 homolog B (S. cerevisiae) | RAD54B | chr8q21.3-q22 | 219494_at | NM_012415 |
| Ras association (RaIGDS/AF-6) domain family member 1 | RASSF1 | chr3p21.3 | 204346_s_at | NM_007182 |
| replication factor C (activator 1) 2, 40kDa | RFC2 | chr7q11.23 | 1053_at | M87338 |
| | | | 203696_s_at | NM_002914 |
| replication factor C (activator 1) 3, 38kDa | RFC3 | chr13q12.3-q13 | 204128_s_at | NM_002915 |
| replication factor C (activator 1) 5, 36.5kDa | RFC5 | chr12q24.2-q24.3 | 203209_at | BC001866 |
| | | | 203210_s_at | NM_007370 |
| ribonuclease H2, subunit A | RNASEH 2A | chr19p13.13 | 203022_at | NM_006397 |
| SET nuclear oncogene | SET | chr9q34 | 213047_x_at | AI278616 |
| S-phase kinase-associated protein 2 (p45) | SKP2 | chr5p13 | 210567_s_at | BC001441 |
| structural maintenance of chromosomes 2 | SMC2 | chr9q31.1 | 204240_s_at | NM_006444 |
| | | | 213253_at | AU154486 |
| sperm associated antigen 5 | SPAG5 | chr17q11.2 | 203145_at | NM_006461 |
| SFRS protein kinase 1 | SRPK1 | chr6p21.3-p21.2 | 202199_s_at | AW082913 |
| signal transducer and activator of transcription 1, 91 kDa | STAT1 | chr2q32.2 | AFFX-HUMISGF3A/ M97935_5_at | AFFX-HUMISGF3A/M9 7935_5 |
| suppressor of variegation 3-9 homolog 2 (Drosophila) | SUV39H 2 | chr10p13 | 219262_at | NM_024670 |
| TAR DNA binding protein | TARDBP | chr1 p36.22 | 200020_at | NM_007375 |
| transcription factor A, mitochondrial | TFAM | chr10q21 | 203177_x_at | NM_003201 |
| topoisomerase (DNA) II binding protein 1 | TOPBP1 | chr3q22.1 | 202633_at | NM_007027 |
| TPX2, microtubule-associated, homolog (Xenopus laevis) | TPX2 | chr20q11.2 | 210052_s_at | AF098158 |
| TTK protein kinase | TTK | chr6q13-q21 | 204822_at | NM_003318 |
| tubulin, gamma 1 | TUBG1 | chr17q21 | 201714_at | NM_001070 |

### Conclusions

The present invention is the first to report an association between a gene proliferation signature and major clinico-pathologic variables as well as outcome in colorectal cancer. The disclosed study investigated the proliferation state of tumours using an *in vitro-* derived multi-gene proliferation signature and by Ki-67 immunostaining. According to the results herein, low expression of the GPS in tumours was associated with a higher risk of recurrence and shorter survival in two independent cohorts of patients. In contrast, Ki-67 proliferation index was not associated with any clinically relevant endpoints.

The colorectal GPS encompasses 38 mitotic cell cycle genes and includes a core set of genes (CDC2, RFC4, PCNA, CCNE1, CDK7, MCM genes, FEN1, MAD2L1, MYBL2, RRM2 and BUB3) that are part of proliferation signatures defined for tumours of the breast (40),(41), ovary (42), liver (43), acute lymphoblastic leukaemia (44), neuroblastoma (45), lung squamous cell carcinoma (46), head and neck (47), prostate (48), and stomach (49). This represents a conserved pattern of expression, as most of these genes have been found to be highly overexpressed in fast-growing tumours and to reflect a high proportion of rapidly cycling cells (50). Therefore, the expression level of the colorectal GPS provides a measure for the proliferative state of a tumour.

In this study, several clinico-pathologic variables related to poor outcome (disease stage, lymph node metastasis and lymphatic invasion) were associated with low GPS expression in Cohort A patients. In Cohort B, consisting entirely of stage II tumours, the study assessed the association between the GPS and lymphatic invasion. The association failed to reach statistical significance due to the small number of tumours with lymphatic invasion in this cohort (5/55). Without being bound by theory, the low GPS expression in more advanced tumours may indicate that CRC progression is not driven by enhanced proliferation. While accelerated proliferation may still be an important driving force during the initial phases of tumourigenesis, it is possible that more advanced disease is more dependent on processes such as genetic instability to allow continuous selection. Consistent with our finding, two large-scale studies reported an association between decreased expression of CDK2, cyclin E and A, and advanced stage, deep infiltration and lymph node metastasis (51),(52).

The relationship between low GPS and unfavourable clinico-pathologic variables suggested that the GPS should also predict patient outcome. Indeed, in both Cohort A and B, low GPS expression was associated with a higher risk of recurrence and shorter overall and recurrence-free survival. In Cohort B, where all patients had stage II tumours, the association remained in multivariate analysis. However, in Cohort A, where patients had stage I-IV disease, the association was not independent of tumour stage. The number of patients with and without recurrence, within each stage of disease in Cohort A, was probably insufficient to demonstrate an independent association between the GPS and survival. In Cohort B, low GPS expression and lymphatic invasion remained independent predictors in multivariate analysis suggesting that the GPS may improve the prediction of CRC patient outcome within the same disease stage. Not surprisingly, the presence of lymph node and distant organ involvement were the most powerful predictors of outcome as these are direct manifestations of tumour metastasis.

Treatment with radiotherapy or chemotherapy, used in 18% and 27% of Cohort A patients respectively, was a possible confounding factor in this study. Theoretically, the improved survival associated with elevated GPS expression might reflect the better response of fast proliferating tumours to cancer treatment (53),(54). However, no correlation was found between treatment and GPS expression. Furthermore, no patients in Cohort B received adjuvant therapy indicating that the association between GPS and survival is independent of treatment. It should be noted that this study was not designed to investigate the relationship between tumour proliferation and response to chemotherapy or radiotherapy.

The sample size may also explain the lack of an association between clinico-pathologic variables and survival with Ki-67 PI in the present study. As mentioned above, other studies on Ki-67 and CRC outcome have reported inconsistent findings. However, in the three other CRC studies with the largest sample size a low Ki-67 PI was associated with a worse prognosis (27),(29),(30). We came to the same conclusion applying the GPS, but based on a much smaller sample size. The multi-gene expression analysis was therefore a more sensitive tool to assess the relationship between proliferation and prognosis than the Ki-67 PI.

The biological reason behind an unfavourable prognosis in tumours with a low GPS will involve further investigation. Mechanisms that could potentially contribute to worse clinical outcome in low GPS tumours include: (i) a more effective immune response to rapidly proliferating tumours; (ii) a higher level of genetic damage that may render cancer cells more resistant to apoptosis, and increase invasiveness, but also perturb smooth replication machinery; (iii) an increased number of cancer stem cells that divide slowly, similar to normal stem cells, but have a high metastatic potential; and (iv) a higher proportion of microsatellite unstable tumours which have a high proliferation rate but a relatively good prognosis.

In sum, the present invention has clarified the previous, conflicting results relating to the prognostic role of cell proliferation in colorectal cancer. A GPS has been developed using CRC cell lines and has been applied to two independent patient cohorts. It was found that low expression of growth-related genes in CRC was associated with more advanced tumour stage (Cohort A) and poor clinical outcome within the same stage (Cohort B). Multi-gene expression analysis was shown as a more powerful indicator than the long-established proliferation marker, Ki-67, for predicting outcome. For future studies, it will be useful to determine the reasons that CRC differs from other common epithelia cancers, such as breast and lung cancers (e.g., in reference to Ki-67). This will likely provide insights into important underlying biological mechanisms. From a practical viewpoint, the ability to stratify recurrence risk within a given pathological stage could enable adjuvant therapy to be targeted more accurately. Thus, GPS expression can be used as an adjunct to conventional staging for identifying patients at high risk of recurrence and death from colorectal cancer.

All publications and patents mentioned in the above specification are herein incorporated by reference.

Wherein in the foregoing description reference has been made to integers or components having known equivalents, such equivalents are herein incorporated as if individually set fourth.

Although the invention has been described by way of example and with reference to possible embodiments thereof, it is to be appreciated that improvements and/or modifications may be made without departing from the scope or the spirit thereof.

### References:

1. Evan GI, Vousden KH: Proliferation, cell cycle and apoptosis in cancer. Nature 411:342-8, 2001
2. Whitfield ML, George LK, Grant GD, et al: Common markers of proliferation. Nat Rev Cancer 6:99-106, 2006
3. Rew DA, Wilson GD: Cell production rates in human tissues and tumours and their significance. Part 1: an introduction to the techniques of measurement and their limitations. Eur J Surg Oncol 26:227-38, 2000
4. Endle E, Gerdes J: The Ki-67 protein: fascinating forms and an unknown function. Exp Cell Res 257:231-7, 2000
5. Brown DC, Gatter KC: Ki67 protein: The immaculate deception. Histopathology 40:2-11, 2002
6. Paik S, Shak S, Tang G, et al: A multigene assay to predict recurrence of tamoxifen-treated, node-negative breast cancer. N Engl J Med 351:2817-26, 2004
7. Ofner D, Grothaus A, Riedmann B, et al: MIB1 in colorectal carcinomas: its evaluation by three different methods reveals lack of prognostic significance. Anal Cell Pathol 12:61-70, 1996
8. Ihmann T, Liu J, Schwabe W, et al: High-level mRNA quantification of proliferation marker pKi-67 is correlated with favorable prognosis in colorectal carcinoma. J Cancer Res Clin Oncol 130:749-756, 2004
9. Van Oijen MG, Medema RH, Slootweg PJ, et al: Positivity of the proliferation marker pKi-67 in non-cycling cells. Am J Clin Pathol 110:24-31, 1998
10. Duchrow M, Ziemann T, Windhövel U, et al: Colorectal carcinomas with high MIB-1 labelling indices but low pKi67 mRNA levels correlate with better prognostic outcome. Histopathology 42:566-574, 2003
11. Evans C, Morrison I, Heriot AG, et al: The correlation between colorectal cancer rates of proliferation and apoptosis and systemic cytokine levels; plus their influence upon survival. Br J Cancer 94:1412-9, 2006
12. Rosati G, Chiacchio R, Reggiardo G, et al: Thymidylate synthase expression, p53, bcl-2, Ki-67 and p27 in colorectal cancer: relationships with tumour recurrence and survival. Tumour Biol 25:258-63, 2004
13. Ishida H, Miwa H, Tatsuta M, et al: Ki-67 and CEA expression as prognostic markers in Dukes' C colorectal cancer. Cancer Lett 207:109-115, 2004
14. Buglioni S, D'Agnano I, Cosimelli M, et al: Evaluation of multiple bio-pathological factors in colorectal adenocarcinomas: independent prognostic role of p53 and bcl-2. Int J Cancer 84:545-52, 1999
15. Guerra A, Borda F, Javier Jimenez F, et al: Multivariate analysis of prognostic factors in resected colorectal cancer: a new prognostic index. Eur J Gastroenterol Hepatol 10:51-8, 1998
16. Kyzer S, Gordon PH: Determination of proliferative activity in colorectal carcinoma using monoclonal antibody Ki67. Dis Colon Rectum 40:322-5, 1997
17. Jansson A, Sun XF: Ki-67 expression in relation to clinicopathological variables and prognosis in colorectal adenocarcinomas. APMIS105:730-4, 1997
18. Baretton GB, Diebold J, Christoforis G, et al: Apoptosis and immunohistochemical bcl-2 expression in colorectal adenomas and carcinomas. Aspects of carcinogenesis and prognostic significance. Cancer 77:255-64, 1996
19. Sun XF, Carstensen JM, Stal O, et al: Proliferating cell nuclear antigen (PCNA) in relation to ras, c-erbB-2, p53, clinico-pathological variables and prognosis in colorectal adenocarcinoma. Int J Cancer 69:5-8, 1996
20. Kubota Y, Petras RE, Easley KA, et al: Ki-67-determined growth fraction versus standard staging and grading parameters in colorectal carcinoma. A multivariate analysis. Cancer 70:2602-9, 1992
21. Valera V, Yokoyama N, Walter B, et al: Clinical significance of Ki-67 proliferation index in disease progression and prognosis of patients with resected colorectal carcinoma. Br J Surg 92:1002-7, 2005
22. Dziegiel P, Forgacz J, Suder E, et al: Prognostic significance of metallothionein expression in correlation with Ki-67 expression in adenocarcinomas of large intestine. Histol Histopathol 18:401-7, 2003
23. Scopa CD, Tsamandas AC, Zolata V, et al: Potential role of bcl-2 and Ki-67 expression and apoptosis in colorectal carcinoma: a clinicopathologic study. Dig Dis Sci 48:1990-7, 2003
24. Bhatavdekar JM, Patel DD, Chikhlikar PR, et al: Molecular markers are predictors of recurrence and survival in patients with Dukes B and Dukes C colorectal adenocarcinoma. Dis Colon Rectum 44:523-33, 2001
25. Chen YT, Henk MJ, Carney KJ, et al: Prognostic Significance of Tumor Markers in Colorectal Cancer Patients: DNA Index, S-Phase Fraction, p53 Expression, and Ki-67 Index. J Gastrointest Surg 1:266-273, 1997
26. Choi HJ, Jung IK, Kim SS, et al: Proliferating cell nuclear antigen expression and its relationship to malignancy potential in invasive colorectal carcinomas. Dis Colon Rectum 40:51-9, 1997
27. Hilska M, Collan YU, O Laine VJ, et al: The significance of tumour markers for proliferation and apoptosis in predicting survival in colorectal cancer. Dis Colon Rectum 48:2197-208, 2005
28. Salminen E, Palmu S, Vahlberg T, et al: Increased proliferation activity measured by immunoreactive Ki67 is associated with survival improvement in rectal/recto sigmoid cancer. World J Gastroenterol 11:3245-9, 2005
29. Garrity MM, Burgart LJ, Mahoney MR, et al: Prognostic value of proliferation, apoptosis, defective DNA mismatch repair, and p53 overexpression in patients with resected Dukes' B2 or C colon cancer: a North Central Cancer Treatment Group Study. J Clin Oncol 22:1572-82, 2004
30. Allegra CJ, Paik S, Colangelo LH, et al: Prognostic value of thymidylate synthase, Ki-67, and p53 in patients with Dukes' B and C colon cancer: a National Cancer Institute-National Surgical Adjuvant Breast and Bowel Project collaborative study. J Clin Oncol 21:241-50, 2003
31. Palmqvist R, Sellberg P, Oberg A, et al: Low tumour cell proliferation at the invasive margin is associated with a poor prognosis in Dukes' stage B colorectal cancers. Br J Cancer 79:577-81, 1999
32. Paradiso A, Rabinovich M, Vallejo C, et al: p53 and PCNA expression in advanced colorectal cancer: response to chemotherapy and long-term prognosis. Int J Cancer 69:437-41, 1996
33. Neoptolemos JP, Oates GD, Newbold KM, et al: Cyclin/proliferation cell nuclear antigen immunohistochemistry does not improve the prognostic power of Dukes' or Jass' classifications for colorectal cancer. Br J Surg 82:184-7, 1995
34. Compton C, Fenoglio-Preiser CM, Pettigrew N, et al: American joint committee on cancer prognostic factors consensus conference. Colorectal working group. Cancer 88: 1739-1757, 2000
35. Colantuoni C, Henry G, Zeger S, et al: SNOMAD (Standarization and NOrmalization of MicroArray Data): web-accessible gene expression data analysis. Bioinformatics 18:1540-1541,2002
36. Livak KJ, Schmittgen TD: Analysis of Relative Gene Expression Data Using Real-Time Quantitative PCR and the 2-ΔΔCT Method. METHODS 25:402-408, 2001
37. Pocock SJ, Clayton TC, Altman DG: Survival plots of time-to-event outcomes in clinical trials: good practice and pitfalls. Lancet 359:1686-89, 2002
38. Trusher VG, Tibshirani R, Chu G: Significance analysis of microarrays applied to the ionizing radiation response. Proc Natl Acad Sci USA 98:5116-21, 2001
39. Hosack DA, Dennis G, Sherman BT, et al: Identifying biological themes within lists of genes with EASE. Genome biology 4:R70, 2003
40. Perou CM, Jeffrey SS, DE Rijn MV: Distinctive gene expression patterns in human mammary epithelial cells and breast cancers. Proc. Natl. Acad. Sci. USA 96:9212-17, 1999
41. Perou CM: Molecular portraits of human breast tumours. Nature 406:747-752, 2000
42. Welsh JB, Zarrinkar PP, Sapinoso LM, et al: Analysis of gene expression profiles in normal and neoplastic ovarian tissue samples identifies candidate molecular markers of epithelial ovarian cancer. Proc. Natl Acad. Sci. USA 98:1176-1181, 2001
43. Chen X, Cheung ST, So S, et al: Gene expression patterns in human liver cancers. Mol. Biol. Cell 13:1929-1939, 2002
44. Kirschner-Schwabe R, Lottaz C, Todling J, et al: Expression of late cell cycle genes and an increased proliferative capacity characterize very early relapse of childhood acute lymphoblastic leukemia. Clin Cancer Res 12:4553-61, 2006
45. Krasnoselsky AL, Whiteford CC, Wei JS, et al: Altered expression of cell cycle genes distinguishes aggressive neuroblastoma. Oncogene 24:1533-1541, 2005
46. Inamura K, Fujiwara T, Hoshida Y, et al: Two subclasses of lung squamous cell carcinoma with different gene expression profiles and prognosis identified by hierarchical clustering and non-negative matrix factorization. Oncogene 24:7105-13, 2005
47. Chung CH, Parker JS, Karaca G, et al: Molecular classification of head and neck squamous cell carcinomas using patterns of gene expression. Cancer Cell 5:489-500, 2004
48. LaTulippe E, Satagopan J, Smith A, et al: Comprehensive gene expression analysis of prostate cancer reveals distinct transcriptional programs associated with metastatic disease. Cancer Res 62:4499-4506, 2002
49. Hippo Y, Taniguchi H, Tsutumi S, et al: Global gene expression analysis of gastric cancer by oligonucleotide microarrays. Cancer Res 62:233-40, 2002
50. Whitfield ML, Sherlock G, Saldanha AJ, et al: Identification of genes periodically expressed in the human cell cycle and their expression in tumours. Mol Biol Cell 13:1977-2000, 2002
51. Li JQ, Miki H, Ohmori M, et al: Expression of cyclin E and cyclin-dependent kinase 2 correlates with metastasis and prognosis in colorectal carcinoma. Hum Pathol 32:945-53, 2001
52. Li JQ, Miki H, Wu F, et al: Cyclin A correlates with carcinogenesis and metastasis, and p27 (kip1) correlates with lymphatic invasion, in colorectal neoplasms. Hum Pathol 33, 1006-15, 2002
53. Itamochi H, Kigawa J, Sugiyama T, et al: Low proliferation activity may be associated with chemoresistance in clear cell carcinoma of the ovary. Obstet Gynecol 100:281-287, 2002
54. Imdahl A, Jenkner J, Ihling C, et al: Is MIB-1 proliferation index a predictor for response to neoadjuvant therapy in patients with esophageal cancer? Am J Surg 179:514-520, 2000

### FURTHER EMBODIMENTS

1. A prognostic signature for determining progression of gastrointestinal cancer in a patient, comprising one or more genes selected from Table A, Table B, Table C or Table D.
2. The signature of item 1, wherein the signature comprises one or more genes selected from any one of CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37.
3. A method of predicting the likelihood of long-term survival of a gastrointestinal cancer patient without the recurrence of gastrointestinal cancer, comprising determining the expression level of one or more prognostic RNA transcripts or their expression products in a gastrointestinal sample obtained from the patient, normalized against the expression level of all RNA transcripts or their products in the gastrointestinal cancer tissue sample, or of a reference set of RNA transcripts or their expression products;
   wherein the prognostic RNA transcript is the transcript of one or more genes selected from table A, Table B, Table C or Table D ; and
   establishing likelihood of long-term survival without gastrointestinal cancer recurrence.
4. The method of item 3, wherein at least one prognostic RNA transcripts or its expression products is selected from any one of CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37
5. The method of item 3 or item 4 comprising determining the expression level of at least two, at least five, at least 10, or at least 15 of the prognostic RNA transcripts or their expression products.
6. The method according to any one of items 3 to 5, wherein increased expression of the one or more prognostic RNA transcripts or their expression products indicates an increased likelihood of long-term survival without gastrointestinal cancer recurrence.
7. The method according to any one of items 3 to 5, wherein a predictive model is applied, established by applying a predictive method to expressions levels of the predictive signature in recurrent and non-recurrent tumour samples, to establishing likelihood of long-term survival without gastrointestinal cancer recurrence.
8. The method of item 7, wherein said predictive method is selected from the group consisting of linear models, support vector machines, neural networks, classification and regression trees, ensemble learning methods, discriminant analysis, nearest neighbor method, bayesian networks, independent components analysis.
9. The method of any one of items 3 to 8 wherein the gastrointestinal cancer is gastric cancer or colorectal cancer.
10. The method of any one of items 3 to 9 wherein the expression level of one or more prognostic RNA transcripts is determined.
11. The method of any one of items 3 to 10 wherein the RNA is isolated from a fixed, wax- embedded gastrointestinal cancer tissue specimen of the patient.
12. The method of any one of items 3 to 10 wherein the RNA is isolated from core biopsy tissue or fine needle aspirate cells.
13. An array comprising polynucleotides hybridizing to two or more genes selected from table A, Table B, Table C or Table D.
14. An array of item 13 comprising polynucleotides hybridizing to two or more of the following genes: CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37.
15. The array of item 13 or item 14 comprising polynucleotides hybridizing to at least 3, at least five, at least 10 or at least 15 of the genes.
16. The array of item 13 comprising polynucleotides hybridizing to the following genes: CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37.
17. The array of any one of items 13 to 16 wherein the polynucleotides are cDNAs.
18. The array of item 17 wherein the cDNAs are about 500 to 5000 bases long.
19. The array of item any one of items 13 to 16 wherein the polynucleotides are oligonucleotides.
20. The array of item 19 wherein the oligonucleotides are about 20 to 80 bases long.
21. The array of any one of items 13 to 20 wherein the solid surface is glass.
22. A method of predicting the likelihood of long-term survival of a patient diagnosed with gastrointestinal cancer, without the recurrence of gastrointestinal cancer, comprising the steps of:
   (1) determining the expression levels of the RNA transcripts or the expression products of genes or a gene selected from table A, Table B, Table C or Table D, in a gastrointestinal cancer tissue sample obtained from the patient, normalized against the expression levels of all RNA transcripts or their expression products in the gastrointestinal cancer tissue sample, or of a reference set of RNA transcripts or their products;
   (2) subjecting the data obtained in step (1) to statistical analysis; and
   (3) determining whether the likelihood of the long-term survival has increased or decreased;
      and establishing the likelihood of long-term survival without gastrointestinal cancer recurrence.
23. The method of item 22, wherein at least one prognostic RNA transcripts or its expression products is selected from any one CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37.
24. The method of item 22 or item 23 wherein the statistical analysis is performed by using the Cox Proportional Hazards model.
25. A method of preparing a personalized genomics profile for a cancer patient, comprising the steps of: (a) subjecting RNA extracted from a gastrointestinal tissue obtained from the patient to gene expression analysis; (b) determining the expression level of one or more genes selected from the gastrointestinal cancer gene set listed in any one of Table A, Table B, Table C or Table D, wherein the expression level is normalized against a control gene or genes and optionally is compared to the amount found in a gastrointestinal cancer reference tissue set; and (c) creating a report summarizing the data obtained by the gene expression analysis.
25. The method of item 24, wherein the gastrointestinal tissue comprises gastrointestinal cancer cells.
26. The method of item 24 wherein the gastrointestinal tissue is obtained from a fixed, paraffin-embedded biopsy sample.
27. The method of item 26 wherein the RNA is fragmented.
28. The method of any on of items 22 to 27 wherein the report includes prediction of the likelihood of long term survival of the patient.
29. The method of any one of items 22 to 29 wherein the report includes recommendation for a treatment modality of the patient.
30. A prognostic method comprising: (a) subjecting a sample comprising gastrointestinal cancer cells obtained from a patient to quantitative analysis of the levels of RNA transcripts of at least one gene selected from any one of Table A, Table B, Table C or table D, or its product, and (b) identifying the patient as likely to have an increased likelihood of long-term survival without gastrointestinal cancer recurrence if normalized expression levels of the gene or genes, or their products, are elevated above a defined expression threshold.
31. The method of item 30, wherein at least one prognostic RNA transcripts or its expression products is selected from any one CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, and CDC37.
32. The method of item 30 or item 31, wherein the levels of the RNA transcripts of the genes are normalized relative to the mean level of the RNA transcript or the product of two or more housekeeping genes.
33. The method of item 32 wherein the housekeeping genes are selected from the group consisting of glyceraldehyde-3-phosphate dehydrogenase (GAPDH), Cypl, albumin, actins, tubulins, cyclophilin hypoxantine phosphoribosyltransferase (HRPT), L32, 28S, and 185.
34. The method of any one of items 30 to 33 wherein the sample is subjected to global gene expression analysis of all genes present above the limit of detection.
35. The method of any one of items 30 to 34 wherein the levels of RNA transcripts of the genes are normalized relative to the mean signal of the RNA transcripts or the products of all assayed genes or a subset thereof.
36. The method of any one of items 30 to 35 wherein the levels of RNA transcripts are determined by quantitative RT-PCR, and the signal is a Ct value.
37. The method of item 35 wherein the assayed genes include at least 50 or at least 100 cancer related genes.
38. The method of any one of items 30 to 37 wherein the patient is human.
39. The method of any one of items 30 to 38 wherein the sample is a fixed, paraffin-embedded tissue (FPET) sample, or fresh or frozen tissue sample.
40. The method of any one of items 30 to 38 wherein the sample is a tissue sample from fine needle, core, or other types of biopsy.
41. The method of any one of items 30 to 40 wherein the quantitative analysis is performed by quantitative RT-PCR.
42. The method of any one of items 30 to 40 wherein the quantitative analysis is performed by quantifying the products of the genes.
43. The method of any one of items 30 to 40 wherein the products are quantified by immunohistochemistry or by proteomics technology.
44. The method of any one of items 30 to 43 further comprising the step of preparing a report indicating that the patient has an increased likelihood of long-term survival without gastrointestinal cancer recurrence.
45. A kit comprising one or more of (1) extraction buffer/reagents and protocol; (2) reverse transcription buffer/reagents and protocol; and (3) quantitative RT-PCR buffer/reagents and protocol suitable for performing the method of any one of items 3, 25, and 30.

## Claims

1. A method of predicting the likelihood of long-term survival of a gastrointestinal cancer patient without the recurrence of gastrointestinal cancer, comprising determining the expression level of one or more prognostic RNA transcripts or their expression products in a gastrointestinal sample obtained from the patient, normalized against the expression level of all RNA transcripts or their products in the gastrointestinal cancer tissue sample, or of a reference set of RNA transcripts or their expression products;
wherein the prognostic RNA transcript or expression product comprises the transcript of replication factor (activator 1) 4 (RFC4); and
establishing likelihood of long-term survival without gastrointestinal cancer recurrence wherein increased expression of the one or more prognostic RNA transcripts or their expression products indicates an increased likelihood of long-term survival without gastrointestinal cancer recurrence.

2. The method of claim 1, wherein the prognostic RNA transcripts or its expression products further comprises one or more genes selected from Table A, Table B, Table C or Table D.

3. The method of claim 1 or claim 2, wherein at least one prognostic RNA transcripts or its expression products, further comprises from any one of CDC2, MCM6, RPA3, MCM7, PCNA, G22P1, KPNA2, ANLN, APG7L, TOPK, GMNN, RRM1, CDC45L, MAD2L1, RAN, DUT, RRM2, CDK7, MLH3, SMC4L1, CSPG6, POLD2, POLE2, BCCIP, Pfs2, TREX1, BUB3, FEN1, DRF1, PREI3, CCNE1, RPA1, POLE3, RFC4, MCM3, CHEK1, CCND1, MYBL2, and CDC37

4. The method according to any one of claims 1 to 3, wherein a predictive model is applied, established by applying a predictive method to expressions levels of the predictive signature in recurrent and non-recurrent tumour samples, to establishing likelihood of long-term survival without gastrointestinal cancer recurrence.

5. The method of claim 4, wherein said predictive method is selected from the group consisting of linear models, support vector machines, neural networks, classification and regression trees, ensemble learning methods, discriminant analysis, nearest neighbor method, bayesian networks, independent components analysis.

6. The method of any one of claims 1 to 5 wherein the gastrointestinal cancer is gastric cancer or colorectal cancer.

7. The method of any one of claims 1 to 6 wherein the expression level of one or more prognostic RNA transcripts is determined.

8. The method of any one of claims 1 to 7, wherein the levels of the RNA transcripts of the genes are normalized relative to the mean level of the RNA transcript or the product of two or more housekeeping genes.

9. The method of claim 8 wherein the housekeeping genes are selected from the group consisting of glyceraldehyde-3-phosphate dehydrogenase (GAPDH), Cypl, albumin, actins, tubulins, cyclophilin hypoxantine phosphoribosyltransferase (HRPT), L32, 28S, and 185.

10. The method of any one of claims 1 to 9 wherein the levels of RNA transcripts of the genes are normalized relative to the mean signal of the RNA transcripts or the products of all assayed genes or a subset thereof.

11. The method of any one of claims 1 to 9 wherein the levels of RNA transcripts are determined by quantitative RT-PCR, and the signal is a Ct value.

12. The method of any one of claims 1 to 10 wherein the patient is human.

13. The method of any one of claims 1 to 12 wherein the sample is a fixed, paraffin-embedded tissue (FPET) sample, or fresh or frozen tissue sample, or a tissue sample from fine needle, core, or other types of biopsy.

14. The method of any one of claims 1 to 13 wherein: (a) the quantitative analysis is performed by quantitative RT-PCR; (b) the quantitative analysis is performed by quantifying the products of the genes; or (c) the products are quantified by immunohistochemistry or by proteomics technology.

15. The method of any one of claims 1 to 14 further comprising the step of preparing a report indicating that the patient has an increased likelihood of long-term survival without gastrointestinal cancer recurrence.
